# EUROPEAN PATENT SPECIFICATION

(11) **EP 3 723 745 B1**
(45) Date of publication and mention of the grant of the patent: **19.06.2024**
(21) Application number: 18815729.1
(22) Date of filing: 14.12.2018
(51) Int. Cl.: A61K 31/192, A61K 31/216, A61P 21/00, C07C 69/14, C07C 59/68, C07C 59/72, C07C 255/54, C07C 323/20, C07C 69/736

(54) **PHENOXY ACIDS FOR THE TREATMENT OF NEUROMUSCULAR DISORDERS**
PHENOXY-SÄURE ZUR BEHANDLUNG NEUROMUSKULÄRER ERKRANKUNGEN
PHÉNOXY ACIDES POUR LE TRAITEMENT DE TROUBLES NEUROMUSCULAIRES

(30) Priority: 14.12.2017 US 201762598940 P; 15.01.2018 EP 18151605
(43) Date of publication of application: 21.10.2020
(73) Proprietor: NMD Pharma A/S, 8200 Århus N (DK)
(72) Inventor: HOLM PEDERSEN, Thomas, 8240 Risskov (DK); J.S. KNUTSEN, Lars, Essex CO 13 9AY (GB); KELLY, Nicholas, 2880 Bagsværd (DK); BROCH-LIPS, Martin, 8541 Skødstrup (DK); ELSBORG OLESEN, Claus, 8230 Åbyhøj (DK); LABELLE, Marc, deceased (DK); BÆKGAARD NIELSEN, Ole, 8250 Egå (DK); KUMAR, Rajesh, Winnipeg, MB R3Y 0R9 (CA)
(74) Representative: Høiberg P/S
(86) International application number: PCT/EP2018/084980
(87) International publication number: WO 2019/115777

(56) References cited:
- WO-A1-2016/202341
- WO-A2-2004/002925

## Description

### Technical field

The present invention relates to compounds and their use in treating, ameliorating and/or preventing neuromuscular disorders, including the reversal of drug-induced neuromuscular blockade. The compounds as defined herein preferably inhibit the CIC-1 ion channel. The invention further relates to the compound for use in treating, preventing and/or ameliorating neuromuscular disorders, by administering said composition to a person in need thereof.

### Background

Walking, breathing, and eye movement are examples of essential everyday physiological activities that are powered by the contractile activity of skeletal muscle. Skeletal muscles are inherently in a resting state and contractile activity occurs exclusively in response to commands from the central nervous system (CNS). Such neuronal commands take the form of action potentials that travel from the brain to the muscle fibres in several steps. The neuromuscular junction (NMJ) is a highly specialized membrane area on muscle fibres where motor neurons come into close contact with the muscle fibres, and it is at the NMJ where neuronal action potentials are transmitted to muscular action potentials in a one-to-one fashion *via* synaptic transmission.

Neuromuscular transmission refers to the sequence of cellular events at the NMJ whereby an action potential in the lower motor neuron is transmitted to a corresponding action potential in a muscle fibre (Wood SJ, Slater CR. Safety factor at the neuromuscular junction. Prog. Neurobiol. 2001, 64, 393-429). When a neuronal action potential arrives at the pre-synaptic terminal it triggers influx of Ca²⁺ through voltage gated P/Q-type Ca²⁺ channels in the nerve terminal membrane. This influx causes a rise in cytosolic Ca²⁺ in the nerve terminal that triggers exocytosis of acetylcholine (ACh). Released ACh next diffuses across the synaptic cleft to activate nicotinic ACh receptors in the post-synaptic, muscle fibre membrane. Upon activation, ACh receptors convey an excitatory current flow of Na⁺ into the muscle fibre, which results in a local depolarization of the muscle fibre at the NMJ that is known as the endplate potential (EPP). If the EPP is sufficiently large, voltage gated Na⁺ channels in the muscle fibre will activate and an action potential in the muscle fibre will ensue. This action potential then propagates from the NMJ throughout the muscle fibre and triggers release of Ca²⁺ release from the sarcoplasmic reticulum. The released Ca²⁺ activates the contractile proteins within the muscle fibres, thus resulting in contraction of the fibre.

Failure of neuromuscular transmission can arise from both pre-synaptic dysfunction [Lambert Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107), amyotrophic lateral sclerosis (Killian JM, Wilfong AA, Burnett L, Appel SH, Boland D. Decremental motor responses to repetitive nerve stimulation in ALS. Muscle Nerve, 1994, 17, 747-754), spinal muscular atrophy (Wadman RI, Vrancken AF, van den Berg LH, van der Pol WL. Dysfunction of the neuromuscular junction in spinal muscular atrophy types 2 and 3. Neurology, 2012, 79, 2050-2055) and as a result of post-synaptic dysfunction as occurs in myasthenia gravis (Le Panse R, Berrih-Aknin S. Autoimmune myasthenia gravis: autoantibody mechanisms and new developments on immune regulation. Curr Opin Neurol., 2013, 26, 569-576)]. Failure to excite and/or propagate action potentials in muscle can also arise from reduced muscle excitability such as in critical illness myopathy (CIM) (Latronico, N., Bolton, C.F. Critical illness polyneuropathy and myopathy: a major cause of muscle weakness and paralysis. Lancet Neurol. 2011, 10, 931-941). In Lambert Eaton syndrome, an autoimmune attack against the pre-synaptic P/Q-type Ca²⁺ channels results in markedly reduced Ca²⁺ influx into the nerve terminal during the pre-synaptic action potential and consequently a reduced release of ACh into the synaptic cleft. In myasthenia gravis, the most common finding is an autoimmune attack on the post-synaptic membrane either against the nicotinic ACh receptors or the muskreceptor in the muscle fibre membrane³. Congenital forms of myasthenia are also known⁵. Common to disorders with neuromuscular transmission failure (Lambert Eaton syndrome, amyotrophic lateral sclerosis, spinal muscular atrophy and myasthenia gravis) is that the current flow generated by ACh receptor activation is markedly reduced, and EPPs therefore become insufficient to trigger muscle fibre action potentials.

Neuromuscular blocking agents also reduce EPP by antagonizing ACh receptors. In CIM with reduced muscle excitability, the EPP may be of normal amplitude but they are still insufficient to trigger muscle fibre action potentials because the membrane potential threshold for action potential excitation has become more depolarized because of loss of function of voltage gated Na⁺ channels in the muscle fibres.

While ACh release (Lambert Eaton, amyotrophic lateral sclerosis, spinal muscular atrophy), ACh receptor function (myasthenia gravis, neuromuscular blockade) and function of voltage gated Na⁺ channels (CIM) are essential components in the synaptic transmission at NMJ, the magnitude of the EPP is also affected by inhibitory currents flowing in the NMJ region of muscle fibres. These currents tend to outbalance excitatory current through ACh receptors and, expectedly, they thereby tend to reduce EPP amplitude. The most important ion channel for carrying such inhibitory membrane currents in muscle fibres is the muscle-specific CIC-1 CI⁻ ion channel (Kwiecihski H, Lehmann-Horn F, Rüdel R. Membrane currents in human intercostal muscle at varied extracellular potassium. Muscle Nerve. 1984, 7, 465-469; Kwieci 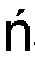 ski H, Lehmann-Horn F, Rüdel R. Drug-induced myotonia in human intercostal muscle. *Muscle Nerve.* **1988,** *11*, 576-581; Pedersen, T.H., F. de Paoli, and O.B. Nielsen. Increased excitability of acidified skeletal muscle: role of chloride conductance. J. Gen. Physiol., 2005, 125, 237-246).

ACh esterase (AChE) inhibitors are traditionally used in the treatment of myasthenia gravis. This treatment leads to improvement in most patients but it is associated with side effects, some of which are serious (Mehndiratta MM, Pandey S, Kuntzer T. Acetylcholinesterase inhibitor treatment for myasthenia gravis. Cochrane Database Syst Rev. 2014, Oct 13; 10). Because ACh is an import neurotransmitter in the autonomic nervous system, delaying its breakdown can lead to gastric discomfort, diarrhea, salivation and muscle cramping. Overdosing is a serious concern as it can lead to muscle paralysis and respiratory failure, a situation commonly referred to as cholinergic crisis. Despite the serious side effects of ACHE inhibitors, these drugs are today the treatment of choice for a number of disorders involving neuromuscular impairment. In patients where pyridostigmine (a parasympathomimetic and a reversible ACHE inhibitor) is insufficient, corticosteroid treatment (prednisone) and immunosuppressive treatment (azathioprine) is used. Plasma exchange can be used to obtain a fast but transient improvement.

Unfortunately, all of the currently-employed drug regimens for treatment of myasthenia gravis are associated with deleterious long-term consequences (Howard, J.F. Jr. Adverse drug effects on neuromuscular transmission. Semin Neurol. 1990, 10, 89 - 102) despite research to identify new treatments (Gilhus, N.E. New England Journal of Medicine, 2016, 375, 2570-2581).

The CIC-1 ion channel (Pedersen, T.H., Riisager, A., Vincenzo de Paoli, F., Chen, T-Y, Nielsen, O.B. Role of physiological CIC-1 CI- ion channel regulation for the excitability and function of working skeletal muscle. J. Gen. Physiol. 2016, 147, 291 - 308) is emerging as a target for potential drugs, although its potential has been largely unrealized.

There have been publications of various ligands at the CIC-1 ion channels, see for example: Liantonio, A., Accardi, A., Carbonara, G., Fracchiolla, G., Loiodice, F., Tortorella P, Traverso S, Guida P, Pierno S, De Luca A, Camerino DC, Pusch M. Molecular requisites for drug binding to muscle CLC-1 and renal CLC-K channel revealed by the use of phenoxy-alkyl derivatives of 2-(p-chlorophenoxy)propionic acid. Mol. Pharmacol., 2002, 62, 265 - 271 and Liantonio, A. et al., Structural requisites of 2-(p-chlorophenoxy)propionic acid analogues for activity on native rat skeletal muscle chloride conductance and on heterologously expressed CLC-1. Br. J. Phamacol., 2003, 129, 1255 - 1264.

In the article Liantonio, A., Pusch, M., Picollo, A., Guida, P., De Luca, A., Pierno, S., Fracchiolla, G., Loiodice, F., Tortorella, P., Conte-Camerino, D. Investigations of pharmacologic properties of the renal CLC-K1 chloride channel co-expressed with barttin by the use of 2-(p-chlorophenoxy)propionic acid derivatives and other structurally unrelated chloride hannels blockers. Journal of the American Society of Nephrology, 2004, 15, 13 - 20, ligands for CLC-K1 chloride channels were disclosed.

In Bettoni, G., Ferorelli, S., Loiodice, F., Tangari, N., Tortorella, V., Gasparrini, F., Misiti, D., Villani, C. Chiral α-substituted α-aryloxyacetic acids: synthesis, absolute configuration, chemical resolution, and direct separation by HPLC. Chirality, 1992, 4, 193 - 203, some further substituted phenoxyacetic acids were investigated.

In the publication Pusch, M., Liantonio, A., Bertorello, L., Accardi, A., De Luca, A., Pierno, S., Tortorella, V., Conte-Camerino, D. Pharmacological characterization of chloride channels belonging to the CIC family by the use of chiral clofibric acid derivatives. Molecular Pharmacology, 2000, 58, 498 - 507, the authors disclosed effects of enantiomers of 2-(p-chlorophenoxy)propionic acid on CLC-1 and CLC-2 ion channels.

In the article Ferorelli, S., Loiodice, F., Tortorella, V., Conte-Camerino, D., De Luca, A.M. Carboxylic acids and skeletal muscle chloride channel conductance: effects on the biological activity induced by the introduction of methyl groups on the aromatic ring of chiral α-(4-chloro-phenoxy)alkanoic acids, Farmaco, 2001, 56, 239 - 246, derivatives of (4-chloro-phenoxy)alkanoic acids were tested for skeletal muscle chloride conductance.

In the publication: Conte-Camerino, D., Mambrini, M., DeLuca, A., Tricarico, D., Bryant, S.H., Tortorella, V., Bettoni, G. Enantiomers of clofibric acid analogs have opposite actions on rat skeletal muscle chloride channels, Pfluegers Archiv., 1988, 413, 105 - 107, enantiomers of clofibric acid were investigated, as well as Feller, D.R., Kamanna, V.S., Newman, H.A.I., Romstedt, K.J., Witiak, D.T., Bettoni, G., Bryant, S.H., Conte-Camerino, D., Loiodice, F., Tortorella, V. Dissociation of hypolipidemic and antiplatelet actions from adverse myotonic effects of clofibric acid related enantiomers. Journal of Medicinal Chemistry, 1987, 30, 1265 - 1267.

Edoardo Aromataris investigated 4-chlorophenoxyisobutyric acid derivatives in his PhD thesis "Pharmacology of the CIC-1 Chloride Channel"; see: https://digital.library.adelaide.edu.au/dspace/bitstream/2440/58973/8/02whole.pdf

In WO 2005/105727, Sandham et al., reported a range of substituted phenoxyacetic acids, including 2-(4-chloro-2-cycloheptylphenoxy)acetic acid, as CRTh2 receptor antagonists for treating inflammatory or allergic conditions.

In WO 2006/037982, Bonnert et al. described a set of substituted phenoxyacetic acids for the treatment of respiratory disorders.

In US 2006/0211765, Pairaudeau et al. reported a further series of substituted biphenyloxy acetic acids for treatment of respiratory disorders

In WO 2016/202341, Pedersen et al. reported a series of of phenoxypropionic acids and related compounds that appear to block the CIC-1 ion channel for use in treating, ameliorating and/or preventing neuromuscular disorders. However, they possess alternative structural features to those in the current invention.

In WO 2004/002925, Kim et al. reported a process for preparing (R)-aryloxypropionic esters.

In WO 2012/020567, Morita et al. reported using acyl piperazines derivatives as TTX-S blockers for us in the treatment of pain.

In the publication Buchinger et al. Chirally functionalized anion-exchange type silica monolith for enantiomer separation of 2-aryloxypropionic acid herbicides by nonaqueous capillary electrochromatography, Electrophoresis 2009, 30, 3804-3813, racemic 2-aryloxypropionic acids were chirally separated.

In the publication Hsiao, Y.L. and Chen, S. LC Separation of Enantiomers on Silica-Bonded Thiostrepton Derivatives, Chromatographia 2009, 70, 1031-8, racemic 2-aryloxypropionic acids were chirally separated.

In the publication Kato et al. Microbial Deracemization of r-Substituted Carboxylic Acids: Substrate Specificity and Mechanistic Investigation, J. Org. Chem. 2003, 68, 7234-7242, 2-phenoxypropanoic acids underwent microbial deracemization to the (*R*)-enantiomer.

In FR 2 360 251, Scott, R.M. and Armitage, G:D: disclosed the racemate and (*R*)-enantiomer of 2-phenoxypropanoic esters.

### Summary

The present invention comprises a new series of compounds that alleviate disorders of the neuromuscular junction through inhibition of CIC-1 channels.

It has been found that a set of novel compounds that inhibit CIC-1 ion channels are capable of restoring neuromuscular transmission, as evidenced by the data generated by investigation of the compound set in biological models described herein. These compounds thus constitute a new group of potential drugs that can be used to treat or ameliorate muscle weakness and muscle fatigue in neuromuscular junction disorders caused by disease or by neuromuscular blocking agents.

The present invention thus concerns the discovery of new CIC-1 ion channel inhibitors with application in the treatment of a range of conditions, such as reversal of block, ALS and myasthenic conditions, in which muscle activation by the nervous system is compromised and symptoms of weakness and fatigue are prominent.

In one aspect, the invention concerns a compound Formula (VII): 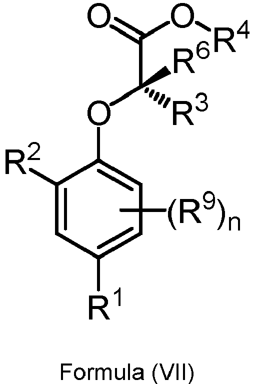 wherein:
R¹ is selected from the group consisting of F, Cl, Br, and I;
R² is selected from the group consisting of hydrogen, deuterium, F, CI and Br;
R³ is selected from the group consisting of C₁₋₅ alkyl, C₂₋₅ alkenyl, C₃₋₅ cycloalkyl, C₅ cycloalkenyl, each of which is substituted with one or more, identical or different, substituents R⁵, and C₂₋₅ alkynyl, which may be optionally substituted with one or more, identical or different, substituents R⁵; and
- R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁵ is independently selected from the group consisting of F, OC₁₋₅ alkyl optionally substituted with one or more, identical or different, substituents R⁷ and OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof.

In another aspect, the invention concerns a composition comprising a compound as defined herein and uses thereof, such as in treating, ameliorating and/or preventing a neuromuscular disorder, and/or for use in reversing and/or ameliorating a neuromuscular blockade.

The invention is defined by the claims. Any subject matter falling outside the scope of the claims is provided for information purposes only.

### Description of Drawings

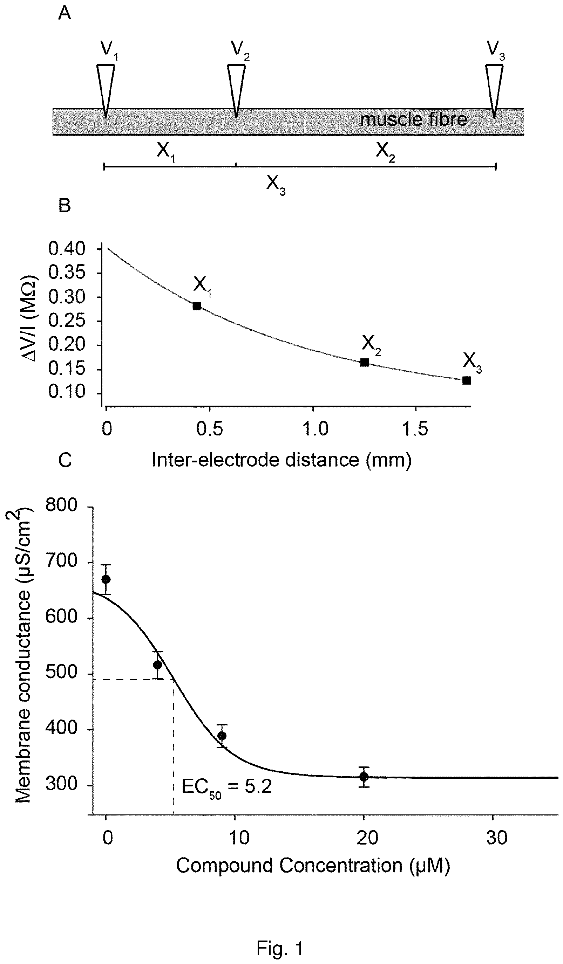

**Figure 1****:** Panel A shows a schematic representation of the positioning of the three microelectrodes (V₁, V₂ and V₃) when inserted in a single skeletal muscle fibre for Gₘ determination. Please note that the drawing illustrates only the impaled fibre although it is part of an intact muscle that contains many such fibres. All electrodes recorded the membrane potential of the fibre and the two peripheral electrodes were used to inject current (-30 nA, 50 ms). The electrodes were inserted with known inter-electrode distances (X₁, X₂ and X₃). After insertion, current was passed first via the V, electrode and then via the V₃ electrode. The resulting deflections in the membrane voltage were measured by the other electrodes. The steady state deflections in membrane potential were measured and divided by the magnitude of the injected current (-30 nA) to obtain transfer resistances. These were next plotted against inter-electrode distances, and fitted to an exponential function (Panel B), from which Gₘ could be calculated using linear cable theory. The approach described in panel A and B, was repeated for several muscle fibres in the muscle during exposure at increasing concentrations of compound A-19, with approx. 10 fibres at each concentration. Average Gₘ at each concentration was plotted as a function of compound concentration in panel C, and fitted to a 4-parameter sigmoidal function from which the EC₅₀ value for the compound was obtained (dashed line)

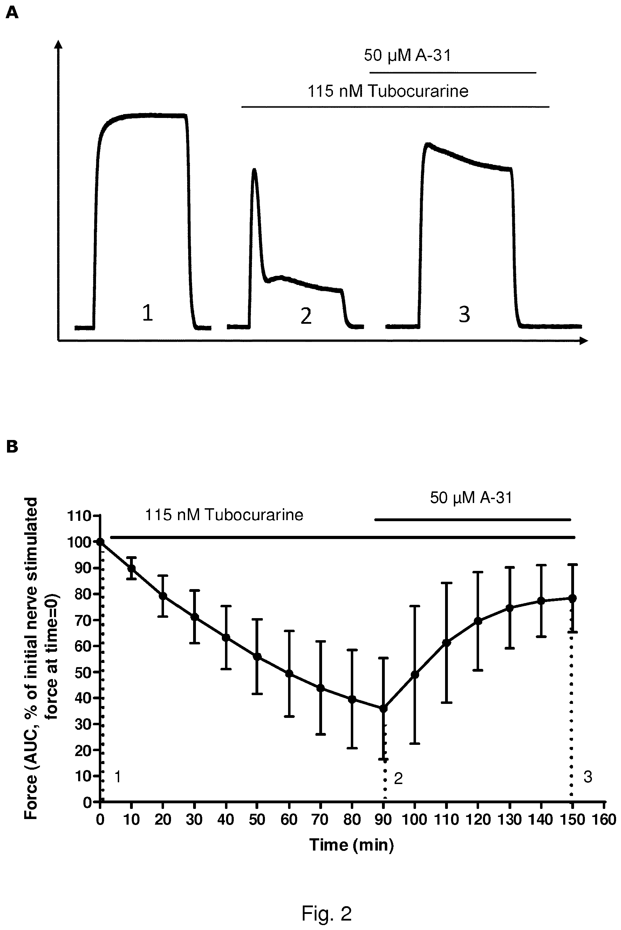

**Figure 2****:** Panel A shows representative force traces before and after exposure to compound A-19. Force traces from a representative muscle stimulated to contract in 1) control condition before addition of neuromuscular blocking agent, 2) the force response to stimulation after 90 minutes incubation with Tubocurarine. Here the muscle displays severe neuromuscular transmission impediment, and 3) The muscle force response after addition of 50 µM compound A-19. Panel B shows average force (AUC) from 3 muscles relative to their initial force. The traces presented in panel A (1, 2, 3), correspond to the dotted lines in panel B, respectively. Thus, force is lost due to 90 min incubation in tubocurarine and is subsequently recovered when compound A-19 is added.

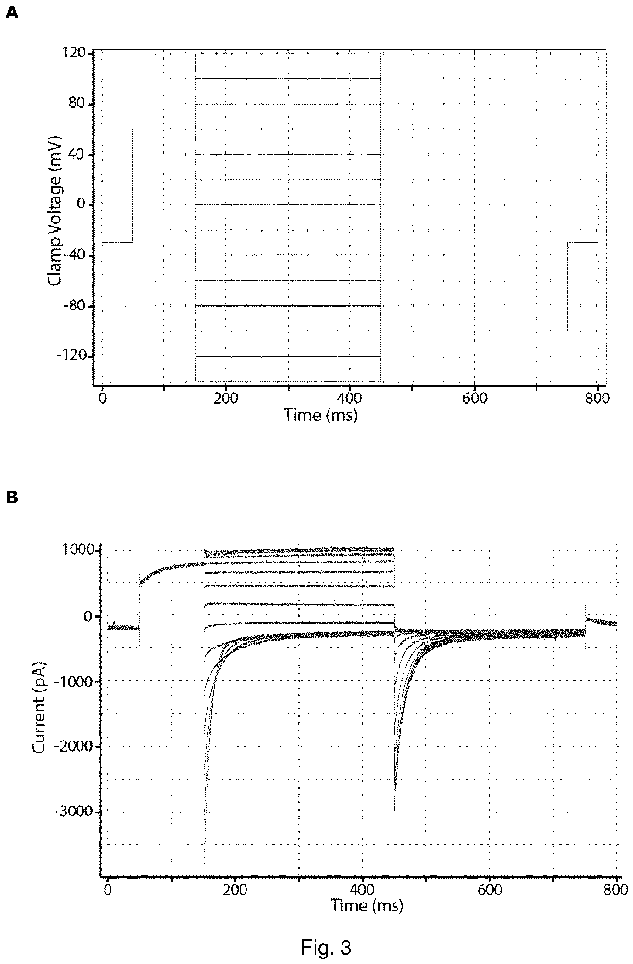

**Figure 3****:** Panel A illustrates the voltage protocol used to evoke currents in whole cell patches of CHO cells expressing human CIC-1 channels. Panel B shows representative whole cell current traces from a patched CHO cell expressing human CIC-1 channels. Currents were evoked by applying the voltage protocol shown in Panel A.

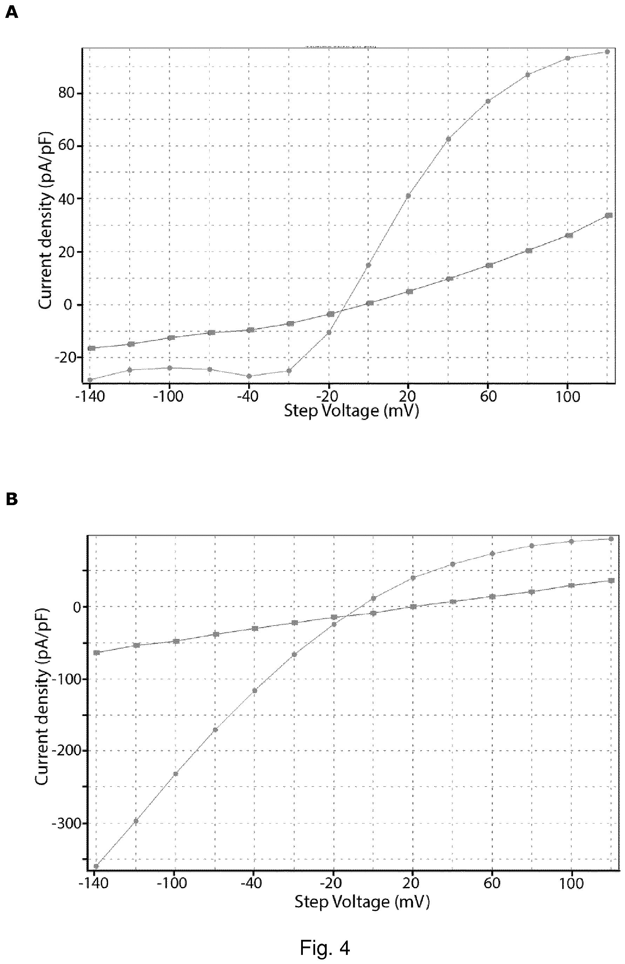

**Figure 4****:** Panel A shows a representative I/V plot of constant current density in a CIC-1 expressing CHO cell before (circles) and after (squares) application of 100 µM of the CIC-1 inhibitor, 9-anthracenecarboxylic acid (9-AC, Sigma A89405). Panel B shows a I/V plot of instant tail current density from the same CIC-1 expressing CHO cell as illustrated in Panel A, before (circles) and after (squares) application of 100 µM 9-AC.

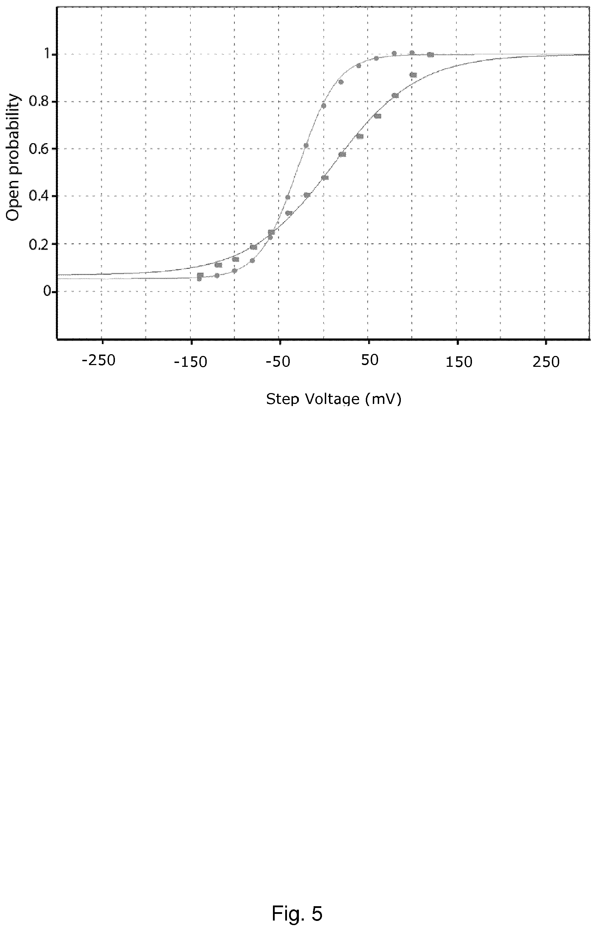

**Figure 5:** Figure 5 shows representative plots of normalized instant tail currents from a CIC-1 expressing CHO cell patch before (circles) and after (squares) application of 100 µM 9-AC. The instant tail currents at each voltage step were normalized to the maximal tail current obtained following the (+)120 mV voltage step and fitted to a Boltzmann function to determine the half activation potential, V_{1/2}.

### Detailed description

### Definitions

The term "C₁₋₄-alkyl" refers to a branched or unbranched alkyl group having from one to four carbon atoms, including but not limited to methyl, ethyl, prop-1-yl, prop-2-yl, 2-methyl-prop-1-yl, 2-methyl-prop-2-yl, but-1-yl and but-2-yl.

The term "C₁₋₄-alkenyl" refers to a branched or unbranched alkenyl group having from one to four carbon atoms, two of which are connected by a double bond, including but not limited to ethenyl, propenyl, isopropenyl, butenyl and isobutenyl.

The term "C₁₋₄-alkynyl" refers to a branched or unbranched alkynyl group having from one to four carbon atoms, two of which are connected by a triple bond, including but not limited to ethynyl, propynyl and butynyl.

The term "C₃₋₄-cycloalkyl" refers to a group having three to four carbon atoms, including but not limited to cyclopropyl, cyclobutyl and cyclopropylmethyl.

The term "half-life" as used herein is the time it takes for the compound to lose one-half of its pharmacologic activity. The term "plasma half-life" is the time that it takes the compound to lose one-half of its pharmacologic activity in the blood plasma.

The term "treatment" refers to the combating of a disease or disorder. "Treatment" or "treating," as used herein, includes any desirable effect on the symptoms or pathology of a disease or condition as described herein, and may include even minimal changes or improvements in one or more measurable markers of the disease or condition being treated. "Treatment" or "treating" does not necessarily indicate complete eradication or cure of the disease or condition, or associated symptoms thereof. In some embodiments, the term "treatment" encompasses amelioration and prevention.

The term "amelioration" refers to moderation in the severity of the symptoms of a disease or condition. Improvement in a patient's condition, or the activity of making an effort to correct, or at least make more acceptable, conditions that are difficult to endure related to patient's conditions is considered "ameliorative" treatment.

The term "prevent" or "preventing" refers to precluding, averting, obviating, forestalling, stopping, or hindering something from happening, especially by advance action.

The term "reversal" or "reversing" refers to the ability of a compound to restore nerve-stimulated force in skeletal muscle exposed either *ex vivo* or *in vivo* to a non-depolarizing neuromuscular blocking agent or another pharmaceutical that is able to depress neuromuscular transmission.

The term "ester hydrolysing reagent" refers to a chemical reagent which is capable of converting an ester functional group to a carboxylic acid with elimination of the alcohol moiety of the original ester, including but not limited to acid, base, a fluoride source, PBr₃, PCl₃ and lipase enzymes.

The term "non-depolarizing blockers" refers to pharmaceutical agents that antagonize the activation of acetylcholine receptors at the post-synaptic muscle fibre membrane by blocking the acetylcholine binding site on the receptor. These agents are used to block neuromuscular transmission and induce muscle paralysis in connection with surgery.

The term "recovery of force in muscle with neuromuscular dysfunction" refers to the ability of a compound to recover contractile force in nerve-stimulated healthy rat muscle after exposure to submaximal concentration of (115 nM) tubocurarine for 90 mins. Recovery of force is quantified as the percentage of the force prior to tubocurarine that is recovered by the compound.

The term "total membrane conductance (Gm)" is the electrophysiological measure of the ability of ions to cross the muscle fibre surface membrane. It reflects the function of ion channels that are active in resting muscle fibres of which CIC-1 is known to contribute around 80 % in most animal species.

### Compounds

It is within the scope of the present invention to provide a compound for use in treating, ameliorating and/or preventing neuromuscular disorders characterized in that the neuromuscular function is reduced. As disclosed herein, inhibition of CIC-1 improves or restores neuromuscular function. The compounds of the present invention comprise compounds capable of inhibiting the CIC-1 channel thereby improving or restoring neuromuscular function.

In one aspect, the invention concerns a compound Formula (VII): 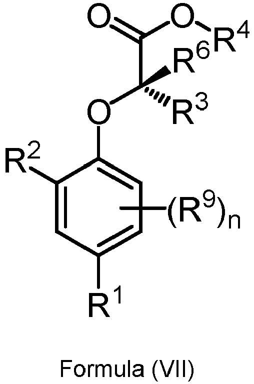 wherein:
R¹ is selected from the group consisting of F, Cl, Br, and I;
R² is selected from the group consisting of hydrogen, deuterium, F, CI and Br;
R³ is selected from the group consisting of C₁₋₅ alkyl, C₂₋₅ alkenyl, C₃₋₅ cycloalkyl, C₅ cycloalkenyl, each of which is substituted with one or more, identical or different, substituents R⁵, and C₂₋₅ alkynyl, which may be optionally substituted with one or more, identical or different, substituents R⁵; and
- R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁵ is independently selected from the group consisting of F, OC₁₋₅ alkyl optionally substituted with one or more, identical or different, substituents R⁷ and OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof.

In one aspect, the invention relates to a compound of Formula (III): 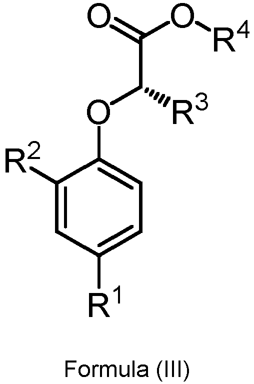 wherein:
- R¹ is selected from the group consisting of Cl, Br, and I;
- R² is selected from the group consisting of deuterium, F, Cl, and Br;
- R³ is selected from the group consisting of C₁₋₅ alkyl, C₂₋₅ alkenyl, C₃₋₅ cycloalkyl and C₅ cycloalkenyl, each of which is substituted with one or more F; and
- R⁴ is selected from the group consisting of H or C₁₋₅ alkyl; preferably H;
or a pharmaceutically acceptable salt, hydrate, polymorph, tautomer, or solvate thereof.

In one aspect, the invention relates to a compound of Formula (IV): 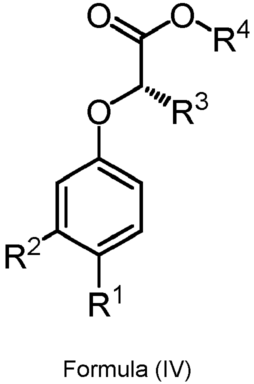 wherein:
- R¹ is selected from the group consisting of Cl, Br, and I;
- R² is selected from the group consisting of deuterium, F, Cl, and Br;
- R³ is selected from the group consisting of C₁₋₅ alkyl, C₂₋₅ alkenyl, C₃₋₅ cycloalkyl and C₅ cycloalkenyl, each of which is substituted with one or more F; and
- R⁴ is selected from the group consisting of H or C₁₋₅ alkyl; preferably H;
or a pharmaceutically acceptable salt, hydrate, polymorph, tautomer, or solvate thereof.

In one aspect, the invention relates to a compound of Formula (V): 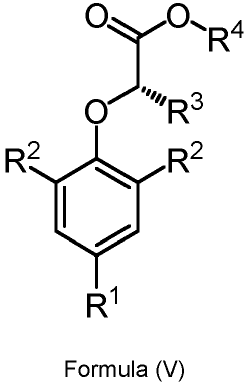 wherein:
- R¹ is Br, Cl or I;
- R² is independently deuterium, F, Cl, or Br;
- R³ is selected from the group consisting of C₁₋₅ alkyl, C₂₋₅ alkenyl, C₃₋₅ cycloalkyl and C₅ cycloalkenyl, each of which is substituted with one or more F; and
- R⁴ is selected from the group consisting of H or C₁₋₅ alkyl; preferably H;
or a pharmaceutically acceptable salt, hydrate, polymorph, tautomer, or solvate thereof.

In one aspect, the invention relates to a compound of Formula (VI): 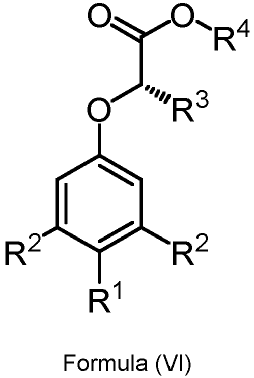 wherein:
- R¹ is Br, Cl or I;
- R² is independently deuterium, F, Cl, or Br;
- R³ is selected from the group consisting of C₁₋₅ alkyl, C₂₋₅ alkenyl, C₃₋₅ cycloalkyl and C₅ cycloalkenyl, each of which is substituted with one or more F; and
- R⁴ is selected from the group consisting of H or C₁₋₅ alkyl; preferably H;
or a pharmaceutically acceptable salt, hydrate, polymorph, tautomer, or solvate thereof.

In one aspect, the invention relates to a compound of Formula (VII), wherein:
- R¹ is selected from the group consisting of CI and Br;
- R² is selected from the group consisting of F, CI and Br;
- R³ is selected from the group consisting of C₁₋₅ alkyl, C₂₋₅ alkenyl, C₂₋₅ alkynyl, C₃₋₅ cycloalkyl and C₅ cycloalkenyl substituted with one or more, identical or different, substituents R⁵;
- R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
- R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
- R⁶ is selected from the group consisting of hydrogen and deuterium;
- R⁷ is independently selected from the group consisting of deuterium and F;
- R⁸ is independently selected from the group consisting of deuterium, methoxy, nitro, cyano, Cl, Br, I and F;
- R⁹ is deuterium; and
- n is 0, 1, 2 or 3
or a pharmaceutically acceptable salt, hydrate, polymorph, tautomer, or solvate thereof.

In one aspect, the invention relates to a compound of Formula (VII), wherein:
- R¹ is Br;
- R² is selected from the group consisting of F, CI and Br;
- R³ is methyl substituted with one or more, identical or different, substituents R⁵, C₂₋₃ alkyl optionally substituted with one or more, identical or different, substituents R⁵ or C₃₋₄ cycloalkyl optionally substituted with one or more, identical or different, substituents R⁵;
- R⁴ is H;
- R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
- R⁶ is selected from the group consisting of hydrogen and deuterium;
- R⁷ is independently selected from the group consisting of deuterium and F;
- R⁹ is deuterium; and
- n is 0, 1, 2 or 3
or a pharmaceutically acceptable salt, hydrate, polymorph, tautomer, or solvate thereof.

In one aspect, the invention relates to a compound of Formula (VII), wherein:
R¹ is selected from the group consisting of F, Cl, Br, I;
R² is selected from the group consisting of hydrogen, deuterium, F, Cl, and Br;
R³ is selected from the group consisting of C₁₋₅ alkyl, C₂₋₅ alkenyl, C₂₋₅ alkynyl, C₃₋₅ cycloalkyl and C₅ cycloalkenyl, each of which is substituted with one or more, identical or different, substituents R⁵; and
R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁵ is independently selected from the group consisting of F, OC₁₋₅ alkyl optionally substituted with one or more, identical or different, substituents R⁷ and OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof.

In one aspect, the invention relates to a compound of Formula (VII), wherein:
R¹ is Br;
R² is selected from the group consisting of hydrogen, deuterium, F, CI and Br;
R³ is C₁₋₃ alkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
n is 0
or a pharmaceutically acceptable salt, hydrate, polymorph, tautomer, or solvate thereof.

In one aspect, the invention relates to a compound of Formula (VII), wherein:
R¹ is Br;
R² is selected from the group consisting of hydrogen, deuterium, F, CI and Br;
R³ is C₃₋₅ cycloalkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
n is 0
or a pharmaceutically acceptable salt, hydrate, polymorph, tautomer, or solvate thereof.

In one aspect, the invention relates to a compound of Formula (VII), wherein:
R¹ is selected from the group consisting of F, Cl, Br, I;
R² is selected from the group consisting of hydrogen, deuterium, F, Cl, and Br;
R³ is selected from the group consisting of C₁₋₅ alkyl and C₃₋₅ cycloalkyl substituted with one or more, identical or different, substituents R⁵;
R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁵ is independently selected from the group consisting of F, OC₁₋₅ alkyl optionally substituted with one or more, identical or different, substituents R⁷ and OC₃₋₅ cycloalkyl optionally substituted with one or more, identical or different, substituents R⁷;
R⁶ is deuterium;
R⁷ is independently selected from the group consisting of deuterium and F;
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof.

In one aspect, the invention relates to a compound of Formula (VII), wherein:
R¹ is Br;
R² is selected from the group consisting of hydrogen, deuterium, F, CI and Br;
R³ is C₁₋₃ alkyl substituted with one or more, identical or different, substituents R⁵ or C₃₋₄ cycloalkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is deuterium;
R⁷ is independently selected from the group consisting of deuterium and F;
R⁹ is deuterium; and
n is 0, 1, 2 or 3.
or a pharmaceutically acceptable salt, hydrate, polymorph, tautomer, or solvate thereof.

In one aspect, the invention relates to a compound of Formula (VII), wherein:
R¹ is selected from the group consisting of F, Cl, Br, and I;
R² is selected from the group consisting of hydrogen, deuterium, F, Cl, and Br;
R³ is selected from the group consisting of fluoromethyl, fluoroethyl and fluoropropyl, each of which may be optionally substituted with one or more deuterium;
R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁶ is selected from the group consisting of hydrogen and deuterium;
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof.

In one embodiment, R¹ is selected from the group consisting of F, Cl, Br, and I, such as R¹ is selected from the group consisting of CI and Br. In one embodiment, R¹ is Br.

In one embodiment, R² is selected from the group consisting of deuterium, F, Cl, Br, and I. In one embodiment, R² is selected from the group consisting of F, Cl and Br. In one embodiment, R² is deuterium or F, such as deuterium. In one embodiment, R² is hydrogen or deuterium.

In some embodiments, R¹ is different from R², such as R¹ is Cl and R² is F; R¹ is Cl and R² is Br; such as R¹ is Cl and R² is H; such as R¹ is Cl and R² is D; such as R¹ is Br and R² is F; such as R¹ is Br and R² is CI; such as R¹ is Br and R² is H; such as R¹ is Br and R² is D.

In some embodiments, R¹ and R² are the same, such as R¹ is Cl and R² is Cl or such as R¹ is Br and R² is Br.

In one embodiment, R³ is selected from the group consisting of C₁₋₃ alkyl and C₃₋₄ cycloalkyl substituted with one or more, identical or different, substituents R⁵. In one embodiment, R³ is selected from the group consisting of C₁₋₃ alkyl and C₃₋₄ cycloalkyl substituted with one or more, identical or different, substituents R⁵. In one embodiment, said C₁₋₃ alkyl is methyl.

In one embodiment, R³ is selected from the group consisting of C₂₋₃ alkyl and C₃₋₄ cycloalkyl substituted with one or more, identical or different, substituents R⁵. In one embodiment, R³ is selected from the group consisting of C₂₋₃ alkyl and C₃₋₄ cycloalkyl substituted with one or more, identical or different, substituents R⁵.

In one embodiment, R³ is selected from the group consisting of methyl, ethyl, n-propyl or isopropyl substituted with one or more, identical or different, substituents R⁵. In one embodiment, R³ is methyl substituted with one or more, identical or different, substituents R⁵; such as R³ is methyl. In one embodiment, R³ is ethyl substituted with one or more, identical or different, substituents R⁵; such as R³ is ethyl. In one embodiment, R³ is n-propyl substituted with one or more, identical or different, substituents R⁵. In one embodiment, R³ is isopropyl substituted with one or more, identical or different, substituents R⁵.

In one embodiment, R³ is C₁₋₅ alkyl substituted with one or more F. Thus, in one embodiment, R³ is selected from the group consisting of fluoromethyl, fluoroethyl and fluoropropyl. In one embodiment, R³ is selected from the group consisting of -CH₂F, - CHF₂, -CF₃, -CH₂CH₂F, -CH₂CHF₂ and -CH₂CF₃, preferably -CH₂F. In one embodiment, R³ is selected from the group consisting of fluoromethyl, difluoromethyl, 2-fluoroeth-1-yl, (1*S*)-1-fluoroeth-1-yl, (1*R*)-1-fluoroeth-1-yl, (1*S*)-1 ,2-difluoroeth-1-yl, (1*R*)-1,2-difluoroeth-1-yl, 3-fluoroprop-1-yl, (1*S*)-1-fluoroprop-1-yl, (1*R*)-1-fluoroprop-1-yl, (2S)-2-fluoroprop-1-yl, (2*R*)-2-fluoroprop-1-yl, (1*S*)-2-fluoro-1-methyl-eth-1-yl, (1*S*)-2-fluoro-1-methyl-eth-1-yl and 2-fluoro-1-(fluoromethyl)eth-1-yl. In one embodiment, R³ is selected from the group consisting of fluoromethyl, 2-fluoroeth-1-yl, (1*S*)-1-fluoroeth-1-yl and (1*R*)-1-fluoroeth-1-yl.

In one embodiment, R³ is cyclopropyl substituted with one or more, identical or different, substituents R⁵. In one embodiment, R³ is cyclopropylmethyl substituted with one or more, identical or different, substituents R⁵. In one embodiment, R³ is cyclobutyl substituted with one or more, identical or different, substituents R⁵.

In one embodiment, R³ is C₃₋₅ cycloalkyl substituted with one or more F. Thus, in one embodiment, R³ is selected from the group consisting of cyclopropyl, cyclobutyl, cyclopentyl, cyclopropylmethyl, cyclopropylethyl and cyclobutylmethyl, substituted with one or more F.

In one embodiment, R³ is C₂₋₅ alkenyl substituted with one or more, identical or different, substituents R⁵. In one embodiment, R³ is selected from the group consisting of ethenyl, propenyl, isopropenyl, butenyl, isobutenyl and pentenyl, substituted with one or more F.

In one embodiment, R³ is substituted with one or more, identical or different, substituents R⁵. In one embodiment, R⁵ is independently selected from the group consisting of deuterium, F, -OC₁₋₅ alkyl optionally substituted with one or more, identical or different, substituents R⁷ and -OC₃₋₅ cycloalkyl optionally substituted with one or more, identical or different, substituents R⁷.

In one embodiment, R⁵ is F.

In one embodiment, R³ is substituted with one fluorine.

In one embodiment, R³ is substituted with one or more -OC₁₋₅ alkyl groups optionally substituted with one or more, identical or different, substituents R⁷; such as R³ is substituted with one or more -OMe groups optionally substituted with one or more, identical or different, substituents R⁷; such as R³ is substituted with one or more -OEt groups optionally substituted with one or more, identical or different, substituents R⁷; or such as R³ is substituted with one or more -OC₃₋₅ cycloalkyl groups optionally substituted with one or more, identical or different, substituents R⁷.

In one embodiment, R⁴ is H forming an acid. In one embodiment, said acid is deprotonated. Said deprotonated acid may interact with a cation, such as an alkali metal cation. In one embodiment, R⁴ is the sodium counterion.

In one embodiment, R⁶ is H. In another embodiment, R⁶ is D.

In one embodiment, n is an integer 0, 1, 2, 3, or 4, such as n is 0; such as n is 1; such as n is 2.

In one embodiment,
- R¹ is selected from the group consisting of F, C!, Br, and I;
- R³ is selected from the group consisting of C₁₋₅ alkyl, C₂₋₅ alkenyl, C₃₋₅ cycloalkyl and C₅ cycloalkenyl, each of which is substituted with one or more F; and
- R⁴ is selected from the group consisting of H and C₁₋₅ alkyl, preferably H;
- n is 0;
or a pharmaceutically acceptable salt, hydrate, polymorph, tautomer, or solvate thereof.

In one embodiment,
- R¹ is CI;
- R² is selected from the group consisting of F, CI and Br;
- R³ is methyl substituted with one or more, identical or different, substituents R⁵, C₂₋₃ alkyl optionally substituted with one or more, identical or different, substituents R⁵ or C₃₋₄ cycloalkyl optionally substituted with one or more, identical or different, substituents R⁵;
- R⁴ is H;
- R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
- R⁶ is selected from the group consisting of hydrogen and deuterium;
- R⁷ is independently selected from the group consisting of deuterium and F;
- R⁹ is deuterium; and
- n is 0, 1, 2 or 3.

In one embodiment,
R¹ is CI;
R² is selected from the group consisting of hydrogen, deuterium, F, CI and Br;
R³ is C₁₋₃ alkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
n is 0.

In one embodiment,
R¹ is Cl;
R² is selected from the group consisting of hydrogen, deuterium, F, CI and Br;
R³ is C₁₋₃ alkyl substituted with one or more, identical or different, substituents R⁵ or C₃₋₄ cycloalkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is deuterium;
R⁷ is independently selected from the group consisting of deuterium and F;
R⁹ is deuterium; and
n is 0, 1, 2 or 3.

In one embodiment,
- R¹ is Br;
- R² is selected from the group consisting of F, CI and Br;
- R³ is methyl substituted with one or more, identical or different, substituents R⁵, C₂₋₃ alkyl optionally substituted with one or more, identical or different, substituents R⁵ or C₃₋₄ cycloalkyl optionally substituted with one or more, identical or different, substituents R⁵;
- R⁴ is H;
- R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
- R⁶ is selected from the group consisting of hydrogen and deuterium;
- R⁷ is independently selected from the group consisting of deuterium and F;
- R⁹ is deuterium; and
- n is 0, 1, 2 or 3.

In one embodiment,
R¹ is Br;
R² is selected from the group consisting of hydrogen, deuterium, F, CI and Br;
R³ is C₁₋₃ alkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
n is 0.

In one embodiment,
R¹ is Br;
R² is selected from the group consisting of hydrogen, deuterium, F, CI and Br;
R³ is C₁₋₃ alkyl substituted with one or more, identical or different, substituents R⁵ or C₃₋₄ cycloalkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is deuterium;
R⁷ is independently selected from the group consisting of deuterium and F;
R⁹ is deuterium; and
n is 0, 1, 2 or 3.

Exemplary compounds of the invention and related compounds used in the Examples:

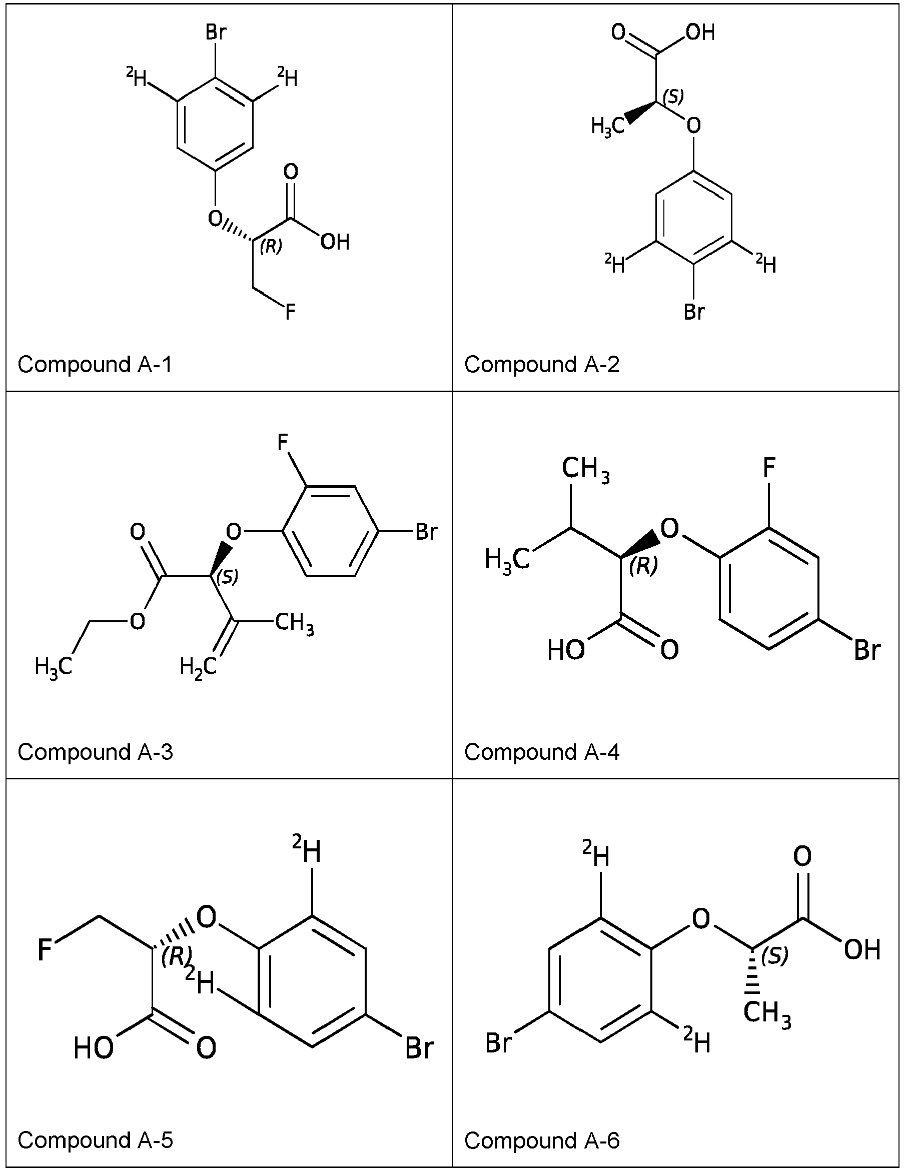

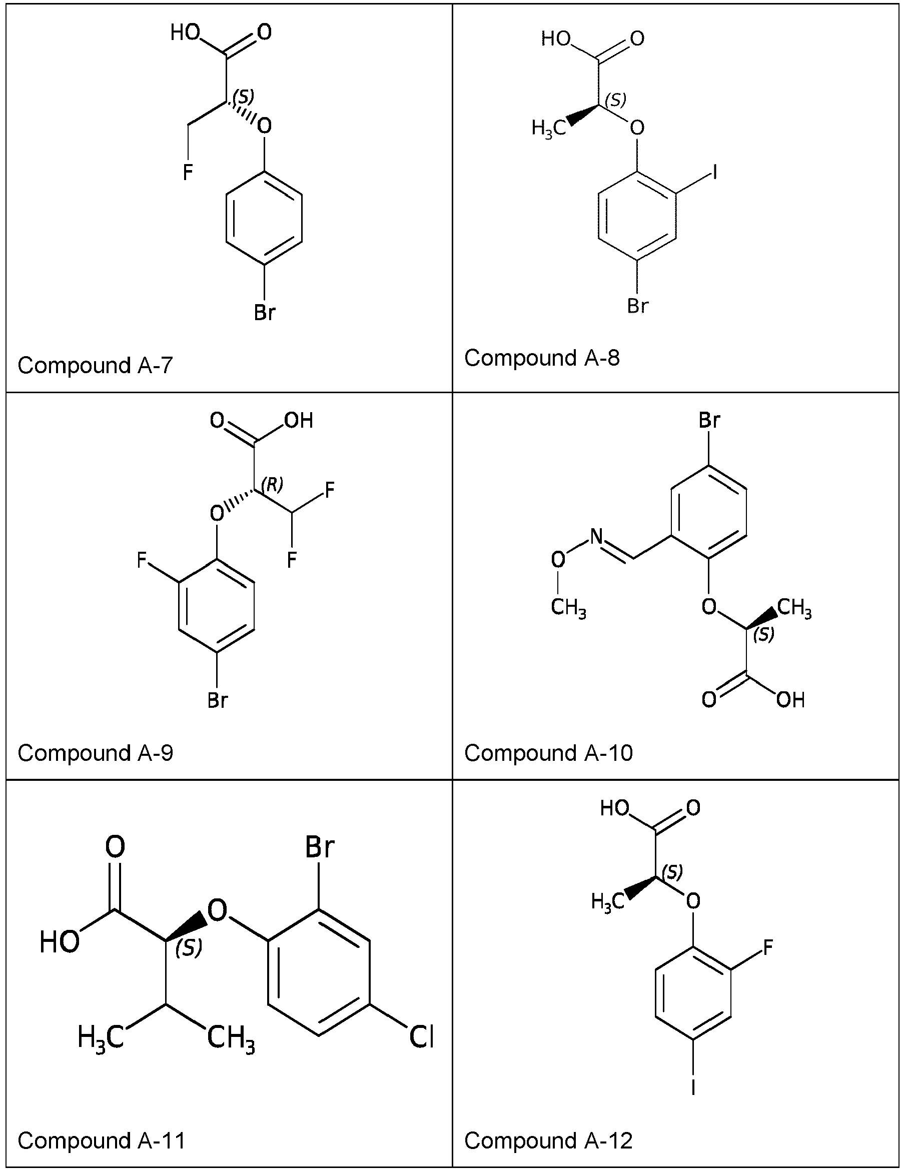

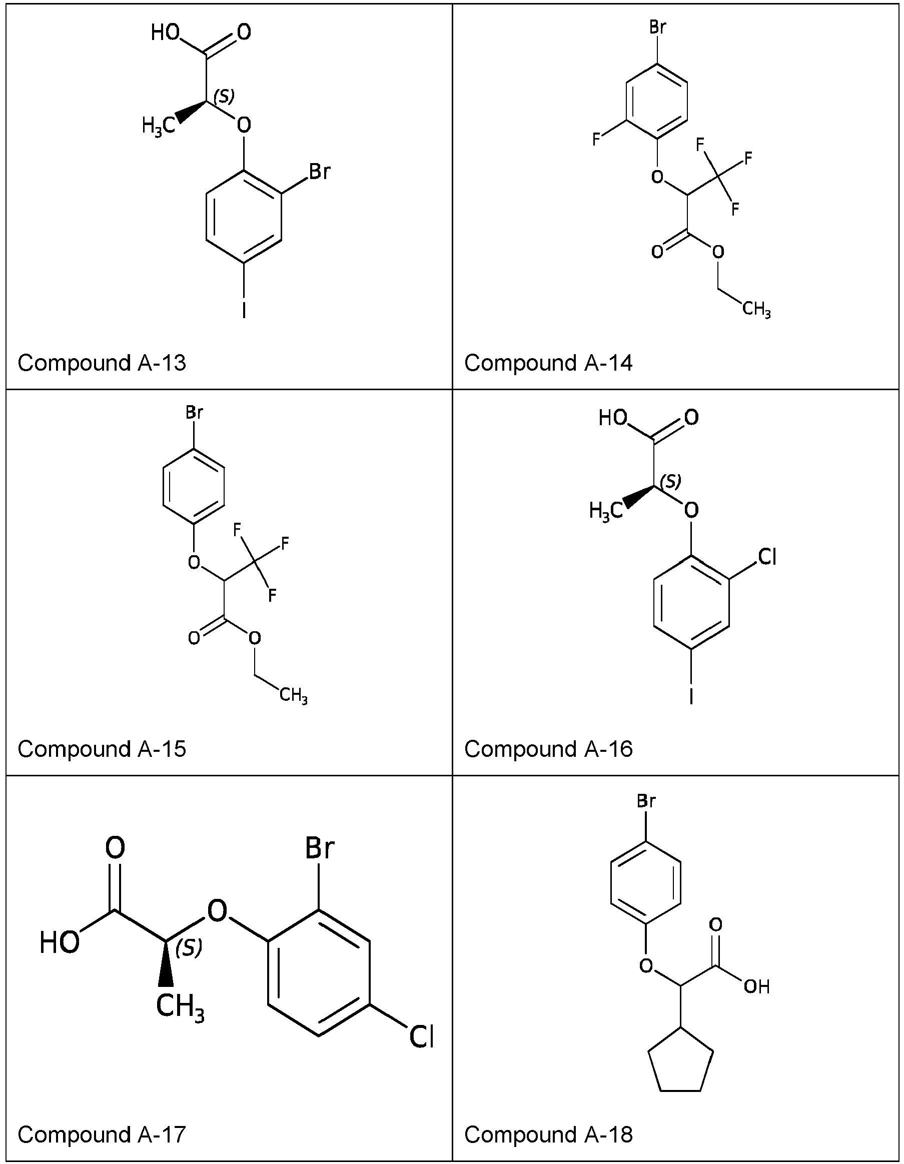

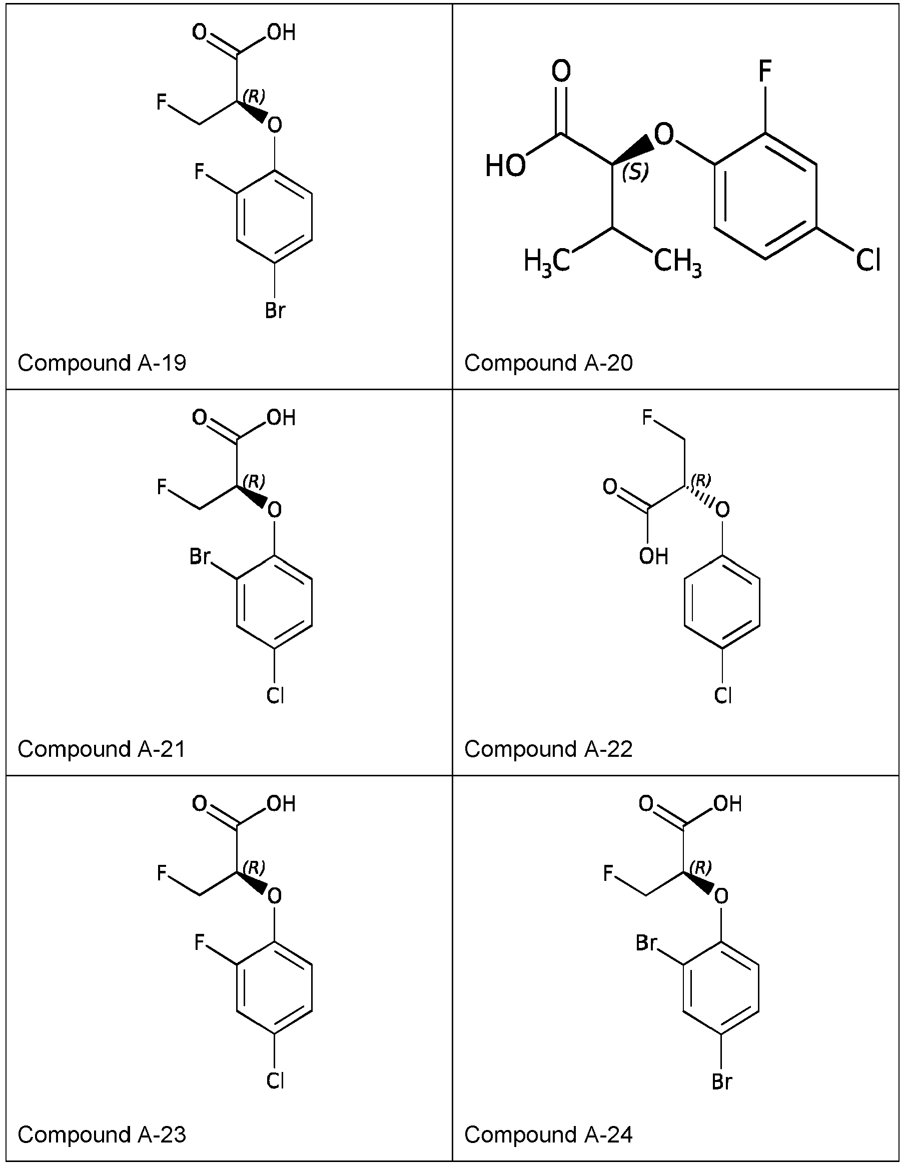

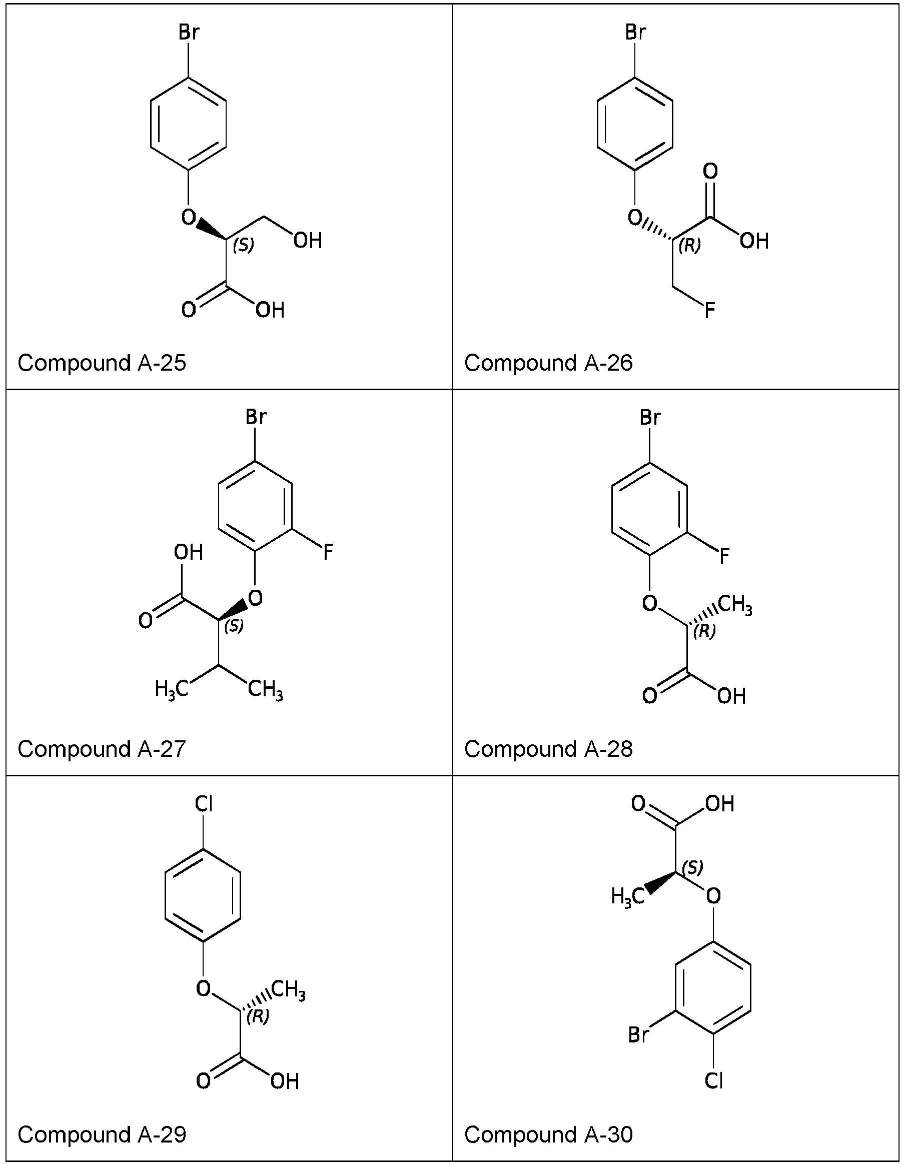

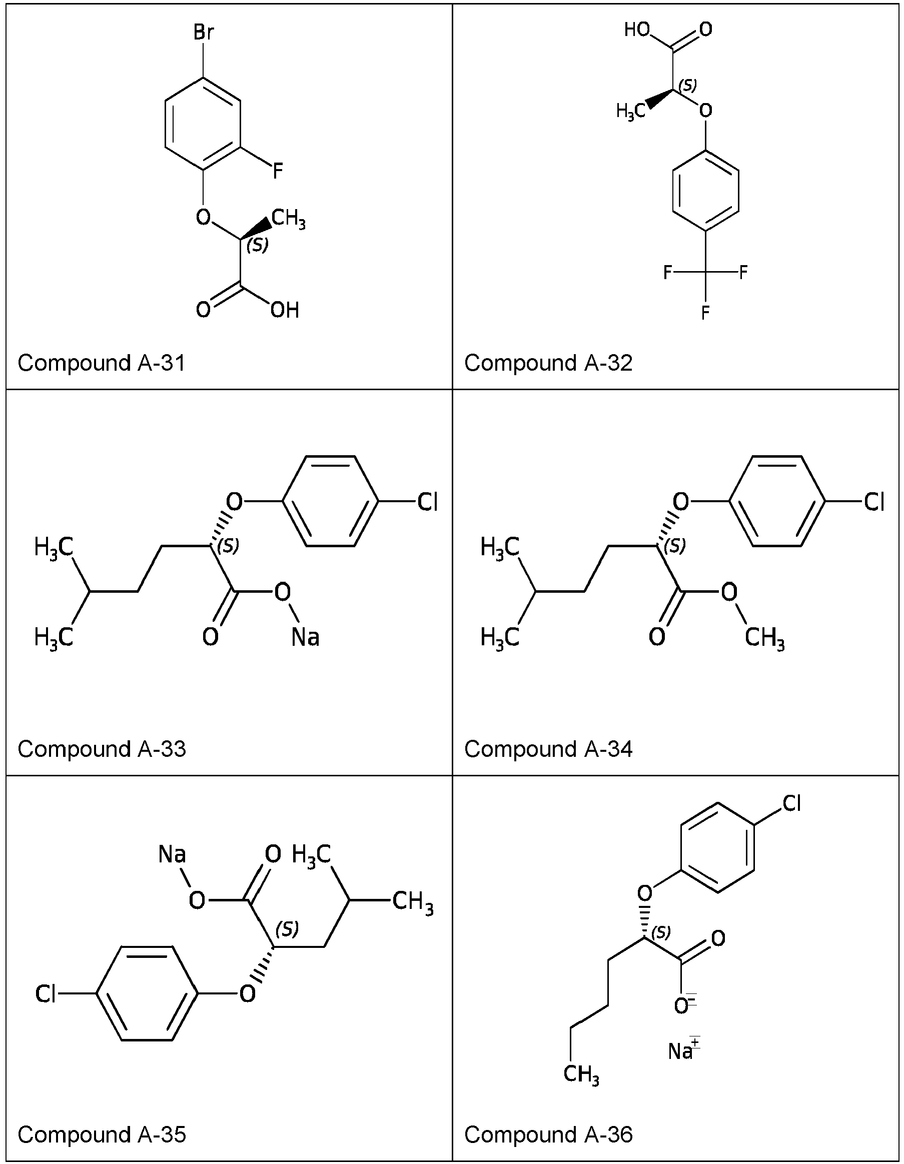

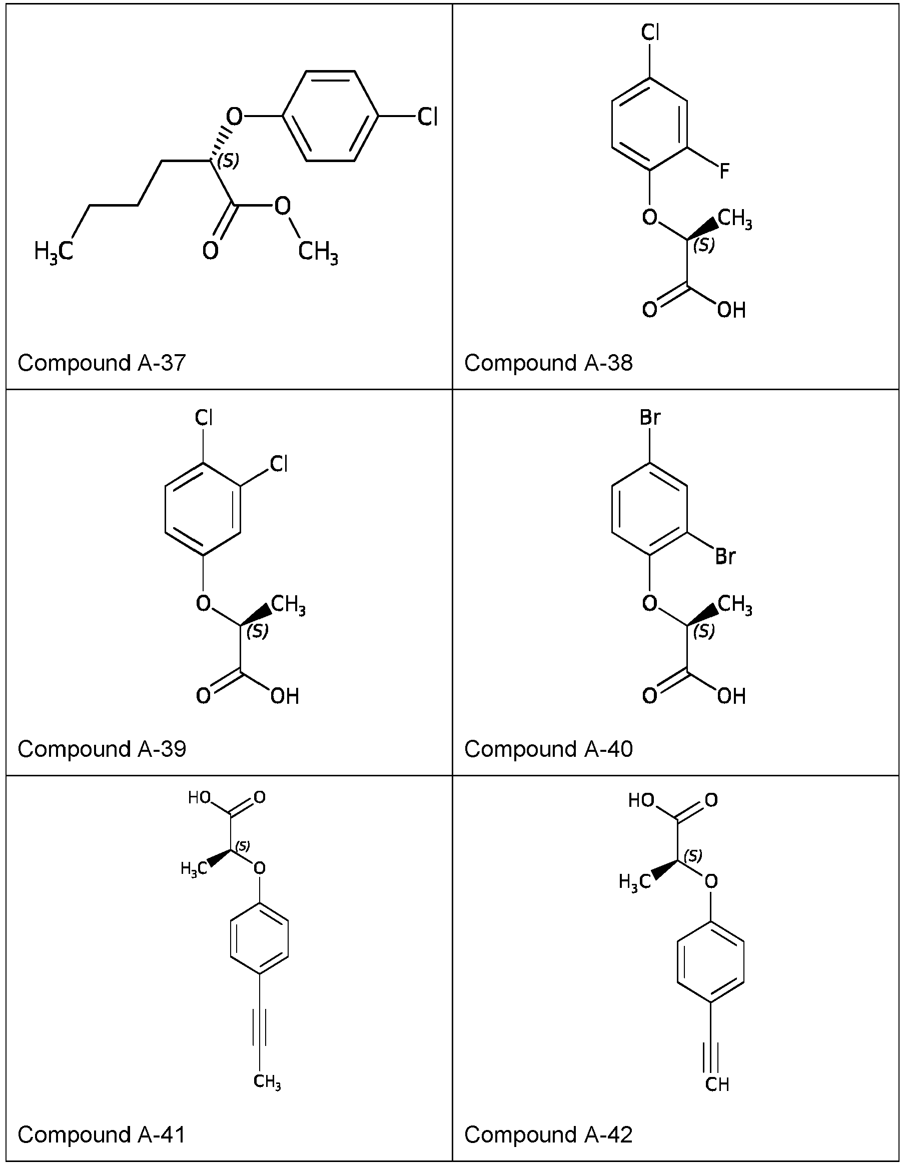

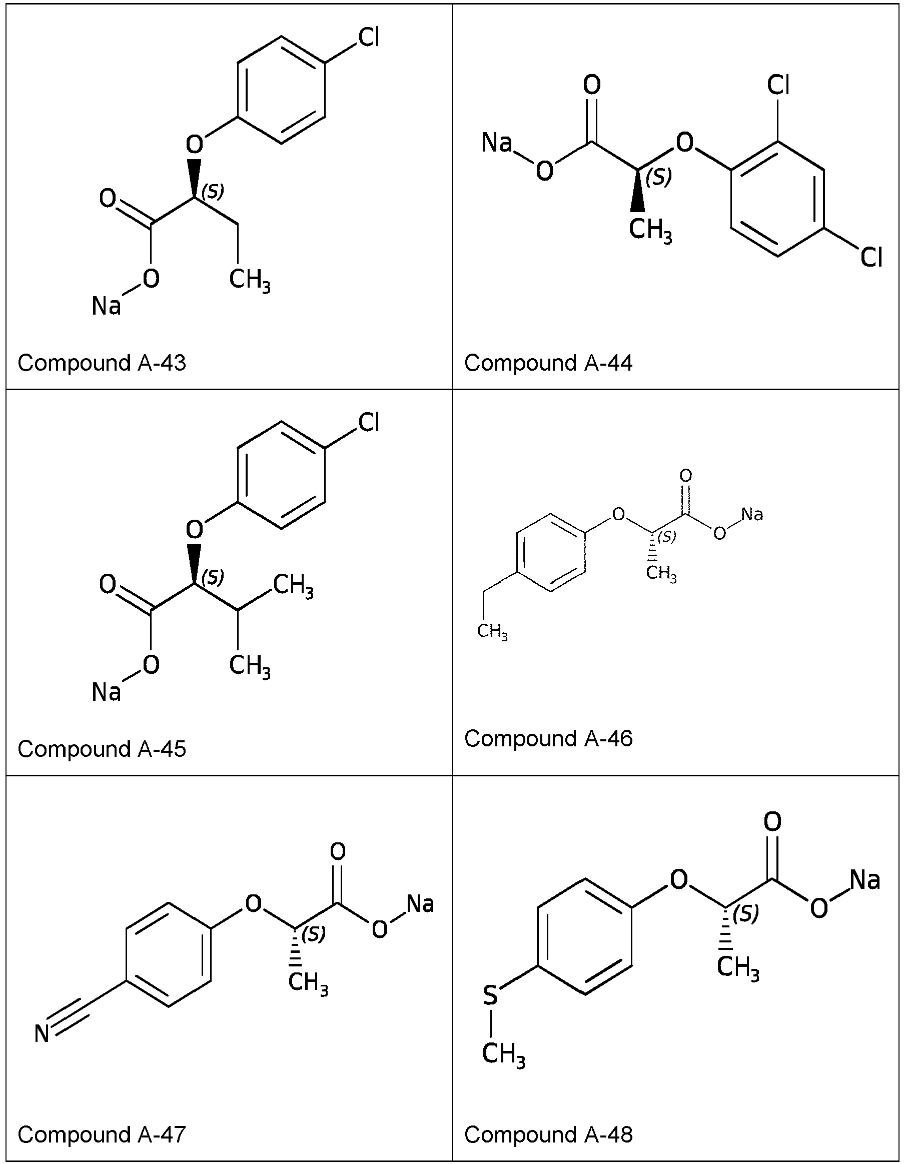

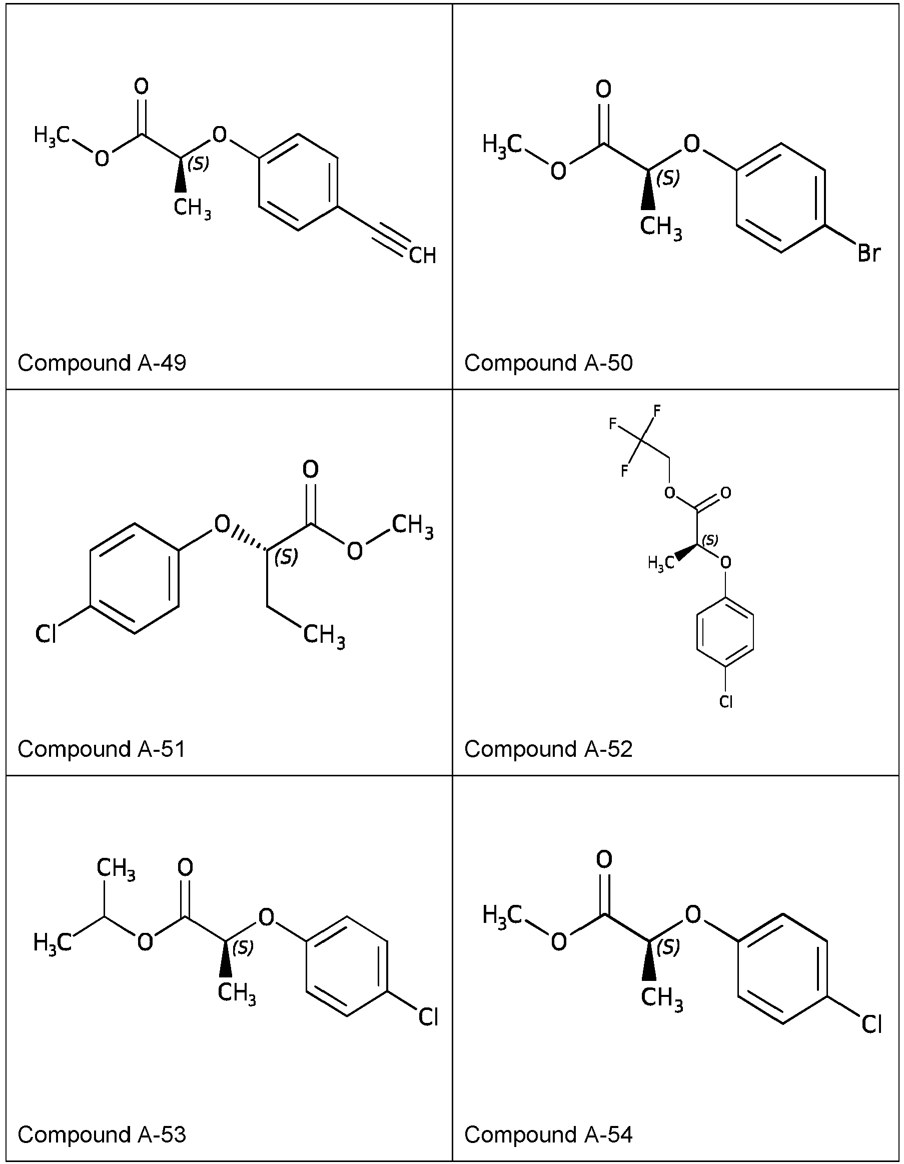

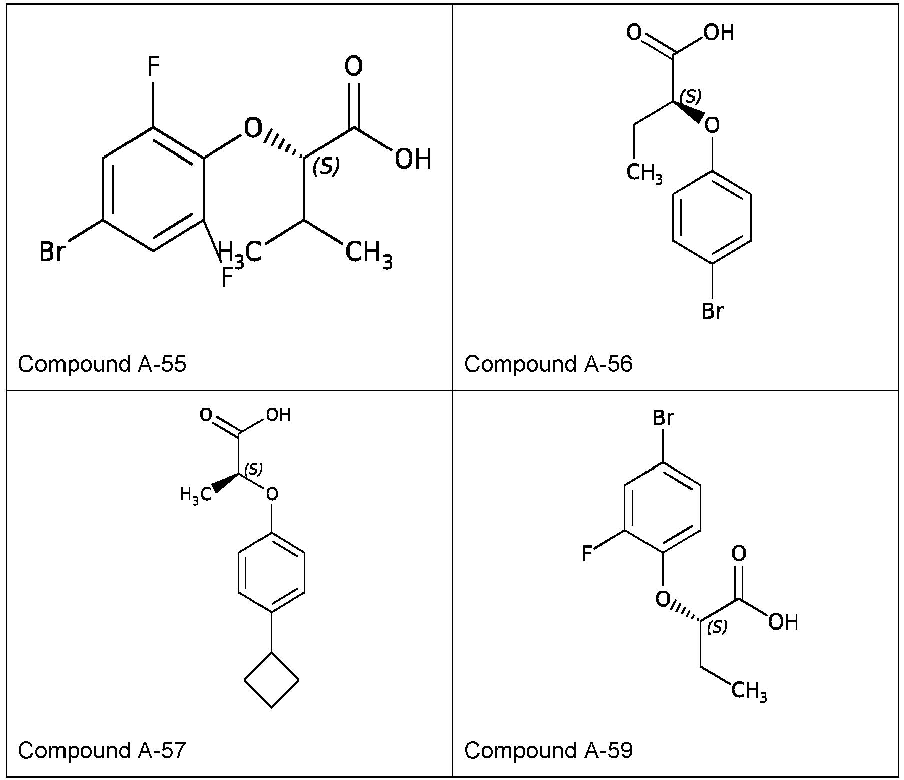

In a specific embodiment, the compound is selected from the group consisting of:
(2*R*)-2-[4-bromo(3,5-²H₂)phenoxy]-3-fluoropropanoic acid;
(2*R*)-2-[4-bromo(2,6-²H₂)phenoxy]-3-fluoropropanoic acid;
(2*R*)-2-(4-bromo-2-fluorophenoxy)-3,3-difluoropropanoic acid;
(2*R*)-2-(4-bromo-2-fluorophenoxy)-3-fluoropropanoic acid;
(2*R*)-2-(2-bromo-4-chlorophenoxy)-3-fluoropropanoic acid;
(2*R*)-2-(4-chlorophenoxy)-3-fluoropropanoic acid;
(2*R*)-2-(4-chloro-2-fluorophenoxy)-3-fluoropropanoic acid;
(2*R*)-2-(2,4-dibromophenoxy)-3-fluoropropanoic acid;
(2*R*)-2-(4-bromophenoxy)-3-fluoropropanoic acid;
(2*S*,3*E*)-2-(4-bromophenoxy)-4-fluorobut-3-enoic acid;
(2*S*)-2-(2,4-dibromophenoxy)-3-methoxypropanoic acid;
(2*R*)-2-(4-bromo-2-chlorophenoxy)-3-fluoropropanoic acid;
(2*S*)-2-(4-bromo-2-chlorophenoxy)-4-fluorobutanoic acid;
(2*R*)-2-(4-bromophenoxy)-3-fluoro(2-²H)propanoic acid;
(2*S*)-2-(4-bromo-2-fluorophenoxy)-4-fluorobutanoic acid;
(2*S*)-2-(4-bromophenoxy)-4-fluorobutanoic acid;
(2*R*,3*R*)-2-(4-bromophenoxy)-3-fluorobutanoic acid;
(2*R*,3*R*)-2-(4-bromo-2-fluorophenoxy)-3-fluorobutanoic acid;
(2*S*)-2-(4-bromophenoxy)pent-4-ynoic acid;
(2*S*)-2-(2,4-dibromophenoxy)pent-4-ynoic acid;
(2*S*)-2-(4-bromo-2-chlorophenoxy)pent-4-ynoic acid; and
(2*S*)-2-(4-bromo-2-fluorophenoxy)pent-4-ynoic acid.

### Methods of treatment

The references to methods of treatment in this description are to be interpreted as references to the compounds, pharmaceutical compositions and medicaments of the present invention for use in a method for the treatment of the human (or animal) body by therapy (or for diagnosis).

In one aspect, the invention relates to a compound as defined herein for use in treating, ameliorating and/or preventing a neuromuscular disorder. In one aspect, the invention relates to the use of a compound as defined herein in reversing and/or ameliorating a neuromuscular blockade.

In one aspect, the invention relates to compounds of formula (VII) for use in treating, ameliorating and/or preventing a neuromuscular disorder. In one aspect, the invention relates to compounds of formula (VII) for use in reversing and/or ameliorating a neuromuscular blockade.

In one aspect, the invention relates to a compound of Formula (III), wherein:
R¹ is selected from the group consisting of Cl, Br, and I;
R² is selected from the group consisting of deuterium, F, Cl, and Br;
R³ is selected from the group consisting of C₁₋₅ alkyl, C₂₋₅ alkenyl, C₃₋₅ cycloalkyl and C₅ cycloalkenyl, each of which is substituted with one or more F; and
R⁴ is selected from the group consisting of H or C₁₋₅ alkyl; preferably H;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one aspect, the invention relates to a compound of Formula (IV), wherein:
R¹ is selected from the group consisting of Cl, Br, and I;
R² is selected from the group consisting of deuterium, F, Cl, and Br;
R³ is selected from the group consisting of C₁₋₅ alkyl, C₁₋₅ alkenyl, C₃₋₅ cycloalkyl and C₅ cycloalkenyl, each of which is substituted with one or more F; and
R⁴ is selected from the group consisting of H or C₁₋₅ alkyl; preferably H;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one aspect, the invention relates to a compound of Formula (V), wherein:
- R¹ is Br, Cl or I;
- R² is independently deuterium, F, Cl, or Br;
- R³ is selected from the group consisting of C₁₋₅ alkyl, C₁₋₅ alkenyl, C₃₋₅ cycloalkyl and C₅ cycloalkenyl, each of which is substituted with one or more F; and
- R⁴ is selected from the group consisting of H or C₁₋₅ alkyl; preferably H;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one aspect, the invention relates to a compound of Formula (VI), wherein:
- R¹ is Br, Cl or I;
- R² is independently deuterium, F, Cl, or Br;
- R³ is selected from the group consisting of C₁₋₅ alkyl, C₁₋₅ alkenyl, C₃₋₅ cycloalkyl and C₅ cycloalkenyl, each of which is substituted with one or more F; and
- R⁴ is selected from the group consisting of H or C₁₋₅ alkyl; preferably H;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade.

In one aspect, the invention relates to a compound of Formula (VII), wherein:
- R¹ is selected from the group consisting of F, Cl, Br, and I;
- R² is independently selected from the group consisting of hydrogen, deuterium, F, Cl, and Br;
- R³ is selected from the group consisting of C₁₋₅ alkyl, C₂₋₅ alkenyl, C₃₋₅ cycloalkyl, C₅ cycloalkenyl each of which is substituted with one or more, identical or different, substituents R⁵, and C₂₋₅ alkynyl, which may be optionally substituted with one or more, identical or different, substituents R⁵;
- R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
- R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
- R⁶ is selected from the group consisting of hydrogen and deuterium;
- R⁷ is independently selected from the group consisting of deuterium and F;
- R⁸ is independently selected from the group consisting of deuterium, methoxy, nitro, cyano, Cl, Br, I and F;
- R⁹ is deuterium; and
- n is 0, 1, 2 or 3
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one aspect, the invention relates to a compound of Formula (VII), wherein:
- R¹ is selected from the group consisting of CI and Br;
- R² is selected from the group consisting of F, CI and Br;
- R³ is selected from the group consisting of C₁₋₅ alkyl, C₂₋₅ alkenyl, C₃₋₅ cycloalkyl, C₅ cycloalkenyl each of which is substituted with one or more, identical or different, substituents R⁵, and C₂₋₅ alkynyl, which may be optionally substituted with one or more, identical or different, substituents R⁵;
- R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
- R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
- R⁶ is selected from the group consisting of hydrogen and deuterium;
- R⁷ is independently selected from the group consisting of deuterium and F;
- R⁸ is independently selected from the group consisting of deuterium, methoxy, nitro, cyano, Cl, Br, I and F;
- R⁹ is deuterium; and
- n is 0, 1, 2 or 3
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one aspect, the invention relates to a compound of Formula (VII), wherein:
- R¹ is Br;
- R² is selected from the group consisting of F, CI and Br;
- R³ is methyl substituted with one or more, identical or different, substituents R⁵, C₂₋₃ alkyl optionally substituted with one or more, identical or different, substituents R⁵ or C₃₋₄ cycloalkyl optionally substituted with one or more, identical or different, substituents R⁵;

- R⁴ is H;
- R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
- R⁶ is selected from the group consisting of hydrogen and deuterium;
- R⁷ is independently selected from the group consisting of deuterium and F;
- R⁹ is deuterium; and
- n is 0, 1, 2 or 3
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one aspect, the invention relates to a compound of Formula (VII), wherein:
R¹ is selected from the group consisting of F, Cl, Br, and I;
R² is selected from the group consisting of hydrogen, deuterium, F, Cl, and Br,;
R³ is selected from the group consisting of C₁₋₅ alkyl, C₂₋₅ alkenyl, C₂₋₅ alkynyl, C₃₋₅ cycloalkyl and C₅ cycloalkenyl, each of which is substituted with one or more, identical or different, substituents R⁵; and
   - R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁵ is independently selected from the group consisting of F, OC₁₋₅ alkyl optionally substituted with one or more, identical or different, substituents R⁷ and OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one aspect, the invention relates to a compound of Formula (VII), wherein:
R¹ is Br;
R² is selected from the group consisting of hydrogen, deuterium, F, CI and Br;
R³ is C₁₋₃ alkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
n is 0
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one aspect, the invention relates to a compound of Formula (VII), wherein:
R¹ is Br;
R² is selected from the group consisting of hydrogen, deuterium, F, CI and Br;
R³ is C₃₋₅ cycloalkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
n is 0
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one aspect, the invention relates to a compound of Formula (VII), wherein:
R¹ is selected from the group consisting of F, Cl, Br, I;
R² is selected from the group consisting of hydrogen, deuterium, F, Cl, Br, I;
R³ is selected from the group consisting of C₁₋₅ alkyl and C₃₋₅ cycloalkyl optionally substituted with one or more, identical or different, substituents R⁵;
   - R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁵ is independently selected from the group consisting of F, OC₁₋₅ alkyl optionally substituted with one or more, identical or different, substituents R⁷ and OC₃₋₅ cycloalkyl optionally substituted with one or more, identical or different, substituents R⁷;
R⁶ is deuterium;
R⁷ is independently selected from the group consisting of deuterium and F;
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one aspect, the invention relates to a compound of Formula (VII), wherein:
R¹ is Br;
R² is selected from the group consisting of hydrogen, deuterium, F, CI and Br;
R³ is C₁₋₃ alkyl substituted with one or more, identical or different, substituents R⁵ or C₃₋₄ cycloalkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is deuterium;
R⁷ is independently selected from the group consisting of deuterium and F;
R⁹ is deuterium; and
n is 0, 1, 2 or 3.
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one aspect, the invention relates to a compound of Formula (VII), wherein:
R¹ is selected from the group consisting of F, CI, and Br;
R² is selected from the group consisting of hydrogen, deuterium, F, Cl, and Br;
R³ is selected from the group consisting of fluoromethyl, fluoroethyl and fluoropropyl, each of which may be optionally substituted with one or more deuterium;
   - R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁶ is selected from the group consisting of hydrogen and deuterium;
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof, for use in treating, ameliorating and/or preventing a neuromuscular disorder, and/or for use in reversing and/or ameliorating a neuromuscular blockade;

In one embodiment, the compound for use in treating, ameliorating and/or preventing a neuromuscular disorder, and/or for use in reversing and/or ameliorating a neuromuscular blockade is selected from the group consisting of Compound A-1, Compound A-5, Compound A-7, Compound A-9, Compound A-14, Compound A-15, Compound A-19, Compound A-21, Compound A-22, Compound A-23, Compound A-24, Compound A-26, Compound A-41, and Compound A-42.

In one embodiment, the compound for use in treating, ameliorating and/or preventing a neuromuscular disorder, and/or for use in reversing and/or ameliorating a neuromuscular blockade is selected from the group consisting of:
(2*R*)-2-[4-bromo(3,5-²H₂)phenoxy]-3-fluoropropanoic acid;
(2*R*)-2-[4-bromo(2,6-²H₂)phenoxy]-3-fluoropropanoic acid;
(2*R*)-2-(4-bromo-2-fluorophenoxy)-3,3-difluoropropanoic acid;
(2*R*)-2-(4-bromo-2-fluorophenoxy)-3-fluoropropanoic acid;
(2*R*)-2-(2-bromo-4-chlorophenoxy)-3-fluoropropanoic acid;
(2*R*)-2-(4-chlorophenoxy)-3-fluoropropanoic acid;
(2*R*)-2-(4-chloro-2-fluorophenoxy)-3-fluoropropanoic acid;
(2*R*)-2-(2,4-dibromophenoxy)-3-fluoropropanoic acid;
(2*R*)-2-(4-bromophenoxy)-3-fluoropropanoic acid;
(2*S*,3*E*)-2-(4-bromophenoxy)-4-fluorobut-3-enoic acid;
(2*S*)-2-(2,4-dibromophenoxy)-3-methoxypropanoic acid;
(2*R*)-2-(4-bromo-2-chlorophenoxy)-3-fluoropropanoic acid;
(2*S*)-2-(4-bromo-2-chlorophenoxy)-4-fluorobutanoic acid;
(2*R*)-2-(4-bromophenoxy)-3-fluoro(2-²H)propanoic acid;
(2*S*)-2-(4-bromo-2-fluorophenoxy)-4-fluorobutanoic acid;
(2*S*)-2-(4-bromophenoxy)-4-fluorobutanoic acid;
(2*R*,3*R*)-2-(4-bromophenoxy)-3-fluorobutanoic acid;
(2*R*,3*R*)-2-(4-bromo-2-fluorophenoxy)-3-fluorobutanoic acid;
(2*S*)-2-(4-bromophenoxy)pent-4-ynoic acid;
(2*S*)-2-(2,4-dibromophenoxy)pent-4-ynoic acid;
(2*S*)-2-(4-bromo-2-chlorophenoxy)pent-4-ynoic acid; and
(2*S*)-2-(4-bromo-2-fluorophenoxy)pent-4-ynoic acid.

The compound or the compound for use according to the present invention may be modified in order to increase its half-life when administered to a patient, in particular its plasma half-life.

The compound or the compound for use according to the present invention may further comprise a moiety conjugated to said compound, thus generating a moiety-conjugated compound. Said moiety-conjugated compound may have a plasma and/or serum half-life being longer than the plasma and/or serum half-life of the non-moiety conjugated compound.

The moiety conjugated to the compound or compound for use according to the present invention, may be one or more type(s) of moieties selected from the group consisting of albumin, fatty acids, polyethylene glycol (PEG), acylation groups, antibodies and antibody fragments.

### Neuromuscular disorders

The compound or compound for use of the present invention is used for treating, ameliorating and/or preventing a neuromuscular disorder, or reversing neuromuscular blockade caused by non-depolarizing neuromuscular blocker or antibiotic agent.

The inventors of the present invention have shown that inhibition of CIC-1 channels strengthens neuromuscular transmission. CIC-1 function may therefore contribute to muscle weakness in conditions of compromised neuromuscular transmission.

Thus, in one embodiment of the present invention, the compound or the compound for use as described herein inhibits CIC-1 channels. Thus, it is appreciated that compounds and/or compounds for use of Formula (I) inhibit CIC-1 channels.

The neuromuscular disorder may also include neuromuscular dysfunctions.

Neuromuscular disorders include for example disorders with symptoms of muscle weakness and fatigue. Such disorders may include conditions with reduced neuromuscular transmission safety factor. In one embodiment the neuromuscular disorders are motor neuron disorders. Motor neuron disorders are disorders with reduced safety in the neuromuscular transmission. In one embodiment motor neuron disorders are selected from the group consisting of amyotrophic lateral sclerosis (ALS) (Killian JM, Wilfong AA, Burnett L, Appel SH, Boland D. Decremental motor responses to repetitive nerve stimulation in ALS. Muscle Nerve, 1994, 17, 747-754), spinal muscular atrophy (SMA) (Wadman RI, Vrancken AF, van den Berg LH, van der Pol WL. Dysfunction of the neuromuscular junction in spinal muscular atrophy types 2 and 3. Neurology, 2012, 79, 2050-2055), Charcot-Marie Tooth disease (Bansagi B, Griffin H, Whittaker RG, Antoniadi T, Evangelista T, Miller J, Greenslade M, Forester N, Duff J, Bradshaw A, Kleinle S, Boczonadi V, Steele H, Ramesh V, Franko E, Pyle A, Lochmüller H, Chinnery PF, Horvath R. Genetic heterogeneity of motor neuropathies. Neurology, 2017, 28;88(13):1226-1234), X-linked spinal and bulbar muscular atrophy (Yamada, M., Inaba, A., Shiojiri, T. X-linked spinal and bulbar muscular atrophy with myasthenic symptoms. Journal of the Neurological Sciences, 1997, 146, 183-185), Kennedy's disorder (Stevic, Z., Peric, S., Pavlovic, S., Basta, I., Lavrnic, D., Myasthenic symptoms in a patient with Kennedy's disorder. Acta Neurologica Belgica, 2014, 114, 71-73), multifocal motor neuropathy (Roberts, M., Willison, H.J., Vincent, A., Newsom-Davis, J. Multifocal motor neuropathy human sera block distal motor nerve conduction in mice. Ann Neurol. 1995, 38, 111-118), Guillain-Barré syndrome (Ansar, V., Valadi, N. Guillain-Barré Syndrome Prim. Care, 2015, 42, 189-193; poliomyelitis (Trojan, D.A., Gendron, D., Cashman, N.R. Electrophysiology and electrodiagnosis of the post-polio motor unit. Orthopedics, 1991, 14, 1353-1361, and Birk T.J. Poliomyelitis and the post-polio syndrome: exercise capacities and adaptation - current research, future directions, and widespread applicability. Med. Sci. Sports Exerc., 1993, 25, 466-472), post-polio syndrome (Garcia, C.C., Potian, J.G., Hognason, K., Thyagarajan, B., Sultatos, L.G., Souayah, N., Routh, V.H., McArdle, J.J. Acetylcholinesterase deficiency contributes to neuromuscular junction dysfunction in type 1 diabetic neuropathy. Am. J. Physiol. Endocrinol. Metab., 2012, 15, E551 - 561) and sarcopenia (Gilmore K.J., Morat T., Doherty T.J., Rice C.L., Motor unit number estimation and neuromuscular fidelity in 3 stages of sarcopenia. 2017 55(5):676-684).

Thus, in one preferred embodiment of the present invention the neuromuscular disorder is amyotrophic lateral sclerosis (ALS). In another preferred embodiment the neuromuscular disorder is spinal muscular atrophy (SMA). In another preferred embodiment the neuromuscular disorder is Charcot-Marie tooth disease (CMT). In another preferred embodiment the neuromuscular disorder is sarcopenia. In yet another preferred embodiment, the neuromuscular disorder is critical illness myopathy (CIM).

As stated above the neuromuscular disorders include for example disorders with symptoms of muscle weakness and fatigue. Such disorder may for example include diabetes (Burton, A. Take your pyridostigmine: that's an (ethical?) order! Lancet Neurol., 2003, 2, 268).

In one embodiment the compound or the compound for use of the present invention is used to prevent neuromuscular disorder. The compound or the compound for use may for example be used prophylactically against nerve gas that is known to cause symptoms of muscle weakness and fatigue (Kawamura, Y., Kihara, M., Nishimoto, K., Taki, M. Efficacy of a half dose of oral pyridostigmine in the treatment of chronic fatigue syndrome: three case reports. Pathophysiology, 2003, 9, 189-194.

In another embodiment the neuromuscular disorders is chronic fatigue syndrome. Chronic fatigue syndrome (CFS) (Fletcher, S.N., Kennedy, D.D., Ghosh, I.R., Misra, V.P., Kiff, K., Coakley, J.H., Hinds, C.J. Persistent neuromuscular and neurophysiologic abnormalities in long-term survivors of prolonged critical illness. Crit. Care Med. 2003, 31, 1012 - 1016) is the common name for a medical condition characterized by debilitating symptoms, including fatigue that lasts for a minimum of six months in adults. CFS may also be referred to as systemic exertion intolerance disorder (SEID), myalgic encephalomyelitis (ME), post-viral fatigue syndrome (PVFS), chronic fatigue immune dysfunction syndrome (CFIDS), or by several other terms.

Symptoms of CFS include malaise after exertion; unrefreshing sleep, widespread muscle and joint pain, physical exhaustion, and muscle weakness.

In a further embodiment the neuromuscular disorder is a critical illness polyneuropathy (Angelini C. Spectrum of metabolic myopathies. Biochim. Biophys. Acta., 2015, 1852, 615 - 621) or CIM (Latronico, N., Bolton, C.F. Critical illness polyneuropathy and myopathy: a major cause of muscle weakness and paralysis. Lancet Neurol. 2011, 10, 931-941). Critical illness polyneuropathy and CIM are overlapping syndromes of widespread muscle weakness and neurological dysfunction developing in critically ill patients.

The neuromuscular disorder may also include metabolic myopathy (Milone, M., Wong, L.J. Diagnosis of mitochondrial myopathies. Mol. Genet. Metab., 2013, 110, 35 - 41) and mitochondrial myopathy (Srivastava, A., Hunter, J.M. Reversal of neuromuscular block. Br. J. Anaesth. 2009, 103, 115 - 129). Metabolic myopathies result from defects in biochemical metabolism that primarily affects muscle. These may include glycogen storage disorders, lipid storage disorder and 3-phosphocreatine stores disorder. Mitochondrial myopathy is a type of myopathy associated with mitochondrial disorder. Symptoms of mitochondrial myopathies include muscular and neurological problems such as muscle weakness, exercise intolerance, hearing loss and trouble with balance and coordination.

In another embodiment the neuromuscular disorder is periodic paralysis, in particular hypokalemic periodic paralysis which is a disorder of skeletal muscle excitability that presents with recurrent episodes of weakness, often triggered by exercise, stress, or carbohydrate-rich meals (Wu, F., Mi, W., Cannon, S.C., Neurology, 2013, 80, 1110-1116 and Suetterlin, K. et at, Current Opinion Neurology, 2014, 27, 583-590) or hyperkalemic periodic paralysis which is an inherited autosomal dominant disorder that affects sodium channels in muscle cells and the ability to regulate potassium levels in the blood (Ammat, T. et at, Journal of General Physiology, 2015, 146, 509-525). In a preferred embodiment the neuromuscular disorder is a myasthenic condition. Myasthenic conditions are characterized by muscle weakness and neuromuscular transmission failure. Congenital myasthenia gravis (Finlayson, S., Beeson, D., Palace, J. Congenital myasthenic syndromes: an update. Pract. Neurol., 2013, 13, 80 - 91) is an inherited neuromuscular disorder caused by defects of several types at the neuromuscular junction.

Myasthenia gravis and Lambert-Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107) are examples of myasthenic conditions. Myasthenia gravis is either an autoimmune or congenital neuromuscular disorder that leads to fluctuating muscle weakness and fatigue. In the most common cases, muscle weakness is caused by circulating antibodies that block ACh receptors at the postsynaptic neuromuscular junction, inhibiting the excitatory effects of the neurotransmitter ACh on nicotinic ACh-receptors at neuromuscular junctions (Gilhus, N.E., Owe, J.F., Hoff, J.M., Romi, F., Skeie, G.O., Aarli, J.A. Myasthenia Gravis: A Review of Available Treatment Approaches, Autoimmune Diseases, 2011, Article ID 84739). Lambert-Eaton myasthenic syndrome (also known as LEMS, Lambert-Eaton syndrome, or Eaton-Lambert syndrome) is a rare autoimmune disorder that is characterized by muscle weakness of the limbs. It is the result of an autoimmune reaction in which antibodies are formed against presynaptic voltage-gated calcium channels, and likely other nerve terminal proteins, in the neuromuscular junction.

Thus, in one embodiment of the present invention the neuromuscular disorder is myasthenia gravis. In another preferred embodiment the neuromuscular disorder is Lambert-Eaton syndrome.

Neuromuscular blockade is used in connection with surgery under general anaesthesia. Reversing agents are used for more rapid and safer recovery of muscle function after such blockade. Complications with excessive muscle weakness after blockade during surgery can result in delayed weaning from mechanical ventilation and respiratory complications after the surgery. Since such complications have pronounced effects on outcome of the surgery and future quality of life of patients, there is a need for improved reversing agents (Murphy GS, Brull SJ. Residual neuromuscular block: lessons unlearned. Part I: definitions, incidence, and adverse physiologic effects of residual neuromuscular block. Anesth Analg. 2010 111(1):120-8). Thus, in one embodiment, the neuromuscular disorder has been induced by a neuromuscular blocking agent. In one particular embodiment the neuromuscular disorder is muscle weakness caused by neuromuscular blockade after surgery. In another preferred embodiment of the present invention the compound or the compound for use is used for reversing and/or ameliorating neuromuscular blockade after surgery. In one embodiment, the neuromuscular blockade is drug induced. In one embodiment the neuromuscular blockade is induced by an antibiotic. In one embodiment the neuromuscular blockade is induced by a non-depolarizing neuromuscular blocker.

### Pharmaceutical formulations

In one embodiment, a composition comprising the compound or the compound for use, according to the present invention, is provided. The composition according to the present invention is used for treating, ameliorating and/or preventing a neuromuscular disorder, and/or for use in reversing and/or ameliorating a neuromuscular blockade. Thus, it is preferred that the compositions and compounds described herein are pharmaceutically acceptable. In one embodiment the composition as described herein is in the form of a pharmaceutical formulation. In one embodiment, the composition as described herein further comprises a pharmaceutically acceptable carrier.

### Combination therapy

The composition of the present invention may comprise further active ingredients/agents or other components to increase the efficiency of the composition. Thus, in one embodiment the composition further comprises at least one further active agent. It is appreciated that the active agent is suitable for treating, preventing or ameliorating said neuromuscular disorder.

The active agent is in a preferred embodiment an acetylcholine esterase inhibitor. Said acetylcholine esterase inhibitor may for example be selected from the group consisting of delta-9-tetrahydrocannabinol, carbamates, physostigmine, neostigmine, pyridostigmine, ambenonium, demecarium, rivastigmine, phenanthrene derivatives, galantamine, piperidines, donepezil, tacrine, edrophonium, huperzine, ladostigil, ungeremine and lactucopicrin.

Preferably the acetylcholine esterase inhibitor is selected from the group consisting of neostigmine, physostigmine and pyridostigmine. It is preferred that the acetylcholine esterase inhibitor is neostigmine or pyridostigmine.

The active agent may also be an immunosuppressive drug. Immunosuppressive drugs are drugs that suppress or reduce the strength of the body's immune system. They are also known as anti-rejection drugs. Immunosuppressive drugs include but are not limited to glucocorticoids, corticosteroids, cytostatics, antibodies and drugs acting on immunophilins. In one embodiment the active agent is prednisone.

The active agent may also be an agent that is used in anti-myotonic treatment. Such agents include for example blockers of voltage gated Na⁺ channels, and aminoglycosides.

The active agent may also be an agent for reversing a neuromuscular blockade after surgery. Such agents include for example neostigmine or sugammadex (Org 25969, tradename Bridion). The active agent may also be an agent for increasing the Ca²⁺ sensitivity of the contractile filaments in muscle. Such agents include tirasemtiv and CK-2127107 (Hwee, D.T., Kennedy, A.R., Hartman, J.J., Ryans, J., Durham, N., Malik, F.I., Jasper, J.R. The small-molecule fast skeletal troponin activator, CK-2127107, improves exercise tolerance in a rat model of heart failure. Journal of Pharmacology and Experimental Therapeutics, 2015, 353, 159 - 168).

The active agent may also be an agent for increasing ACh release by blocking voltage-gated K⁺ channels in the pre-synaptic terminal. Such agent includes 3,4-aminopyridine.

### Method of manufacturing

Methods of manufacturing compounds or compounds for use according to formula (VII) are described:
One method for manufacturing the compounds or compounds for use according to the present invention comprises the steps of reacting a compound having formula (VIII) 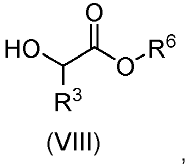 wherein R³ is as defined herein and R⁶ is a protecting group, such as selected from the group consisting of alkyl, alkenyl, akynyl, cycloalkyl, cycloalkenyl, aromatic ring, heteroaromatic ring and -alkylene-Si-alkyl, with first a reagent capable of converting the alcohol (OH) into a leaving group and secondly with a compound having formula (VII) 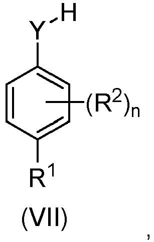 wherein R¹, R², and n are as defined herein and Y is O to generate a compound having formula (IX) 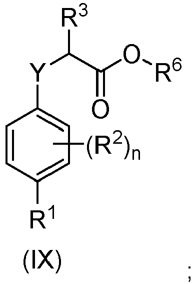 and
reacting the product compound of a) with an ester hydrolysing reagent thus generating a compound as defined herein.

A second method for manufacturing the compounds or compounds for use according to the present invention comprises the steps of
reacting a compound having formula (XI) 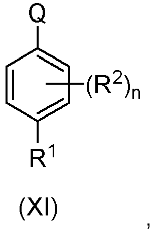 wherein R¹, R² and n are as defined herein and Q is a leaving group, such as selected from the group consisting of fluorine and iodine, with a compound having formula (XII) 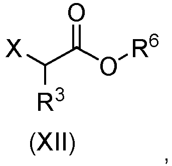 wherein R³ is as defined herein, and R⁶ is a protecting group, such as selected from the group consisting of alkyl, alkenyl, akynyl, cycloalkyl, cycloalkenyl, aromatic ring, heteroaromatic ring and -alkylene-Si-alkyl to generate a compound having formula (X) 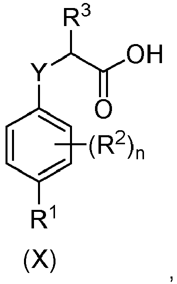 wherein Y is O; and
b. reacting the product compound of a) with an ester hydrolysing reagent thus generating a compound as defined herein.

Yet a third method for manufacturing the compounds or compounds for use according to the present invention comprises the steps of
reacting a compound having formula (XIII) 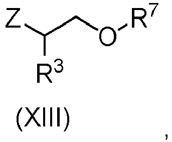 wherein R³ is as defined herein, Z is OH and R⁷ is a protecting group, such as an -Si-alkyl, with first a reagent capable of converting the alcohol (Z) into a leaving group and secondly with a compound having formula (VII) 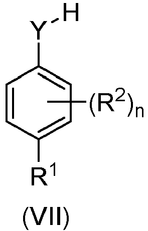 wherein R¹, R² and n are as defined herein, and Y is O to generate a compound having formula (XIV) 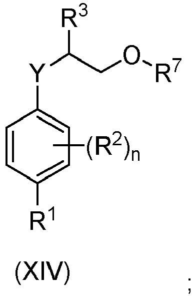 reacting the product compound of a) with an ether cleaving reagent to generate a compound having formula (XV) 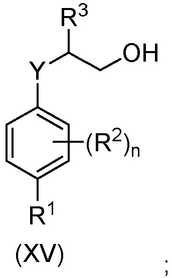 and
reacting the product compound of b) with an oxidising agent thus generating a compound as defined herein.

### Examples

### Materials and methods

### Chemicals

Some of the compounds for testing were obtained from a range of different suppliers including Enamine, Vitas, and CanAm Bioresearch. For synthesis of particular compounds please see below.

### General synthetic strategies

Compounds of Formula (I) may be synthesized by the following synthetic strategies, general methods A-C:

### NMR Spectra

¹H-NMR spectra were recorded either on a Bruker AM-300 spectrometer and were calibrated using residual nondeuterated solvent as internal reference. Spectra were processed using Spinworks version 4.0 (developed by Dr. Kirk Marat, Department of Chemistry, University of Manitoba), or on a Bruker 400 MHZ Ultrashield plus equipped with probe BBO 400MHz S1 5mm with Z gradient probe or a Bruker 500 MHz Avance III HD spectrometer, equipped with a Bruker 5mm SmartProbeTM, calibrated using residual non-deuterated solvent as internal reference and spectra processed using topspin version 3.2.7.

### HPLC method 1

The product was analysed by Waters 2695 HPLC consisting of a Waters 996 photodiode array detector, Kromasil Eternity C18, 5 µm, 4.6 X 150 mm column. Flow rate: 1 mL/minute, run time 20 minutes. Solvent A: methanol; solvent B: 0.1% formic acid in water. Gradient 0-100 % Solvent B over 15 minutes with monitoring at 280 nm.

### HPLC method 2

Waters Acquity UPLC, X-Select; column: Waters X-Select UPLC C18, 1.7 µm, 2.1 × 30mm. Solvent A: 0.1% formic acid in water; solvent B: 0.1% formic acid in MeCN. Gradient 5-95 % Solvent B over 10 minutes; detector: diode array.

### HPLC method 3

Waters Acquity UPLC, X-Select; column: Waters X-Select UPLC C18, 1.7 µm, 2.1 × 30mm. Solvent A: 0.1% formic acid in water; solvent B: 0.1% formic acid in MeCN. Gradient 5-95 % Solvent B over 3 minutes; detector: diode array.

### General Method A

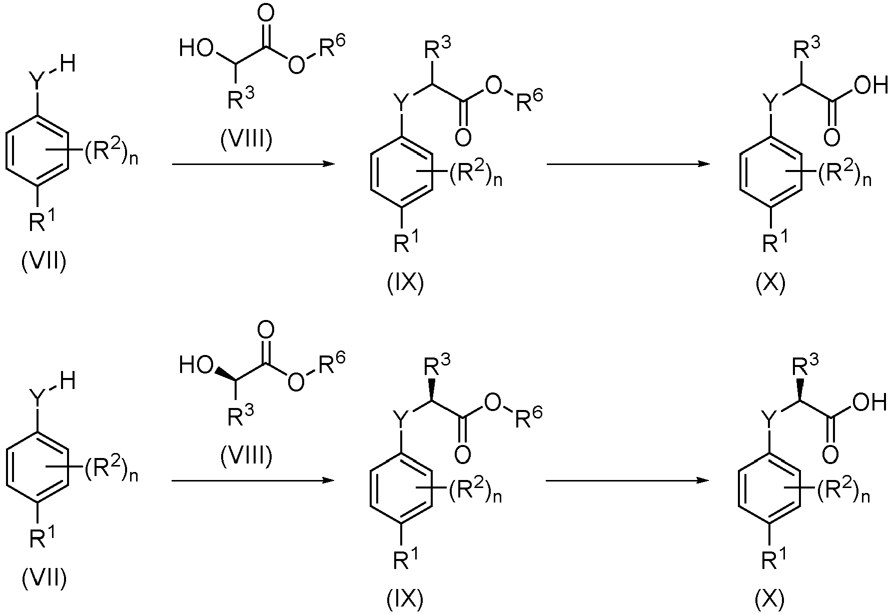

Method A involves the synthesis of Formula (X) (which is the same as Formula (I) in which R⁴ is H), which is an ether structure when Y = oxygen, and -R¹, -R² and -R³ are as defined in Formula (I) above. Compound (VII), in the case where Y = O is a phenol, is available either commercially or synthetically, and can be converted into an ether (IX) by methods which include Mitsunobu reaction conditions and compounds of Formula (VIII). This ether contains an ester functionality -CO₂R⁶, which can be hydrolysed under a range of standard conditions, involving acid or base, to provide the carboxylic acid structure (X), Y = O. These standard conditions can also for example involve an enzymic hydrolysis, employing for example an esterase or lipase. If an ester molecule (IX) includes for example a (CH₃)₃*S*iCH₂CH₂O- group as -OR⁶, then a fluoride ion source such as tetra-n-butylammonium fluoride can be employed to convert (IX) into the corresponding carboxylic acid (X). Some compounds can optionally be tested without hydrolysis of the ester group, and in this case R⁶ is equivalent to R⁴ as defined in Formula (I) above.

Substituted phenols of general formula (VII), Y = O, can be prepared by a variety of standard methods, for example by an ester rearrangement in the Fries rearrangement, by a rearrangement of *N*-phenylhydroxylamines in the Bamberger rearrangement, by hydrolysis of phenolic esters or ethers, by reduction of quinones, by replacement of an aromatic amine or by a hydroxyl group with water and sodium bisulfide in the Bucherer reaction. Other methods include hydrolysis of diazonium salts, by rearrangement reaction of dienones in the dienone phenol rearrangement, by the oxidation of aryl silanes, by the Hock process.

If a salt form of the product carboxylic acid is required, and alkali metal salt, e.g. a sodium salt can be prepared utilizing for example one equivalent of sodium hydroxide or sodium bicarbonate in aqueous solvent. This procedure also applies to General Methods B and C.

### General Method B

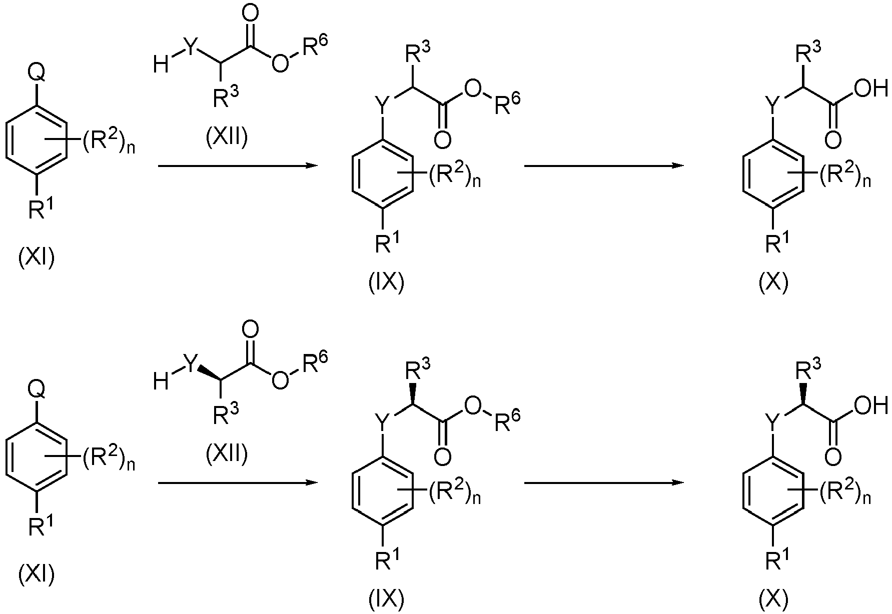

Carboxylic acids of Formula (X) (which is the same as Formula (I) in which R⁴ is H), can also be prepared by the procedure illustrated as General Method B. A phenolic ether of Formula (IX) can be prepared by displacement of a suitable leaving group Q in (XI). Q can for example be a halogen such as fluorine or iodine, and the ether product of formula (IX) can be converted into the carboxylic acid derivative (X) by one of a range of methods outlined in Method A, involving hydrolysis of the ester functionality. Some compounds can optionally be tested without hydrolysis of the ester group, and in this case R⁶ is equivalent to R⁴ as defined in Formula (I) above.

### General Method C

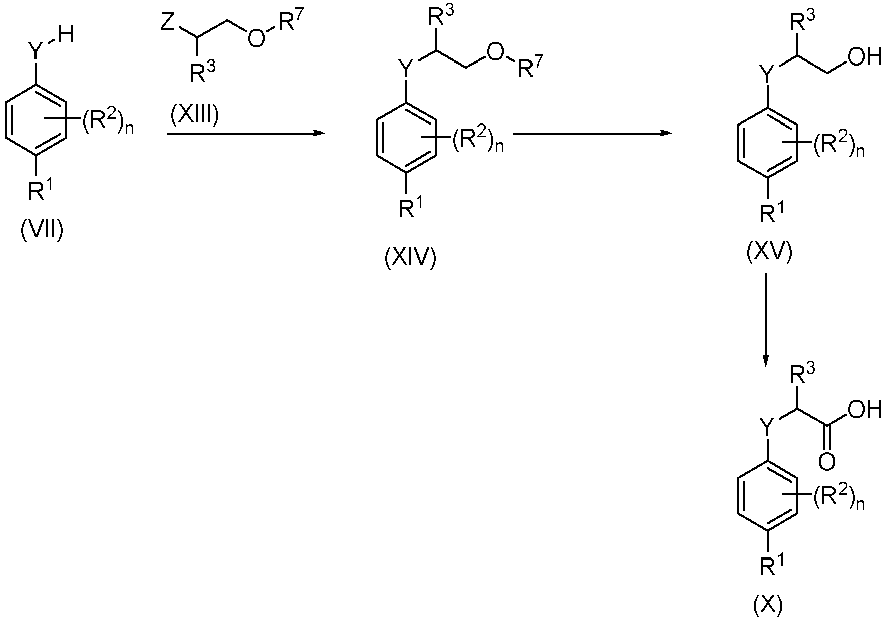

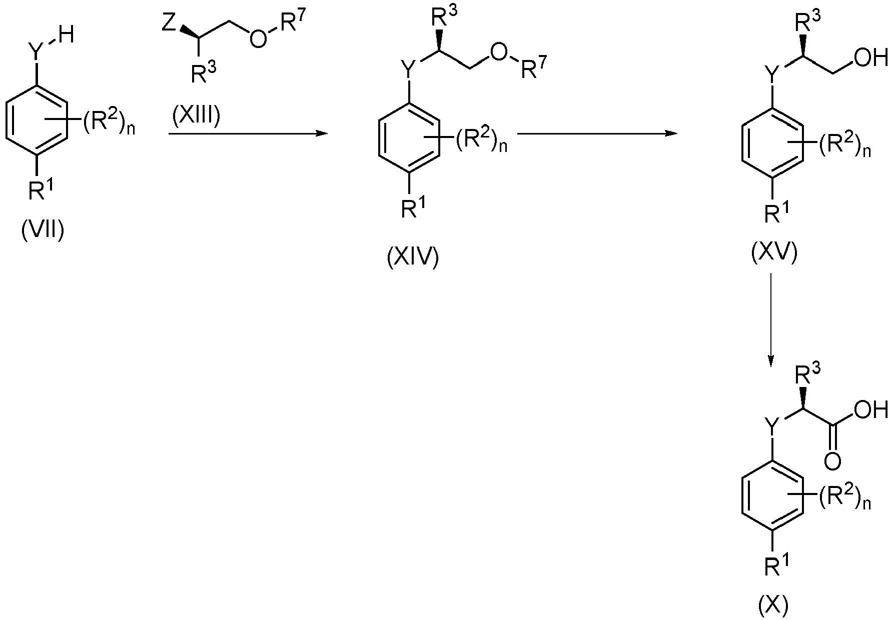

Carboxylic acids of Formula (X) (which is the same as Formula (I) in which R⁴ is H), can be prepared by the procedure illustrated as General Method C. A phenolic ether of formula (XIV) can be prepared by utilising e.g. Mitsunobu conditions when (VII) is a phenol structure, i.e. Y = O, and XIII is a suitable secondary alcohol, i.e. Z = OH, and - R⁷ is a suitable protecting group, such as a silyl-containing moiety. On removal of the protecting group -R⁶ the primary alcohol (XV) can be oxidised to a carboxylic acid under standard conditions involving potassium permanganate, Jones oxidation conditions, the Heyns oxidation, ruthenium tetroxide or TEMPO.

Table 1 below illustrates Example compounds defined by the general Formula (I). In table 1, the HPLC System is one of the methods as defined in the Materials and methods section.

**Table 1: Example of General Formula (I)**

| **Compound Number** | **IUPAC name** | **¹H NMR** | **HPLC retention time** | **Synthesis method** |
|---|---|---|---|---|
| A-1 | (*2R*)-2-[4-bromo(3,5-²H₂)phenoxy]-3-fluoropropanoic acid | 1H NMR (500 MHz, DMSO-d6) δ 13.53 (s, 1H), 6.92 (s, 2H), 5.19 (ddd, J = 29.2, 4.1, 2.4 Hz, 1H), 4.95 - 4.74 (m, 2H). | 2.911 (2) | C |
| A-2 | (2*S*)-2-[4-bromo(3,5-²H₂)phenoxy]propanoic acid | 1H NMR (500 MHz, DMSO-d6) δ 13.08 (s, 1H), 6.85 (s, 2H), 4.84 (q, J = 6.8 Hz, 1H), 1.50 (d, J = 6.8 Hz, 3H). | 3.036 (2) | A |
| A-3 | ethyl (2*S*)-2-(4-bromo-2-fluorophenoxy)-3-methylbut-3-enoate | 1H NMR (300 MHz, CDCl3) δ 7.29 - 7.13 (m, 2H), 5.21 (d, 1H), 5.04 (s, 1H), 1.88 (s, 3H),1.28 (t, 3H). | 13.499 (1) | A |
| A-4 | (2*R*)-2-(4-bromo-2-fluorophenoxy)-3-methylbutanoic acid | 1H NMR (500 MHz, DMSO-d6) δ 13.15 (s, 1H), 7.54 (dd, J = 10.9, 2.4 Hz, 1H), 7.31 (ddd, J = 8.8, 2.4, 1.5 Hz, 1H), 6.98 (t, J = 9.0 Hz, 1H), 4.62 (d, J = 4.6 Hz, 1H), 2.25 (pd, J = 6.8, 4.5 Hz, 1H), 1.03 (d, J = 6.9 Hz, 3H), 1.01 (d, J = 6.8 Hz, 3H). | 4.358 (2) | A |
| A-5 | (2*R*)-2-[4-bromo(2,6-²H₂)phenoxy]-3-fluoropropanoic acid | 1H NMR (500 MHz, DMSO-d6) δ 13.54 (s, 1H), 7.46 (s, 2H), 5.17 (d, J = 28.7 Hz, 1H), 4.96 - 4.75 (m, 2H). | 2.852 (2) | C |
| A-6 | (2*S*)-2-[4-bromo(2,6-²H₂)phenoxy]propanoic acid | 1H NMR (400 MHz, DMSO-d6) δ 13.17 (s, 1H), 7.44 (s, 2H), 4.88 - 4.76 (m, 1H), 1.49 (d, J = 6.8 Hz, 3H). | 3.028 (2) | A |
| A-7 | (2*S*)-2-(4-bromophenoxy)-3-fluoropropanoic acid | 1H NMR (400 MHz, DMSO-d6) δ 13.53 (s, 1H), 7.51 - 7.41 (m, 2H), 6.97 - 6.88 (m, 2H), 5.20 (ddd, J = 29.2, 4.0, 2.5 Hz, 1H), 4.96 - 4.72 (m, 2H). | 2.970 (2) | C |
| A-8 | (2*S*)-2-(4-bromo-2-iodophenoxy)propanoic acid | ¹H NMR (300 MHz, CDCl₃) δ 10.38-9.28 (br, 1H); 7.93 (d, 1H); 7.39 (dd, 1H); 6.63 (d, 1H); 4,78 (q, 1H); 1.75 (d, 3H). | 14.547 (1) | A |
| A-9 | (2*R*)-2-(4-bromo-2-fluorophenoxy)-3,3-difluoropropanoic acid | 1H NMR (400 MHz, DMSO-d6) δ 7.58 (dd, J = 10.8, 2.4 Hz, 1H), 7.36 - 7.29 (m, 1H), 7.13 (t, J = 9.0 Hz, 1H), 6.51 (td, J = 53.1, 2.1 Hz, 1H), 5.30 (dd, J = 20.1, 7.9 Hz, 1H). | 3.424 (2) | C |
| A-10 | (2*S*)-2-{4-bromo-2-[(1E)-(methoxyimino)methyl]p henoxy}propanoic acid | 1H NMR (300 MHz, CDCl3) δ 7.40 (d, 1H), 4.80 (q, 1H), 3.99 (s, 3H), 1.69 (d, 3H). | 14.473 (1) | A |
| A-11 | (2*S*)-2-(2-bromo-4-chlorophenoxy)-3-methylbutanoic acid | 1H NMR (300 MHz, CDCl3) δ 6.68 (d, 1H), 4.48 (d, 1H), 2.41 (m, 1H), 1.18 (d, 3H), 1.16 (d, 3H); | 15.404 (1) | A |
| A-12 | (2*S*)-2-(2-fluoro-4-iodophenoxy)propanoic acid | 1H-NMR (300 MHz, CDCl3): 10.50-10.12 (br, 1H), 7.40 (dd, 2H), 4.79 (q, 1H), 1.69 (d, 3H). | 13.629 (1) | A |
| A-13 | (2*S*)-2-(2-bromo-4-iodophenoxy)propanoic acid | 1H-NMR (300 MHz, CDCl3): δ 10.48-10.09 (br, 1H), 7.88 (s, 1H), 4.79 (q, 1H), 1.73 (d, 3H). | 14.713 (1) | A |
| A-14 | ethyl 2-(4-bromo-2-fluorophenoxy)-3,3,3-trifluoropropanoate | 1H NMR (300 MHz, CDCl3) δ 6.99 (t, 1H), 4.94 (q, 1H), 4.41 - 4.29 (m, 2H), 1.32 (t, 3H). | 18.077 (1) | A |
| A-15 | ethyl 2-(4-bromophenoxy)-3,3,3-trifluoropropanoate | 1H NMR (300 MHz, CDCl3) δ 7.48-7.40 (m, 2H), 4.93 (q, 1H), 4.40 - 4.30 (m, 2H), 1.31 (t, 3H). | 17.92 (1) | A |
| A-16 | (2*S*)-2-(2-chloro-4-iodophenoxy)propanoic acid | 1H-NMR (300 MHz, CDCl3): δ 10.38-10.19 (br, 1H), 7.72 (s, 1H), 4.79 (q, 1H), 1.73 (d, 3H). | 14.528 (1) | A |
| A-17 | (2*S*)-2-(2-bromo-4-chlorophenoxy)propanoi c acid | δ 7.58 (d, 1H), 7.28 (dd, 1H), 6.90 (d, 1H), 4.85 (m, 1H), 1.64 (d, 3H). | 14.188 (1) | A |
| A-18 | 2-(4-bromophenoxy)-2-cyclopentylacetic acid | ¹H NMR (300 MHz, CDCl₃) δ 9.93-9.27 (br, 1H); 7.38 (d, 2H); 6.79 (d, 2H); 4.47 (d, 1H); 2.58-2.40 (m, 1H); 1.94-1.44 (m, 8H). | 15.452 (1) | A |
| A-19 | (*2R*)-2-(4-bromo-2-fluorophenoxy)-3-fluoropropanoic acid | 1H NMR (400 MHz, DMSO-d6) δ 7.58 (dd, J = 10.9, 2.4 Hz, 1H), 7.33 (ddd, J = 8.8, 2.4, 1.5 Hz, 1H), 7.11 (t, J = 9.0 Hz, 1H), 5.30 (ddd, J = 29.3, 3.9, 2.4 Hz, 1H), 5.02 - 4.77 (m, 2H). | 3.109 (2) | C |
| A-20 | (2*S*)-2-(4-chloro-2-fluorophenoxy)-3-methylbutanoic acid | 1H NMR (400 MHz, DMSO-d6) δ 13.19 (s, 1H), 7.44 (dd, J = 11.1, 2.6 Hz, 1H), 7.19 (ddd, J = 8.9, 2.6, 1.6 Hz, 1H), 7.03 (t, J = 9.0 Hz, 1H), 4.62 (d, J = 4.6 Hz, 1H), 2.25 (pd, J = 6.9, 4.6 Hz, 1H), 1.02 (app dd, J = 6.8, 6.0 Hz, 6H). | 4.148 (2) | A |
| A-21 | (*2R*)-2-(2-bromo-4-chlorophenoxy)-3-fluoropropanoic acid | 1H NMR (400 MHz, DMSO-d6) δ 13.71 (s, 1H), 7.73 (d, J = 2.6 Hz, 1H), 7.40 (dd, J = 8.9, 2.6 Hz, 1H), 7.07 (d, J = 8.9 Hz, 1H), 5.32 (ddd, J = 29.1, 4.0, 2.4 Hz, 1H), 5.00 - 4.77 (m, 2H). | 1.936 (2) | C |
| A-22 | (2*R*)-2-(4-chlorophenoxy)-3-fluoropropanoic acid | 1H NMR (400 MHz, DMSO-d6) δ 13.47 (s, 1H), 7.38 - 7.30 (m, 2H), 7.02 - 6.93 (m, 2H), 5.20 (ddd, J = 29.1, 3.9, 2.5 Hz, 1H), 4.97 - 4.72 (m, 2H). | 1.088 (2) | C |
| A-23 | (2*R*)-2-(4-chloro-2-fluorophenoxy)-3-fluoropropanoic acid | 1H NMR (400 MHz, DMSO-d6) δ 13.60 (s, 1H), 7.47 (dd, J = 11.2, 2.4 Hz, 1H), 7.21 (ddd, J = 8.9, 2.4, 1.0 Hz, 1H), 7.16 (t, J = 8.8 Hz, 1H), 5.30 (ddd, J = 29.3, 3.9, 2.4 Hz, 1H), 5.03 - 4.70 (m, 2H). | 1.188 (2) | C |
| A-24 | (2*R*)-2-(2,4-dibromophenoxy)-3-fluoropropanoic acid | 1H NMR (400 MHz, DMSO-d6) δ 13.63 (s, 1H), 7.83 (d, J = 2.4 Hz, 1H), 7.52 (dd, J = 8.8, 2.4 Hz, 1H), 7.01 (d, J = 8.9 Hz, 1H), 5.32 (ddd, J = 29.0, 3.9, 2.4 Hz, 1H), 5.02 - 4.76 (m, 2H). | 2.041 (2) | C |
| A-25 | (2*S*)-2-(4-bromophenoxy)-3-hydroxypropanoic acid | 1H NMR (400 MHz, DMSO-d6) δ 7.47 - 7.41 (m, 2H), 6.90 - 6.83 (m, 2H), 4.74 (dd, J = 5.1, 3.8 Hz, 1H), 3.86 - 3.78 (m, 2H). | 0.942 (2) | A |
| A-26 | (2*R*)-2-(4-bromophenoxy)-3-fluoropropanoic acid | 1H NMR (400 MHz, DMSO-d6) δ 13.51 (s, 1H), 7.49 - 7.43 (m, 2H), 6.96 - 6.89 (m, 2H), 5.24 - 5.12 (m, 1H), 4.95 - 4.75 (m, 2H). | 2.913 (2) | C |
| A-27 | (2*S*)-2-(4-bromo-2-fluorophenoxy)-3-methylbutanoic acid | 1H NMR (400 MHz, DMSO-d6) δ 13.19 (s, 1H), 7.54 (dd, J = 10.9, 2.4 Hz, 1H), 7.31 (ddd, J = 8.9, 2.4, 1.5 Hz, 1H), 6.98 (t, J = 9.0 Hz, 1H), 4.61 (d, J = 4.5 Hz, 1H), 2.31-2.20 (m, 1H), 1.02 (app dd, J = 6.9, 6.0 Hz, 6H). | 4.343 (2) | A |
| A-28 | (2*R*)-2-(4-bromo-2-fluorophenoxy)propanoi c acid | 1H NMR (400 MHz, DMSO-d6) δ 13.21 (s, 1H), 7.55 (dd, J = 10.9, 2.4 Hz, 1H), 7.31 (ddd, J = 8.8, 2.4, 1.5 Hz, 1H), 7.00 (t, J = 9.0 Hz, 1H), 4.95 (q, J = 6.8 Hz, 1H), 1.53 (d, J = 6.8 Hz, 3H). | 3.207 (2) | A |
| A-29 | (2*R*)-2-(4-chlorophenoxy)propanoi c acid | 1H NMR (400 MHz, DMSO-d6) δ 13.10 (s, 1H), 7.38 - 7.26 (m, 2H), 6.94 - 6.84 (m, 2H), 4.84 (q, J = 6.8 Hz, 1H), 1.50 (d, J = 6.8 Hz, 3H). | 2.825 (2) | A |
| A-30 | (2*S*)-2-(3-bromo-4-chlorophenoxy)propanoi c acid | 1H NMR (400 MHz, DMSO-d6) δ 13.16 (s, 1H), 7.52 (d, J = 8.9 Hz, 1H), 7.29 (d, J = 3.0 Hz, 1H), 6.95 (dd, J = 8.9, 3.0 Hz, 1H), 4.96 (q, J = 6.7 Hz, 1H), 1.50 (d, J = 6.8 Hz, 3H). | 3.683 (2) | A |
| A-31 | (2*S*)-2-(4-bromo-2-fluorophenoxy)propanoi c acid | 1H NMR (Chloroform-d, 400 MHz) δ 7.29 (0.5H, d, J=2.3 Hz), 7.26 (0.5H, d, J=2.3 Hz), 7.19 (0.5H, dd, J=2.3, 1.6 Hz), 7.17 (0.5H, dd, J=2.3, 1.6 Hz), 6.85 (1H, t, J=8.7 Hz), 4.78 (1H, q, J=6.9 Hz), 1.69 (3H, d, J=6.9 Hz) | 1.283 (3) | A |
| A-32 | (2*S*)-2-[4-(trifluoromethyl)phenoxy ]propanoic acid | 1H NMR (400 MHz, CDCl₃) δ 7.56 (2H, d, J=8.5 Hz), 6.96 (2H, d, J=8.5 Hz), 4.86 (1H, q, J=6.9 Hz), 1.70 (3H, d, J=6.9 Hz) | 1.302 (3) | A |
| A-33 | sodium (2*S*)-2-(4-chlorophenoxy)-5-methylhexanoate | 1H-NMR (300 MHz, CD₃OD): δ 7.18 (d, 2H), 4.29 (t, 1H), 1.97-1.84 (m, 2H), 0.93 (d, 6H). | 15.797 (1) | A |
| A-34 | methyl (2*S*)-2-(4-chlorophenoxy)-5-methylhexanoate | 1H-NMR (300 MHz, CDCl3): δ 7.23 (d, 2H), 4.56 (t, 1H), 3.76 (s, 3H), 1.68-1.51 (m, 1H), 1.49-1.26 (m, 2H), 0.93 (d, 6H). | 16.382 (1) | A |
| A-35 | sodium (2*S*)-2-(4-chlorophenoxy)-4-methylpentanoate | 1H NMR (300 MHz, CDCl3) δ 6.84 (d, 2H), 4.68-4.59 (m, 1H), 1.01 (d, 3H), 0.95 (d, 3H); | 15.306 (1) | A |
| A-36 | sodium (2*S*)-2-(4-chlorophenoxy)hexanoic acid | 1H NMR (300 MHz, CD3OD) δ 7.17 (d, 2H), 4.31 (t, 1H), 1.88 (q, 2H), 1.60 - 1.30 (m, 4H), 0.92 (t, 3H). | 15.39 (1) | A |
| A-37 | methyl (2*S*)-2-(4-chlorophenoxy)hexanoat e | 1H NMR (300 MHz, CDCl3) δ 6.82 (d, 2H), 4.57 (t, 1H), 3.75 (s, 3H), 1.95 (m, 2H), 0.93 (t, 3H). | 16.07 (1) | A |
| A-38 | (2*S*)-2-(4-chloro-2-fluorophenoxy)propanoi c acid | 1H NMR (400 MHz, DMSO-d6) δ 13.15 (s, 1H), 7.45 (dd, J = 11.2, 2.6 Hz, 1H), 7.19 (ddd, J = 8.8, 2.5, 1.5 Hz, 1H), 7.06 (t, J = 9.0 Hz, 1H), 4.95 (q, J = 6.8 Hz, 1H), 1.53 (d, J = 6.8 Hz, 3H). | 3.04 (2) | A |
| A-39 | (2*S*)-2-(3,4-dichlorophenoxy)propan oic acid | 1H NMR (400 MHz, DMSO-d6) δ 13.16 (s, 1H), 7.53 (d, J = 9.0 Hz, 1H), 7.18 (d, J = 2.9 Hz, 1H), 6.92 (dd, J = 9.0, 2.9 Hz, 1H), 4.96 (q, J = 6.8 Hz, 1H), 1.50 (d, J = 6.8 Hz, 3H). | 7.59 (2) | A |
| A-40 | (2*S*)-2-(2,4-dibromophenoxy)propan oic acid | 1H NMR (400 MHz, CDCl₃) δ 7.71 (1H, d, J=2.4 Hz), 7.36 (1H, dd, J=8.7, 2.4 Hz), 6.75 (1H, d, J=8.8 Hz), 4.78 (1H, q, J=6.9 Hz), 1.72 (3H, d, J=6.9 Hz) | 1.451 (3) | A |
| A-41 | (2*S*)-2-[4-(prop-1-yn-1-yl)phenoxy]propanoic acid | 1H NMR (300 MHz, CD₃OD) δ 7.25 (d, 2H), 4.77 (q, 1H), 1.98 (s, 3H), 1.57 (d, 3H). | 12.97 (1) | A |
| A-42 | (2*S*)-2-(4-ethynylphenoxy)propano ic acid | 1H-NMR (300 MHz, CD3OD): δ 7.31 (d, 2H), 4.49 (q, 1H), 1.52 (d, 3H). | 11.175 (1) | A |
| A-43 | sodium (2*S*)-2-(4-chlorophenoxy)butanoat e | 1H NMR (300 MHz, CD3OD) δ 6.87 (d, 2H), 4.28 (t, 1H), 1.07 (t, 3H); | 13.299 (1) | A |
| A-44 | sodium (2*S*)-2-(2,4-dichlorophenoxy)propan oate | 1H-NMR (300 MHz, CD3OD): δ 7.35 (s, 1H), 6.86 (d, 1H), 4.44 (q, 1H), 1.58 (d, 3H). | 13.564 (1) | A |
| A-45 | sodium (2*S*)-2-(4-chlorophenoxy)-3-methylbutanoate | 1H-NMR (300 MHz, CD3OD): δ 7.16 (d, 2H), 4.05 (q, 1H), 2.26-2.09 (m, 1H), 1.04 (dd, 6H). | 9.686 (1) | A |
| A-46 | sodium (2*S*)-2-(4-ethylphenoxy)propanoat e | 1H NMR (300 MHz, CD3OD) δ 6.81 (d, 2H), 4.47 (q, 1H), 2.54 (q, 2H), 1.51 (d, 3H), 1.17 (t, 3H); | 12.937 (1) | A |
| A-47 | sodium (2*S*)-2-(4-cyanophenoxy)propano ate | 1H NMR (300 MHz, CD3OD) δ 7.00 (d, 2H), 4.58 (q, 1H), 1.57 (d, 3H); | 10.35 (1) | A |
| A-48 | sodium (2*S*)-2-[4-(methylsulfanyl)phenoxy ]propanoate | 1H NMR (300 MHz, CD3OD) δ 6.85 (d, 2H), 4.47, (q, 1H), 2.39 (s, 3H), 1.52 (d, 3H); | 5.858 (1) | A |
| A-49 | methyl (2*S*)-2-(4-ethynylphenoxy)propano ate | 1H NMR (300 MHz, CDCl3) δ 7.42 (d, 2H), 4.78 (q, 1H), 3.76 (s, 3H), 3.01 (s, 1H), 1.67 (d, 3H). | 11.73 (1) | A |
| A-50 | methyl (2*S*)-2-(4-bromophenoxy)propano ate | 1H NMR (300 MHz, CDCl3) δ 7.38 (d, 2H), 4.73 (q, 1H), 3.76 (s, 3H), 1.63 (d, 3H). | 15.11 (1) | A |
| A-51 | methyl (2*S*)-2-(4-chlorophenoxy)butanoat e | 1H NMR (300 MHz, CDCl3) δ 7.24 (d, 2H), 4.54 (t, 1H), 3.76 (s, 3H), 1.99 (m, 2H), 1.07 (t, 3H). | 12.98 (1) | A |
| A-52 | 2,2,2-trifluoroethyl (2*S*)-2-(4-chlorophenoxy)propano ate | 1H NMR (300 MHz, CDCl3) δ 7.25 (m, 2H), 4.84 (q, 1H), 4.56 (q, 2H), 1.67 (d, 3H). | 13.279 (1) | A |
| A-53 | propan-2-yl (2*S*)-2-(4-chlorophenoxy)propano ate | 1H NMR (300 MHz, CDCl3) δ 7.23 (m, 2H), 5.07 (m, 1H), 4.67 (q, 1H), 1.61 (d, 3H), 1.27 (d, 3H). | 13.595 (1) | A |
| A-54 | methyl (2*S*)-2-(4-chlorophenoxy)propano ate | 1H NMR (300 MHz, CDCl3) δ 7.20 (d, 2H), 4.70 (q, 1H), 3.78 (s, 3H), 1.65 (d, 3H). | 14.969 (1) | A |
| A-55 | (2*S*)-2-(4-bromo-2,6-difluorophenoxy)-3-methylbutanoic acid | 1H NMR (400 MHz, DMSO-d6) δ 13.11 (s, 1H), 7.55 - 7.42 (m, 2H), 4.58 (dt, J = 4.5, 1.2 Hz, 1H), 2.21 (pd, J = 6.8, 4.5 Hz, 1H), 1.03 (d, J = 6.8 Hz, 3H), 1.01 (d, J = 6.9 Hz, 3H). | 4.444 (2) | A |
| A-56 | (2*S*)-2-(4-bromophenoxy)butanoic acid | ¹H NMR (300 MHz, CDCl₃) δ 10.21-9.28 (br, 1H); 7.39 (d, 2H); 6.79 (d, 2H); 4.58 (t, 1H); 2.03 (q, 2H); 1.11 (t, 3H). | 14.016 (1) | A |
| A-57 | (2*S*)-2-(4-cyclobutylphenoxy)prop anoic acid | 1H NMR (300 MHz, CDCl3) δ 11.19 (br s, 1H), 7.16 (d, 2H), 4.78 (q, 1H), 3.57 - 3.43 (m, 1H), 1.67 (d, 3H). | 14.623 (1) | A |
| A-58 | (2*S*)-2-(4-bromo-2-fluorophenoxy)butanoic acid | 1H NMR (300 MHz, CDCl3) δ 10.68 (br s, 1H), 6.85 (t, 1H), 4.61 (m, 1H), 2.06 (m, 2H), 1.13 (t, 3H). | 14.177 (1) | A |
| A-59 | (2*S*,3*E*)-2-(4-bromophenoxy)-4-fluorobut-3-enoic acid | 1H NMR (300 MHz, CDCl3) δ 7.40 (d, 2H), 6.14-5.97 (m, 0.5 H), 5.69-5.55 (m, 1H), 5.45 (d, 0.5H). | 9.836 (1) | A |
| A-60 | (2*S*)-2-(4-bromophenoxy)(2-²H)butanoic acid | 1H NMR (400 MHz, DMSO-d6) δ 7.35 - 7.28 (d, J = 8.5 Hz, 2H), 6.77 - 6.70 (d, JU = 8.5 Hz, 2H), 1.83 - 1.66 (m, 2H), 0.93 (t, J = 7.4 Hz, 3H). | 3.895 (2) | A |
| A-61 | (2S)-2-(4-bromophenoxy)pent-4-ynoic acid | 1H NMR (300 MHz, CDCl3) δ 7.99-7.48 (br, 1H), 7.41 (d, 2H), 4.78 (t, 1 H), 2.13 (s, 1H). | 9.962 (1) | A |
| A-62 | (2*S*)-2-(4-bromo-2-fluorophenoxy)pentanoic acid | 1H NMR (300 MHz, CD3OD) δ 7.25 (d, 1H), 7.16 (d, 1H), 4.35 (t, 1 H), 2.03- 1.69-1.44 (m, 2H), 0.98 (t, 3H). | 11.552 (1) | A |
| A-63 | (2*S*)-2-(2,4-dibromophenoxy)pentan oic acid | 1H NMR (300 MHz, CD3OD) δ 7.64 (s, 1H), 6.77 (d, 1H), 4.37-4.27 (m, 1H), 0.98 (t, 3H); | 12.621 (1) | A |
| A-64 | (2*S*)-2-(4-bromo-2-chlorophenoxy)pentanoi c acid | 1H-NMR (300 MHz, CD3OD): δ 7.44 (d, 1H), 7.26 (d, 1H), 4.28 (dd, 1H), 2.0-1.78 (m, 2H), 1.69-1.44 (m, 2H), 0.94 (t, 3H). | 12.324 (1) | A |
| A-65 | (2*S*)-2-(4-bromophenoxy)-3-cyclopropylpropanoic acid | 1H NMR (300 MHz, CDCl3) δ 10.75-10.32 (br, 1H), 7.40 (d, 2H), 4.71 (t, 1H), 2.07-1.93 (m, 1H), 1.89-1.74 (m, 1H), 0.28-0.09 (m, 2H). | 12.16 (1) | A |
| A-66 | (2*S*)-2-(2,4-dibromophenoxy)pent-4-ynoic acid | 1H NMR (300 MHz, CDCl3) δ 7.73 (s, 1H), 6.87 (d, 1H), 4.82 (t, 1H), 2.15 (s, 1H); | 11.909 (1) | A |
| A-67 | (2*S*)-2-(4-bromo-2-chlorophenoxy)pent-4-ynoic acid | 1H-NMR (300 MHz, CD3OD): δ 9.73-8.96 (br, 1H), 7.55 (s, 1H), 7.33 (d, 1H), 4.81 (t, 1H), 2.98 (d, 2H), 2.15 (s, 1H). | 10.83 (1) | A |
| A-68 | (2*S*)-2-(4-bromophenoxy)-2-cyclopropylacetic acid | 1H NMR (300 MHz, CD3OD) δ 7.31 (d, 2H), 6.81 (d, 2H), 3.86 (d, 1 H), 1.45-1.21 (m, 1H), 0.72-0.37 (m, 4H). | 10.358 (1) | A |
| A-69 | (2*S*)-2-(4-bromo-2-fluorophenoxy)pent-4-ynoic acid | 1H NMR (300 MHz, CDCl3) δ 9.39-8.42 (br, 1H), 7.29 (d, 1H), 7.21 (d, 1H), 4.81 (t, 1H), 2.94 (d, 2H), 2.14 (s, 1H). | 10.91 (1) | A |
| A-70 | (2*S*)-2-(4-bromophenoxy)pentanoi c acid | 1H-NMR (300 MHz, CD3OD): δ 7.18 (d, 2H), 4.20 (t, 1H), 1.81-1.65 (m, 2H), 1.53-1.27 (m, 2H), 0.82 (t, 3H). | 14.589 (1) | A |
| A-71 | (2*S*)-2-(4-bromo-2-chlorophenoxy)-2-cyclobutylacetic acid | 1H NMR (300 MHz, CDCl3) δ 10.54-9.99 (br, 1H), 7.54 (d, 1H), 7.29 (dd,1 H), 4.55 (d, 1H), 3.07-2.87 (m, 1H), 2.34-1.76 (m, 6H). | 12.735 (1) | A |
| A-72 | (2*S*)-2-(4-bromo-2-chlorophenoxy)-3-cyclopropylpropanoic acid | 1H NMR (300 MHz, CD3OD) δ 7.55 (s, 1H), 6.86 (d, 1H), 4.80 (t, 1H), 1.11-0.93 (m, 1H), 0.61-0.41 (m, 2H); | 13.104 (1) | A |
| A-73 | (2*S*)-2-(4-bromo-2-chlorophenoxy)-3-methylbutanoic acid | 1H NMR (500 MHz, DMSO-d6) δ 7.92 (d, J = 2.4 Hz, 1H), 7.34 (dd, J = 8.9, 2.5 Hz, 1H), 6.80 (d, J = 8.9 Hz, 1H), 3.87 (d, J = 4.8 Hz, 1H), 2.27-2.08 (m, 1H), 0.98 (d, J = 6.8 Hz, 3H), 0.97 (d, J = 6.8 Hz, 3H) | 2.378 (2) | A |
| A-74 | (2*S*)-2-(2,4-dibromophenoxy)-3-methoxypropanoic acid | 1H NMR (300 MHz, CDCl3) δ 7.71 (d, 1H), 7.37 (dd, 1 H), 6.24-5.16 (br, 1H), 4.83 (t, 1H), 3.51 (s, 3H). | 10.51 (1) | A |
| A-75 | (2*S*)-2-(4-bromophenoxy)but-3-enoic acid | 1H NMR (300 MHz, CDCl3) δ 8.16-7.54 (br, 1H), 7.40 (d, 2H), 6.14-5.98 (m, 1H), 5.47 (d, 1H), 5.14 (d, 1H). | 10.051 (1) | A |
| A-76 | (2*S*)-2-(4-bromophenoxy)(3,4-²H₂)butanoic acid | 1H NMR (300 MHz, CD3OD) δ 7.34 (d, 2H), 4.41 (d, 1H), 2.02-1.83 (m, 1H), 1.04 (d, 2H). | 10.499 (1) | A |
| A-77 | (2*R*)-2-(4-bromo-2-chlorophenoxy)-3-fluoropropanoic acid | 1H NMR (500 MHz, DMSO-d6) δ 7.58 (d, J = 2.5 Hz, 1H), 7.37 (dd, J = 8.9, 2.5 Hz, 1H), 6.83 (d, J = 8.9 Hz, 1H), 4.78 (ddd, J = 47.7, 10.2, 2.2 Hz, 1H), 4.69 (ddd, J = 49.3, 10.2, 7.6 Hz, 1H), 4.54 (ddd, J = 23.3, 7.6, 2.2 Hz, 1H). | 3.657 (2) | A |
| A-78 | (2*S*)-2-(4-bromo-2-chlorophenoxy)butanoic acid | 1H NMR (500 MHz, DMSO-d6) δ 7.55 (d, J = 2.5 Hz, 1H); 7.35 (dd, J = 8.8, 2.5 Hz, 1H); 6.80 (d, J = 8.9 Hz, 1H); 4.07 (dd, J = 7.7, 4.5 Hz, 1H); 1.95-1.60 (m, 2H); 0.97 (t, J = 7.4 Hz, 3H). | 1.874 (2) | A |
| A-79 | (2*S*)-2-(4-bromo-3-fluorophenoxy)-3-methylbutanoic acid | 1H NMR (300 MHz, CDCl3) δ 9.94-9.50 (br, 1H), 7.42 (t, 1H), 6.61 (dd, 1 H), 4.41 (d, 1H), 2.44-2.25 (m, 1H), 1.11 (d, 6H). | 11.551 (1) | A |
| A-80 | (2*S*)-2-(4-bromo-2-chlorophenoxy)-4-fluorobutanoic acid | 1H-NMR (300 MHz, CD3OD): δ 7.45 (d, 1H), 7.28 (dd, 1H), 4.81-4.63 (m, 1H), 4.64-4.48 (m, 1H), 2.45-2.05 (m, 2H). | 11.811 (1) | A |
| A-81 | (2*S*)-2-(4-bromo-2,3-difluorophenoxy)-3-methylbutanoic acid | 1H NMR (300 MHz, CDCl3) δ 9.88 (s, 1H), 6.59 (t, 1H), 4.43 (d, 1H), 1.09 (t, 6H); | 12.541 (1) | A |
| A-82 | (2*R*)-2-(4-bromophenoxy)-3-fluoro(2-²H)propanoic acid | 1H NMR (500 MHz, DMSO-d6) δ 13.53 (s, 1H), 7.54 - 7.34 (m, 2H), 6.99 - 6.85 (m, 2H), 4.87 (dd, J = 46.9, 10.5 Hz, 1H), 4.83 (dd, J = 47.8, 10.4 Hz, 1H). | 2.955 (2) | A |
| A-83 | (2*S*)-2-(4-bromo-2-iodophenoxy)-4-fluorobutanoic acid | 1H-NMR (300 MHz, CD3OD): δ 9.67-8.38 (br, 1H), 7.92 (d, 1H), 7.41 (dd, 1H), 4.94-4.80 (m, 2H), 2.62-2.29 (m, 2H). | 15.038 (1) | A |
| A-84 | (2*S*)-2-(4-bromophenoxy)-2-cyclobutylacetic acid | 1H NMR (300 MHz, CDCl3) δ 10.0-8.66 (br, 1H), 7.39 (d, 2H), 4.51 (d, 1H), 2.24-1.80 (m, 6H). | 12.88 (1) | A |
| A-85 | (2*S*)-2-(4-bromo-2-fluorophenoxy)-4-fluorobutanoic acid | 1H NMR (300 MHz, CDCl3) δ 11.18-10.40 (br, 1H), 7.28 (dd, 1H), 7.19 (dd, 1 H), 4.92- 4.64 (t, 1H), 2.56-2.20 (m, 2H). | 11.56 (1) | A |
| A-86 | (2*S*)-2-(4-bromo-2-fluorophenoxy)-2-cyclobutylacetic acid | 1H NMR (300 MHz, CD3OD) δ 7.26 (dd, 1H), 7.16 (d, 1 H), 4.26 (d, 1H), 2.99-2.83 (m, 1H), 2.31-1.74 (m, 6H). | 15.117 (1) | A |
| A-87 | (2*S*)-2-(4-bromophenoxy)-4-fluorobutanoic acid | 1H-NMR (300 MHz, CD3OD): δ 7.33 (d, 2H), 4.70 (t, 1H), 4.59-4.44 (m, 2H), 2.44-2.06 (m, 2H). | 10.955 (1) | A |
| A-88 | (2*S*)-2-(4-bromo-2-iodophenoxy)-3-methylbutanoic acid | 1H NMR (500 MHz, Chloroform-d) δ 7.91 (d, J = 2.4 Hz, 1H), 7.36 (dd, J = 8.7, 2.4 Hz, 1H), 6.51 (d, J = 8.7 Hz, 1H), 4.48 (d, J = 4.2 Hz, 1H), 2.40 (heptd, J = 6.9, 4.3 Hz, 1H), 1.20 (d, J = 6.9 Hz, 3H), 1.18 (d, J = 6.9 Hz, 3H). | 5.194 (2) | A |
| A-89 | (2*S*)-2-(4-bromo-2-fluorophenoxy)-2-cyclopropylacetic acid | 1H NMR (500 MHz, DMSO-d6) δ 13.13 (s, 1H), 7.55 (dd, J = 10.9, 2.4 Hz, 1H), 7.31 (ddd, J = 8.8, 2.5, 1.5 Hz, 1H), 6.92 (t, J = 9.0 Hz, 1H), 4.26 (d, J = 8.4 Hz, 1H), 1.36 - 1.27 (m, 1H), 0.69 - 0.46 (m, 4H). (N.B. free acid) | 3.826 (1) | A |
| A-90 | (2*S*)-2-(4-bromo-2-iodophenoxy)butanoic acid | 1H-NMR (300 MHz, CDCl3): δ 7.93 (d, 1H), 7.39 (dd, 1H), 4.67 (t, 1H), 3.35 (s, 1H), 2.11 (q, 2H), 1.16 (t, 3H). | 15.25 (1) | A |
| A-91 | (2*S*)-2-(4-chloro-2-fluorophenoxy)butanoic acid | 1H NMR (300 MHz, CDCl3) δ 9.14-7.91 (br, 1H), 7.14 (dd, 1H), 7.04 (dq, 1 H), 4.62 (t, 1H), 2.15-1.99 (m, 2H), 1.14 (t, 3H). | 13.821 (1) | A |
| A-92 | (2*S*)-2-cyclopropyl-2-(2,4-dibromophenoxy)acetic acid | 1H NMR (400 MHz, DMSO-d6) δ 7.68 (s, 1H), 7.37 (s, 1H), 6.74 (d, J = 9.0 Hz, 1H), 3.81 (s, 1H), 1.26 (s, 1H), 0.48 (d, J = 28.9 Hz, 4H) | 4.863 (2) | A |
| A-93 | (2*S*)-2-(4-bromo-2-chlorophenoxy)-2-cyclopropylacetic acid | 1H NMR (400 MHz, DMSO-d6) δ 7.55 (d, J = 2.5 Hz, 1H), 7.33 (dd, J = 8.8, 2.5 Hz, 1H), 6.78 (d, J = 8.9 Hz, 1H), 3.76 (d, J = 6.9 Hz, 1H), 1.33 - 1.13 (m, 1H), 0.56 - 0.33 (m, 4H). | 4.708 (2) | A |
| A-94 | (2*R*,3*R*)-2-(4-bromophenoxy)-3-fluorobutanoic acid | 1H NMR (400 MHz, DMSO-d6) δ 7.40 - 7.30 (m, 2H), 6.85 - 6.75 (m, 2H), 4.92 (dp, J = 49.1, 6.3 Hz, 1H), 4.05 (dd, J = 22.4, 5.7 Hz, 1H), 1.33 (dd, J = 23.9, 6.4 Hz, 3H). | 3.675 (2) | A |
| A-95 | (2*R*,3*R*)-2-(4-bromo-2-fluorophenoxy)-3-fluorobutanoic acid | 1H NMR (400 MHz, DMSO-d6) δ 7.44 (dd, J = 11.0, 2.4 Hz, 1H), 7.22 (ddd, J = 8.8, 2.5, 1.5 Hz, 1H), 6.91 (t, J = 9.0 Hz, 1H), 4.99 (dqd, J = 48.6, 6.4, 5.3 Hz, 1H), 4.14 (dd, J = 23.5, 5.3Hz, 1H), 1.35 (dd, J = 23.9, 6.4 Hz, 3H). | 3.886 (2) | A |

### Specific examples of Syntheses

### Example 1: Synthesis of (2R)-2-(4-bromophenoxy)-3-fluoropropanoic acid; following the synthetic strategy of General Method C

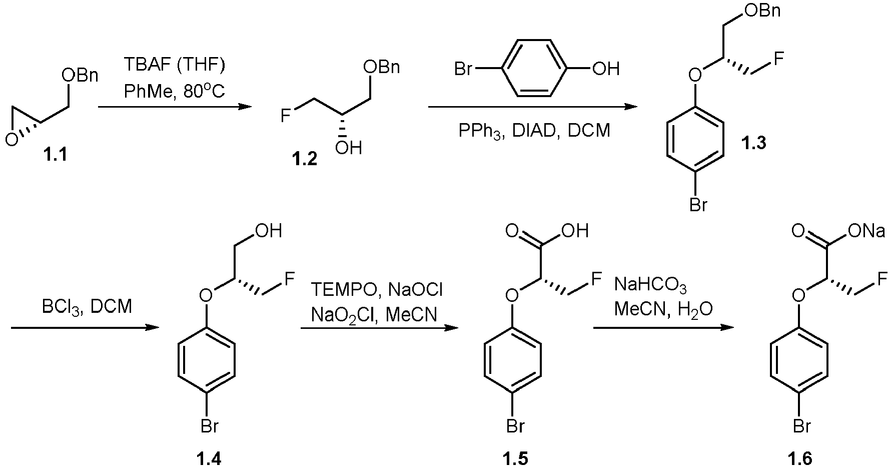

### (S)-1-(Benzyloxy)-3-fluoropropan-2-ol (1.2)

Tetrabutylammonium fluoride, 1M solution in THF (114 mL, 114 mmol) was added to a solution of (R)-2-((benzyloxy)methyl)oxirane (**1.1**) (15.56 g, 95 mmol) in anhydrous toluene (300 mL, 95 mmol) and the mixture stirred at 80 °C under nitrogen for 24 h. The mixture was cooled to room temp., diluted with water and extracted with ethyl acetate. The organic phase was washed with brine, dried over MgSO₄ and evaporated *in vacuo* to afford a crude oil. The material was adsorbed onto silica then purified by chromatography on silica gel (220 g column, 0-100% EtOAc/ isohexane) to afford (*S*)-1-(benzyloxy)-3-fluoropropan-2-ol (**1.2**) (6.419 g, 31.4 mmol, 33% yield) as a pale yellow oil. The product was analysed by LCMS (Waters Acquity UPLC, C18, Waters X-Bridge UPLC C18, 1.7 µm, 2.1 × 30mm, Acidic (0.1% Formic acid) 3 min method, 5-95% MeCN/water) 0.978 min, 91% purity @ 254 nm; ¹H NMR (400 MHz, CDCl3) δ 7.43 - 7.30 (m, 5H); 4.59 (s, 2H); 4.58 - 4.49 (m, 1H); 4.43 (qd, J = 9.6, 4.9 Hz, 1H); 4.12-3.96 (m, 1H); 3.67 - 3.54 (m, 2H); 2.52 (d, J = 4.7 Hz, 1H).

### (R)-1-((1-(Benzyloxy)-3-fluoropropan-2-yl)oxy)-4-bromobenzene (1.3)

To a solution of (*S*)-1-(benzyloxy)-3-fluoropropan-2-ol (**1.2**) (1.725 g, 9.36 mmol), 4-bromophenol (1.62 g, 9.36 mmol) and triphenylphosphine (3.44 g, 13.11 mmol) in anhydrous THF (90 mL, 1098 mmol) at 0°C was added DIAD (2.55 mL, 13.11 mmol). The solution was allowed to warm to room temperature and stirred for 4 hours. After the allotted time, MeOH was added and volatiles removed *in vacuo.* The crude product was purified by chromatography on silica gel (40 g column, 0-30% EtOAc/ isohexane) to afford (*R*)-1-((1-(benzyloxy)-3-fluoropropan-2-yl)oxy)-4-bromobenzene (**1.3**) (998 mg, 2.80 mmol, 30% yield) as a clear colourless oil. The product was analysed by LCMS (Waters Acquity UPLC, C18, Waters X-Bridge UPLC C18, 1.7 µm, 2.1 × 30mm, Acidic (0.1% Formic acid) 3 min method, 5-95% MeCN/ water): m/z 339.822 (M+H)+ (ES+) at 1.858 min, 99% purity @ 254 nm. ¹H NMR (400 MHz, DMSO-d⁶) δ 7.45 (d, J = 9.0 Hz, 2H); 7.37-7.25 (m, 5H); 7.00 (d, J = 9.0 Hz, 2H); 4.89-4.67 (m, 2H); 4.61 (qd, J = 10.4, 4.1 Hz, 1H), 4.53 (s, 2H); 3.73-3.62 (m, 2H).

### (R)-2-(4-Bromophenoxy)-3-fluoropropan-1-ol (1.4)

Trichloroborane, 1M solution in dichloromethane (3.24 mL, 3.24 mmol) was added to a stirred solution of (*R*)-1-((1-(benzyloxy)-3-fluoropropan-2-yl)oxy)-4-bromobenzene (**1.3**) (998 mg, 2.94 mmol) in anhydrous dichloromethane (45 mL, 699 mmol) under nitrogen at 0°C. After 2 hours the mixture was allowed to warm to room temperature, aqueous NaHCOs was added and the mixture stirred for 1 h at room temperature. The phases were then separated and the aqueous further extracted with EtOAc. The combined organics were dried over MgSO₄ and volatiles removed *in vacuo* to afford a pale yellow oil. The material was carried forward without further purification.

### (2R)-2-(4-Bromophenoxy)-3-fluoropropanoic acid (1.5)

Sodium chlorite (1.342 g, 14.84 mmol) was dissolved in 1M sodium phosphate buffer pH6 (60 mL, 60.0 mmol) and added to a solution of (*R*)-2-(4-bromophenoxy)-3-fluoropropan-1-ol (2.843 g, 7.42 mmol) (**1.4**) in acetonitrile (60 mL), followed by aqueous sodium hypochlorite solution (0.087 mL, 0.074 mmol) and TEMPO (0.041 g, 0.260 mmol). The resulting 2-phase mixture was stirred at room temp for 1 hour. Further aqueous sodium hypochlorite solution (0.087 mL, 0.074 mmol) and TEMPO (0.041 g, 0.260 mmol) were added and the mixture was stirred overnight at room temperature. Further aqueous sodium hypochlorite solution (0.27 mL, 0.222 mmol) and TEMPO (0.123 g, 0.780 mmol) were added and the reaction stirred for a further 3 h. To the mixture was added saturated sodium metabisulfite solution (40 mL) and the mixture was diluted with water, acidified by addition of conc. hydrochloric acid and extracted with ethyl acetate. The organic phase was extracted with aq. sodium hydroxide solution (1 M). The aqueous phase was acidified to pH 2, by addition of conc. hydrochloric acid, then extracted with ethyl acetate. The combined organic extracts were dried over MgSO₄ and volatiles removed in vacuo. The material was adsorbed onto silica then purified by chromatography on silica gel (80 g column, 0-50% EtOAc/ isohexane) to afford (*R*)-2-(4-bromophenoxy)-3-fluoropropanoic acid (**1.5**) (1.307 g, 4.72 mmol, 63% yield) as a colourless solid. The product was analysed by LCMS (Agilent Infinity, X-Select, Waters X-Select C18, 2.5 µm, 4.6x30 mm, Acidic (0.1% Formic acid) 15 min method, 5-95% MeCN/water): (M+H)+ (ES+); 260.900 (M-H)- (ES-), at 4.166 min, 95% purity @ 254 nm. (Note: product has poor absorption at 254 nm). ¹H NMR (400 MHz, DMSO-d⁶) δ 13.53 (s, 1H); 7.46 (d, J = 9.0 Hz, 2H); 6.93 (d, J = 9.0 Hz, 2H); 5.20 (ddd, J = 29.1, 3.9, 2.5 Hz, 1H); 4.97-4.71 (m, 2H).

To a solution of (*R*)-2-(4-bromophenoxy)-3-fluoropropanoic acid (**1.5**) (867 mg, 3.30 mmol) in acetonitrile (23 mL) was added sodium bicarbonate (277 mg, 3.30 mmol) in H₂O (8 mL) and the reaction stirred at ambient temperature for 30 min. After the allotted time, volatiles were removed *in vacuo* and excess water removed by coevaporation with toluene, resulting in a clear oil. Dichloromethane was added and removed resulting in a colourless solid. The solid was triturated with further dichloromethane and dried in a dessicator overnight to afford sodium (*R*)-2-(4-bromophenoxy)-3-fluoro-propanoate (**1.6**) (0.496 g, 1.65 mmol, 50%). The colourless solid product was analysed by LCMS (Agilent Infinity, X-Select, Waters X-Select C18, 2.5 µm, 4.6x30 mm, Acidic (0.1% Formic acid) 15 min method, 5-95% MeCN/water): (ES+); 260.976 (M-Na)- (ES-), at 1.476 min, 95% purity @ 254 nm. ¹H NMR (400 MHz, DMSO-d⁶) δ 7.36 (d, J = 9.0 Hz, 2H); 6.78 (d, J = 9.0 Hz, 2H); 4.86-4.53 (m, 2H); 4.47 (ddd, J = 23.5, 7.5, 2.2 Hz, 1H).

### Example 2: (2S)-2-(4-bromo-2-fluorophenoxy)-3-methylbutanoic acid, adapted from General Method A

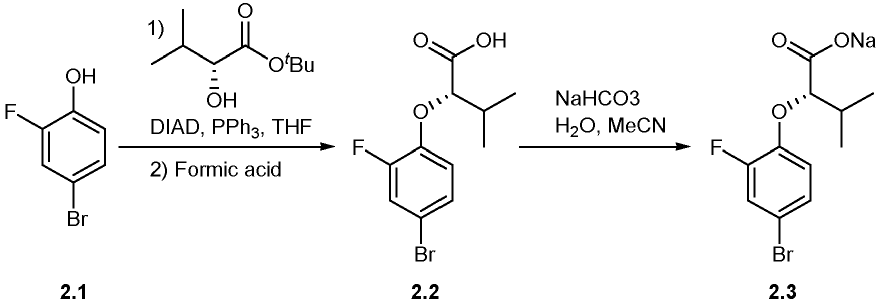

### (S)-2-(4-bromo-2-fluorophenoxy)-3-methyl)butanoic acid (2.2)

DIAD (2.343 mL, 12.05 mmol) was added to a stirred solution of (*R*)-tert-butyl 2-hydroxy-3-methylbutanoate (**2.1**) (1.5 g, 8.61 mmol), 4-bromo-2-fluorophenol (1.037 mL, 9.47 mmol) and triphenylphosphine (3.16 g, 12.05 mmol) in anhydrous tetrahydrofuran (50.6 mL, 8.61 mmol) at 0°C and stirred for 30 min before being allowed to warm to room temperature. After 16 hours, the mixture was evaporated in vacuo to a syrup which was re-dissolved in formic acid (33.0 mL, 861 mmol) and heated to 70°C for 1 hour. MeOH (20 mL) was added and the resulting solution was evaporated *in vacuo* and the residue co-evaporated with toluene (2 x 30mL). The residue was dissolved in ethyl acetate (100mL) and extracted with 0.5M sodium hydroxide solution (100mL). The aqueous phase was washed with ethyl acetate (2 x 100mL) then acidified to pH 2-3 by dropwise addition of c. hydrochloric acid. The resulting cloudy solution was extracted with ethyl acetate (3x 100mL). Organic extracts were filtered through a phase separating funnel. The residue was purified by column chromatography (40g Grace silica cartridge) with 0-40% ethyl acetate in isohexane gradient elution to give an oil which was dried in vacuo at 40°C overnight to give (*S*)-2-(4-bromo-2-fluorophenoxy)-3-methylbutanoic acid (**2.2**) (1.6436 g, 5.53 mmol, 64.3 % yield) as a white waxy solid.

The product was analysed by LCMS (Waters Acquity UPLC, X-Select, Waters X-Select UPLC C18, 1.7 µm, 2.1 × 30mm, Acidic (0.1% Formic acid) 10 min method, 5-95% MeCN/ water): m/z 289-291 (M-H)- (ES-), at 4.352 min, 97.6% purity @ 254 nm; ¹H NMR (400 MHz, DMSO-d⁶) δ 13.16 (s, 1H), 7.53 (dd, J = 10.9, 2.4 Hz, 1H), 7.34 - 7.27 (m, 1H), 6.97 (t, J = 9.0 Hz, 1H), 4.61 (d, J = 4.6 Hz, 1H), 2.31 - 2.18 (m, 1H), 1.01 (dd, J = 6.5 Hz, 6H).

To (*S*)-2-(4-bromo-2-fluorophenoxy)-3-methylbutanoic acid (**2.2**) (0.8254 g, 2.77 mmol) in MeCN (27 mL) was added NaHCOs (0.232 g, 2.77 mmol) in H₂O (9 mL) and the mixture was stirred at room temperature for 30 min. The aqueous solvent was removed under reduced pressure to give a white solid which was dissolved in H₂O (30 mL) and washed with DCM (3 x 30 mL). The water was removed under reduced pressure and the residue dried at 45°C in vacuo for 72 hours to give sodium (*S*)-2-(4-bromo-2-fluorophenoxy)-3-methylbutanoate (**2.3**) (0.866 g, 2.63 mmol, 95 % yield) as a white solid. The product was analysed by LCMS (Waters Acquity UPLC, X-Select, Waters X-Select UPLC C18, 1.7 µm, 2.1x30mm, Acidic (0.1% Formic acid) 10 min method, 5-95% MeCN/ water): m/z 288.915, 290.966 (M-H)- (ES-), 89% purity @ 254 nm. 100% purity @ 210-400 nm. (Poor absorbance @254). 1H NMR (400 MHz, DMSO-d6) δ 7.39 (dd, J = 11.0, 2.4 Hz, 1H), 7.18 (ddd, J = 8.8, 2.5, 1.5 Hz, 1H), 6.86 (t, J = 9.0 Hz, 1H), 3.92 (d, J = 5.0 Hz, 1H), 2.21 - 2.08 (m, 1H), 0.95 (d, J = 6.7 Hz, 6H).

### Description of Pharmacological Methods and Drawings

### Example 3: Electrophysiological measurement of compound inhibition of CIC-1 in rat muscle

The investigatory goal of these experiments was to evaluate whether compounds inhibit CIC-1 channels in native tissue of rat skeletal muscle fibres. Apparent CIC-1 affinity was reported by the concentration of compound at which 50% of the compound's full inhibition of CIC-1 was observed (EC₅₀).

CIC-1 Cl⁻ ion channels generate around 80% of the total membrane conductance (Gₘ) in resting skeletal muscle fibres of most animals including rat and human (Bretag, A H. Muscle chloride channels. Physiological Reviews, **1987**, *67*, 618-724). Other ion channels that contribute to Gₘ can therefore be considered negligible, and it is possible to evaluate whether a compound inhibits CIC-1 in rat muscle by comparing Gₘ measurements before and after exposure to a compound. CIC-1 inhibition would in such recordings be reflected by a reduction of Gₘ.

Experimentally, Gₘ was measured in individual fibres of whole rat soleus muscles using a three micro-electrodes technique described in this example and in full detail elsewhere (Riisager A. *et al.,* Determination of cable parameters in skeletal muscle fibres during repetitive firing of action potentials. *Journal of Physiology,* **2014,** *592*, 4417-4429). Briefly, intact rat soleus muscles were dissected out from 12 - 14-week old Wistar rats and placed in an experimental chamber that was perfused with a standard Krebs Ringer solution containing 122 mM NaCl, 25 mM NaHCOs, 2.8 mM KCI, 1.2 mM KH₂PO₄, 1.2 mM MgSO₄, 1.3 mM CaCl₂, 5.0 mM D-glucose. During experiments, the solution was kept at approx. 30°C and continuously equilibrated with a mixture of 95% O₂ and 5% CO₂, pH -7.4. The experimental chamber was placed in Nikon upright microscope that was used to visualize individual muscle fibres and the three electrodes (glass pipettes filled with 2 M potassium citrate). For Gₘ measurements, the electrodes were inserted into the same fibre with known inter-electrode distances of 0.35 - 0.5 mm (V1-V2, X1) and 1.1-1.5 mm (V1-V3, X3) (Figure 1A). The membrane potential of the impaled muscle fibre was recorded by all electrodes. Two of the electrodes were furthermore used to inject 50 ms current pulses of -30 nA. Given the positions of the electrodes, three different inter-electrode distances could be identified (X1-X2, X1-X3, X2-X3) and hence the membrane potential responses to the current injections could be obtained at three distances from the point of current injection. The steady state voltage deflection at each distance was divided by the magnitude of current injected (-30 nA) and the resulting transfer resistances were plotted against inter-electrode distance and the data was fitted to a mono-exponential function from which Gₘ could be calculated using linear cable theory (Figure 1B).

To establish a dose response relationship, Gₘ was first determined in 10 muscle fibres in the absence of compound and then at four increasing compound concentrations with Gₘ determinations in 5-10 fibres at each concentration. The average Gₘ values at each concentration were plotted against compound concentration and the data was fitted to sigmoidal function to obtain an EC₅₀ value (Figure 1C). Table 2 shows the EC₅₀ values for a range of compounds with n values referring to number of experiments that each reflect recordings from around 50 fibres.

**Table 2: Inhibition of CIC-1 ion channel using compounds of the invention**

| Compound investigated | EC₅₀ (µM) |
|---|---|
| Compound A-6 | 7.4 ± 1.4 (n=4) |
| Compound A-26 | 3.8 ± 0.8 (n=7) |
| Compound A-27 | 4.5 ± 1.7 (n=7) |
| Compound (2*R*)-A-27 | >80 (n=1) |
| Compound A-31 | 7.2 ± 2.8 (n=2) |
| Compound A-40 | 4.2 ± 0.6 (n=3) |
| Compound A-54 | 6.8 ± 1.3 (n=2) |
| Compound A-58 | 6.3 ± 2.4 (n=2) |
| Compound A-60 | 7.5 ± 1.0 (n=3) |
| Compound A-66 | 7.2 (n=1) |
| Compound A-67 | 9.4 ± 0.6 (n=2) |
| Compound A-71 | 10.1 ± 0.2 (n=2) |
| Compound A-73 | 7.2 (n=1) |
| Compound A-74 | 4.0 ± 0.1 (n=2) |
| Compound A-76 | 3.8 ± 2.1 (n=3) |
| Compound A-78 | 7.6 ± 1.6 (n=3) |
| Compound A-82 | 5.2 ± 1.3 (n=4) |
| Compound A-83 | 8.0 (n=1) |
| Compound A-85 | 10.0 ± 9.5 (n=4) |
| Compound A-86 | 8.9 ± 3.2 (n=3) |
| Compound A-87 | 4.5 ± 0.8 (n=3) |
| Compound A-88 | 5.9 ± 1.6 (n=3) |
| Compound A-89 | 8.7 ± 1.4 (n=2) |
| Compound A-90 | 2.3 (n=1) |
| Compound A-94 | 12.0 ± 3.2 (n=3) |
| Compound A-95 | 12.5 ± 1.4 (n=4) |

In conclusion, this example demonstrates that the compounds of the present invention have an EC₅₀ value in the range of 3-10 µM. For example, compound A-27 has an EC₅₀ value of 4.5 µM. In comparison, the (2*R*)-enantiomer of compound A-27 has an EC₅₀ value higher than 80 µM, which demonstrates that the chiral centre significantly influences the activity on the CIC-1 channel.

### Example 4: Measurement of force in an in vitro model

The current invention relates to compounds that inhibit CIC-1 ion channels and increase muscle excitability and thereby improve muscle function in clinical conditions where muscle activation is failing. Such conditions result in loss of contractile function of skeletal muscle, weakness and excessive fatigue. In this series of experiments the compounds were tested for their ability to restore contractile function of isolated rat muscle when the neuromuscular transmission had been compromised akin to neuromuscular disorders.

Experimentally, soleus muscles from 4-5 wk old rats were isolated with the motor nerve remaining attached. The nerve-muscle preparations were mounted in experimental setups that enabled electrical stimulation of the motor nerve. Stimulation of the motor nerve led to activation of the muscle fibres and ensuing force production that was recorded. The nerve-muscle preparations were also in these experiments incubated in the standard Krebs Ringer (see example 5) and the solution was heated to 30°C and continuously equilibrated with a mixture of 95% O₂ and 5% CO₂, pH ~7.4.

After mounting the nerve-muscle preparation in the experimental setup, the contractile function of the muscle was initially assessed under the control conditions (Figure 2A). Sub-maximal concentration of tubocurarine (115 nM), an acetylcholine receptors antagonist, was then added to the experimental bath to impose partial inhibition of the ability of the motor nerve to activate the muscle fibres. The experimental condition mimics the failing neuromuscular transmission in a range of neuromuscular disorders. After addition of tubocurarine the contractile force declined over the next 90 mins to 10-50 % of the control force. 50 µM of the test compound was then added and the contractile force recovered despite the continued presence of tubocurarine. To quantify the ability of the compound to restore force the percentage of the initial force that was restored was determined after 40 mins of compound exposure (Figure 2B) and the point increase is reported in Table 3.

**Table 3: Percentage increase of initial force that was restored using compounds of the invention**

| Compound investigated | Point increase (%) |
|---|---|
| Compound A-6 | 44 |
| Compound A-26 | 39 |
| Compound (2*R*)-A-26 | -6 |
| Compound A-27 | 46 |
| Compound A-31 | 42 |
| Compound (2*R*)-A-31 | -8 |
| Compound A-40 | 20 |
| Compound A-58 | 44 |
| Compound A-60 | 62 |
| Compound (2*R*)-A-60 | -2 |
| Compound A-66 | 51 |
| Compound A-67 | 40 |
| Compound A-71 | 49 |
| Compound A-73 | 51 |
| Compound A-74 | 42 |
| Compound A-76 | 62 |
| Compound A-78 | 38 |
| Compound A-82 | 48 |
| Compound (2*R*)-A-82 | -4 |
| Compound A-83 | 35 |
| Compound A-85 | 37 |
| Compound A-86 | 45 |
| Compound A-87 | 37 |
| Compound A-88 | 55 |
| Compound A-89 | 33 |
| Compound A-90 | 57 |
| Compound A-94 | 31 |
| Compound (2*R*,3*R*)-A-94 | -4 |
| Compound A-95 | 39 |
| Compound (2*R*,3*R*)-A-95 | -7 |

In conclusion, this example demonstrates that the compounds of the present invention are able to increase muscle excitability and thereby improve muscle function in clinical conditions. The muscle contractility was recovered by 20 - 62 % points, which meant almost complete restoration of the force.

The data further demonstrates that neither the (2*R*)-enantiomers are unable to recover force compared to the enantiomerically pure (2*S*)-enantiomers.

### Example 5: Screening of compounds on the human isoform of CIC-1 expressed in CHO cells using automated patch-clamp

The investigatory goal of these experiments was to evaluate how compounds affect the open probability and current amplitude of human CIC-1 channels expressed in CHO cells. Experiments were performed using an automated patch clamp system that allowed high throughput testing of whole cell patches together with both intracellular and extracellular addition of compound.

### Automated voltage clamp measurements

Automated whole cell patch clamp experiments were performed with the Qpatch 16 system (Sophion Bioscience, Ballerup, Denmark) at room temperature. Data acquisition and analysis were performed in the Qassay software (ver. 5.6, Odense).

### Voltage protocol and analysis of whole cell CIC-1 currents

To evoke CIC-1 currents in whole cell patches, the membrane potential was initially stepped from a holding potential of -30 mV to +60 mV for 100 ms and then to various test voltages (sweeps) ranging from +120 mV to -140 mV in steps of 20 mV for 300 ms. To obtain tail currents, the membrane potential was stepped to -100 mV after each test voltage for 300 ms and then relaxed to -30 mV for 2 sec between sweeps (Figure 3). I/V relationships for whole cell instant and steady state current amplitudes were obtained by plotting average current densities at the beginning and at the end of the 300 ms step against the membrane potential (Figure 4).

In order to determine the relative overall open probability (P₀), the instantaneous tail currents were normalized to the maximal tail current obtained following the most positive voltage step and plotted against the test voltage. Plots of normalized tail currents from each whole cell patch were then fitted to a Boltzmann function allowing determination of half activation voltages (V_{1/2}, Figure 5).

### Solutions

For automated patch clamp experiments extracellular solutions contained: 2 mM CaCl₂, 1 mM MgCl₂, 10 mM HEPES, 4 mM KCI, 145 mM NaCl, 10 mM Glucose, pH adjusted to 7.4 with NaOH (2 M). Osmolality adjusted to -320 using sucrose.

Intracellular solutions contained: 80 mM CsF, 60 mM CsCI, 5/1 mM KOH/EGTA, 10 mM HEPES, 10 mM NaCl, pH adjusted to 7.2 with NaOH (2 M). Osmolality adjusted to -320 mOsm using sucrose.

### Cell line information:

Cells used in patch clamp experiments were Chinese hamster ovary cells (CHO) constitutively expressing human CIC-1 channels. The amino acid sequence encoded by the cDNA used to create this cell line was identical to the translated sequence for GenBank accession number NM_000083.2. Cells were produced by Charles River (Catalogue CT6175, Cleveland OH, USA) in a cryopreserved format. Experiments were performed on the cells directly after thawing (3 × 10⁶ cells used in each experiment).

### Test protocol

To evaluate the compound effect on CIC-1, when applied directly to the intracellular side of the cell membrane, the half activation voltage, V_{1/2}, was determined from whole cell patches with compound added to the intracellular solution and then compared to V_{1/2} determined from control cell patches with only vehicle added to the intracellular solution. Additionally, the effect of extracellular added compound was evaluated by determine V_{1/2} and steady state current amplitudes before and after exchanging the extracellular solution to contain compound.

The difference in half activation voltage of CIC-1 channels, ΔV_{1/2}, was determined as the difference between the cell patches treated intracellularly with compound and control cells patches and is reported in Table 4 below. A positive shift in ΔV_{1/2} is reflecting CIC-1 channel inhibition by the tested compound. P-values of <0.05 is considered significant.

**Table 4: Percentage increase of initial force that was restored**

| **Compound investigated** | **ΔV1/2 (mV)** | **P-value** |
|---|---|---|
| Compound A-6 | 13.0 | <0.01 |
| Compound A-8 | 9.4 | <0.01 |
| Compound A-9 | 15.2 | <0.01 |
| Compound A-26 | 19.7 | <0.01 |
| Compound A-27 | 20.2 | <0.01 |
| Compound A-38 | 17.4 | <0.01 |
| Compound A-40 | 5.19 | <0.01 |
| Compound A-58 | 28.2 | <0.01 |
| Compound A-60 | 20.0 | <0.01 |
| Compound A-66 | 27.4 | <0.01 |
| Compound A-67 | 37.8 | <0.01 |
| Compound A-69 | 32.6 | <0.01 |
| Compound A-74 | 10.4 | <0.01 |
| Compound A-76 | 26.2 | <0.01 |
| Compound A-82 | 19.0 | <0.01 |
| Compound A-85 | 12.5 | <0.01 |
| Compound A-87 | 16.4 | <0.01 |
| Compound A-89 | 10.3 | <0.01 |
| Compound A-95 | 10.3 | <0.01 |

### Example 6: Measurement of In Situ Muscle Contractile Characteristics

Isometric hindlimb force was measured in 12-week old female Lewis rats in the presence and absence of compound.

Rats were placed under anesthesia with isoflurane (2-4%), intubated and subsequently connected to a micro ventilator (Microvent 1, Hallowell EMC, US). Two stimulation electrodes were inserted through the skin to stimulate the sciatic nerve. A small incision was made proximal to the ankle, to expose the Achilles tendon, which was tied by cotton string, and connected to a force transducer (Fort250, World Precision Instruments) with adjustable position (Vernier control). The Achilles tendon was then cut distal to the attached cotton string. The rat was placed on a heated pad, and to prevent movement artefacts from contraction of the ankle dorsiflexors, the foot was fixated by tape on a footplate.

Muscle contractile properties were assessed by applying an electrical current (under supramaximal voltage conditions) to the nerve and recording the force generated by the muscle. The muscle was stretched until maximal force was obtained, when assessed by 2 Hz stimulation. Isometric force was measured every 30 seconds at 12 Hz (Twitch), 10 pulses, and at every 5 minutes at 80 Hz (Tetanic) for 1 second (80 pulses). This stimulation pattern was employed throughout the experiment, expect in few cases where 80 Hz stimulation was replaced by 12 Hz (10 pulses). Neuromuscular transmission was partially inhibited by constant infusion of Cisatracurium (Nimbex, GlaxoSmithKline) at a concentration of 0.1 mg/kg at an adjustable infusion speed, adjusted individually for each animal to obtain a level of inhibition of ca. 50% of the forced generated at 12 Hz stimulation on the 4^{th} pulse. When the level of neuromuscular inhibition was stable, the test article was injected i.v. at the chosen concentration. The effect of test article was assessed on its ability to increase force generated from the stimulation pattern applied. The effect was assessed in the ability to increase force per se (tetanic, 80 Hz, stimulation), and the ratio between individual twitch peaks (12 Hz stimulation). The effect was monitored for at least 1 hour after injection of test article. In addition, the time from injection of test article to maximal effect on force (both twitch and tetanic) was noted and the time for the effect to disappear (return to baseline), if possible. When appropriate the infusion of neuromuscular blocking agent was ceased, with the stimulation pattern continued, and the return of force to control levels was monitored. Animals were sacrificed by cervical dislocation while still fully sedated.

Compound A-27 was dosed 40 mg/kg i.v. The average increase in tetanic force was 36.4% and the average increase in twitch peaks was 12.2% (3 experiments). Compound A-31 was dosed 20 mg/kg i.v. The average increase in tetanic force was 29.8% and the average increase in twitch peaks was 7.3% (3 experiments). Compound A-60 was dosed 20 mg/kg i.v. The average increase in tetanic force was 52.7% and the average increase in twitch peaks was 18.5% (3 experiments). Compound A-87 was dosed 42 mg/kg i.v. The average increase in tetanic force was 19.3% and the average increase in twitch peaks was 5.8% (2 experiments). Compound A-94 was dosed 21 mg/kg i.v. The average increase in tetanic force was 34.8% and the average increase in twitch peaks was 11.5% (2 experiments).

This demonstrates that compounds of the invention, such as Compounds A-27, A-31, A-60, A-87 and A-94 can restore force to muscles *in vivo* which have been partially inhibited by a neuromuscular blocker.

## Claims

1. A compound Formula (VII): 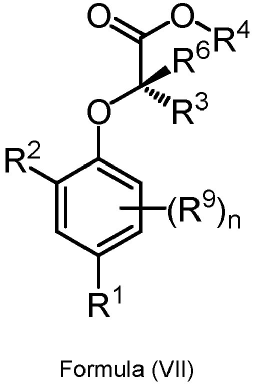 wherein:
R¹ is selected from the group consisting of F, Cl, Br, and I;
R² is selected from the group consisting of hydrogen, deuterium, F, Cl and Br;
R³ is selected from the group consisting of C₁₋₅ alkyl, C₂₋₅ alkenyl, C₃₋₅ cycloalkyl, C₅ cycloalkenyl, each of which is substituted with one or more, identical or different, substituents R⁵, and C₂₋₅ alkynyl, which may be optionally substituted with one or more, identical or different, substituents R⁵; and
- R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁵ is independently selected from the group consisting of F, OC₁₋₅ alkyl optionally substituted with one or more, identical or different, substituents R⁷ and OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof.

2. The compound according to claim 1, wherein:
R¹ is selected from the group consisting of F, Cl, Br and I;
R² is selected from the group consisting of hydrogen, deuterium, F, Cl, and Br;
R³ is selected from the group consisting of C₁₋₅ alkyl, C₂₋₅ alkenyl, C₂₋₅ alkynyl, C₃₋₅ cycloalkyl, and C₅ cycloalkenyl, each of which is substituted with one or more, identical or different, substituents R⁵; and
R⁴ is selected from the group consisting of H, C₁₋₅ alkyl optionally substituted with one or more, identical or different, substituents R⁷, C₃₋₆ cycloalkyl optionally substituted with one or more, identical or different, substituents R⁷, phenyl optionally substituted with one or more, identical or different, substituents R⁸ and benzyl optionally substituted with one or more, identical or different, substituents R⁸;
R⁵ is independently selected from the group consisting of F, OC₁₋₅ alkyl optionally substituted with one or more, identical or different, substituents R⁷ and OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
R⁸ is independently selected from the group consisting of deuterium, methoxy, nitro, cyano, Cl, Br, I and F;
R⁹ is deuterium; and
n is 0, 1, 2 or 3;
or a pharmaceutically acceptable salt, hydrate, polymorph, tautomer, or solvate thereof.

3. The compound according to any one of claims 1 to 2, wherein:
R¹ is Br;
R² is selected from the group consisting of hydrogen, deuterium, F, Cl and Br;
R³ is C₁₋₃ alkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
n is 0;
or a pharmaceutically acceptable salt, hydrate, polymorph, tautomer, or solvate thereof.

4. The compound according to any one of claims 1 to 3, wherein:
R¹ is Br;
R² is selected from the group consisting of hydrogen, deuterium, F, Cl and Br;
R³ is C₃₋₅ cycloalkyl substituted with one or more, identical or different, substituents R⁵;
R⁴ is H;
R⁵ is independently selected from the group consisting of F, -OC₁₋₅ alkyl optionally substituted with one or more, identical or different, substituents R⁷ and -OC₃₋₅ cycloalkyl optionally substituted with one or more, identical or different, substituents R⁷;
R⁶ is selected from the group consisting of hydrogen and deuterium;
R⁷ is independently selected from the group consisting of deuterium and F; and
n is 0
or a pharmaceutically acceptable salt, hydrate, polymorph, tautomer, or solvate thereof.

5. The compound according to any one of claims 1 to 4, wherein R³ is selected from the group consisting of fluoromethyl, difluoromethyl, 2-fluoroeth-1-yl, (1*S*)-1-fluoroeth-1-yl, (1*R*)-1-fluoroeth-1-yl, (1*S*)-1,2-difluoroeth-1-yl, (1*R*)-1,2-difluoroeth-1-yl, 3-fluoroprop-1-yl, (1*S*)-1-fluoroprop-1-yl, (1*R*)-1-fluoroprop-1-yl, (2*S*)-2-fluoroprop-1-yl, (2*R*)-2-fluoroprop-1-yl, (1*S*)-2-fluoro-1-methyl-eth-1-yl, (1*S*)-2-fluoro-1-methyl-eth-1-yl and 2-fluoro-1-(fluoromethyl)eth-1-yl.

6. The compound according to any one of claims 1 to 5, wherein R¹ is Br.

7. The compound according to any one of claims 1 to 6, wherein R⁴ is H.

8. The compound according to any one of claims 1 to 7, wherein n is 0.

9. The compound according to any one of claims 1 to 8, wherein the compound is selected from the group consisting of:
(2*R*)-2-[4-bromo(3,5-²H₂)phenoxy]-3-fluoropropanoic acid;
(2*R*)-2-[4-bromo(2,6-²H₂)phenoxy]-3-fluoropropanoic acid;
(2*R*)-2-(4-bromo-2-fluorophenoxy)-3,3-difluoropropanoic acid;
(2*R*)-2-(4-bromo-2-fluorophenoxy)-3-fluoropropanoic acid;
(2*R*)-2-(2-bromo-4-chlorophenoxy)-3-fluoropropanoic acid;
(2*R*)-2-(4-chlorophenoxy)-3-fluoropropanoic acid;
(2*R*)-2-(4-chloro-2-fluorophenoxy)-3-fluoropropanoic acid;
(2*R*)-2-(2,4-dibromophenoxy)-3-fluoropropanoic acid;
(2*R*)-2-(4-bromophenoxy)-3-fluoropropanoic acid;
(2*S*,3*E*)-2-(4-bromophenoxy)-4-fluorobut-3-enoic acid;
(2*S*)-2-(2,4-dibromophenoxy)-3-methoxypropanoic acid;
(2*R*)-2-(4-bromo-2-chlorophenoxy)-3-fluoropropanoic acid;
(2*S*)-2-(4-bromo-2-chlorophenoxy)-4-fluorobutanoic acid;
(2*R*)-2-(4-bromophenoxy)-3-fluoro(2-²H)propanoic acid;
(2*S*)-2-(4-bromo-2-fluorophenoxy)-4-fluorobutanoic acid;
(2*S*)-2-(4-bromophenoxy)-4-fluorobutanoic acid;
(2*R*,3*R*)-2-(4-bromophenoxy)-3-fluorobutanoic acid;
(2*R*,3*R*)-2-(4-bromo-2-fluorophenoxy)-3-fluorobutanoic acid;
(2*S*)-2-(4-bromophenoxy)pent-4-ynoic acid;
(2*S*)-2-(2,4-dibromophenoxy)pent-4-ynoic acid;
(2*S*)-2-(4-bromo-2-chlorophenoxy)pent-4-ynoic acid; and
(2*S*)-2-(4-bromo-2-fluorophenoxy)pent-4-ynoic acid.

10. The compound according to any one of the preceding claims, wherein the compound is an inhibitor of the CIC-1 ion channel.

11. A composition comprising the compound of any one of claims 1 to 10 and a pharmaceutically acceptable carrier.

12. The compound according to any one of claims 1 to 10, or the composition according to claim 11 for use as a medicament.

13. The compound according to any one of the preceding claims for use in treating, ameliorating and/or preventing a neuromuscular disorder, and/or for use in reversing and/or ameliorating a neuromuscular blockade.

14. The compound for use according to claim 13 wherein the neuromuscular disorder is selected from the group consisting of myasthenia gravis (such as autoimmune and congenital myasthenia gravis), Lambert-Eaton Syndrome, critical illness myopathy, amyotrophic lateral sclerosis (ALS), spinal muscular atrophy (SMA), critical illness myopathy (CIM), reversal diabetic polyneuropathy, Guillain-Barre syndrome, poliomyelitis, post-polio syndrome, chronic fatigue syndrome, critical illness polyneuropathy, and hyperkalemic periodic paralysis.

15. The compound for use according to claim 13 wherein the neuromuscular disorder has been induced by a neuromuscular blocking agent.

## Patentansprüche

1. Verbindung der Formel (VII) 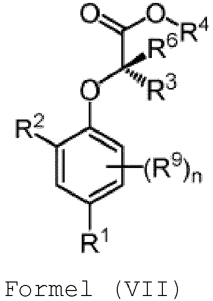 wobei:
R¹ aus der Gruppe bestehend aus F, Cl, Br und I ausgewählt ist;
R² aus der Gruppe bestehend aus Wasserstoff, Deuterium, F, Cl und Br ausgewählt ist;
R³ aus der Gruppe bestehend aus C₁₋₅-Alkyl, C₂₋₅-Alkenyl, C₃₋₅-Cycloalkyl, C₅-Cycloalkenyl ausgewählt ist, die jeweils mit einem oder mehreren gleichen oder verschiedenen Substituenten R⁵ substituiert sind und C₂₋₅-Alkinyl, das optional mit einem oder mehreren gleichen oder verschiedenen Substituenten R⁵ substituiert sein kann; und
- R⁴ aus der Gruppe bestehend aus H, C₁₋₅-Alkyl, optional substituiert mit einem oder mehreren gleichen oder verschiedenen Substituenten R⁷, C₃₋₆-Cycloalkyl, optional substituiert mit einem oder mehreren gleichen oder verschiedenen Substituenten R⁷, Phenyl, optional substituiert mit einem oder mehreren gleichen oder verschiedenen Substituenten R⁸ und Benzyl, optional substituiert mit einem oder mehreren gleichen oder verschiedenen Substituenten R⁸ ausgewählt ist;
R⁵ unabhängig aus der Gruppe bestehend aus F, OC₁₋₅-Alkyl, optional substituiert mit einem oder mehreren, gleichen oder verschiedenen Substituenten R⁷ und OC₃₋₅-Cycloalkyl, optional substituiert mit einem oder mehreren, gleichen oder verschiedenen Substituenten R⁷ ausgewählt ist;
R⁶ aus der Gruppe bestehend aus Wasserstoff und Deuterium ausgewählt ist;
R⁷ unabhängig aus der Gruppe bestehend aus Deuterium und F ausgewählt ist; und
R⁸ unabhängig aus der Gruppe bestehend aus Deuterium, Methoxy, Nitro, Cyano, Cl, Br, I und F ausgewählt ist;
R⁹ Deuterium ist; und
n 0, 1, 2 oder 3 ist;
oder ein pharmazeutisch verträgliches Salz, Hydrat, Polymorph, Tautomer oder Solvat davon.

2. Verbindung nach Anspruch 1, wobei:
R¹ aus der Gruppe bestehend aus F, Cl, Br und I ausgewählt ist;
R² aus der Gruppe bestehend aus Wasserstoff, Deuterium, F, Cl und Br ausgewählt ist;
R³ aus der Gruppe bestehend aus C₁₋₅-Alkyl, C₂₋₅-Alkenyl, C₂₋₅-Alkinyl, C₃₋₅-Cycloalkyl und C₅-Cycloalkenyl ausgewählt ist, die jeweils mit einem oder mehreren gleichen oder verschiedenen Substituenten R⁵ substituiert sind;
R⁴ aus der Gruppe bestehend aus H, C₁₋₅-Alkyl, optional substituiert mit einem oder mehreren gleichen oder verschiedenen Substituenten R⁷, C₃₋₆-Cycloalkyl, optional substituiert mit einem oder mehreren gleichen oder verschiedenen Substituenten R⁷, Phenyl, optional substituiert mit einem oder mehreren gleichen oder verschiedenen Substituenten R⁸ und Benzyl, optional substituiert mit einem oder mehreren gleichen oder verschiedenen Substituenten R⁸ ausgewählt ist;
R⁵ unabhängig aus der Gruppe bestehend aus F, OC₁₋₅-Alkyl, optional substituiert mit einem oder mehreren, gleichen oder verschiedenen Substituenten R⁷ und OC₃₋₅-Cycloalkyl, optional substituiert mit einem oder mehreren, gleichen oder verschiedenen Substituenten R⁷ ausgewählt ist;
R⁶ aus der Gruppe bestehend aus Wasserstoff und Deuterium ausgewählt ist;
R⁷ unabhängig aus der Gruppe bestehend aus Deuterium und F ausgewählt ist; und
R⁸ unabhängig aus der Gruppe bestehend aus Deuterium, Methoxy, Nitro, Cyano, Cl, Br, I und F ausgewählt ist;
R⁹ Deuterium ist; und
n 0, 1, 2 oder 3 ist;
oder ein pharmazeutisch verträgliches Salz, Hydrat, Polymorph, Tautomer oder Solvat davon.

3. Verbindung nach einem der Ansprüche 1 bis 2, wobei:
R¹ Br ist;
R² aus der Gruppe bestehend aus Wasserstoff, Deuterium, F, Cl und Br ausgewählt ist;
R³ C₁₋₃-Alkyl, substituiert mit einem oder mehreren gleichen oder verschiedenen Substituenten R⁵ ist;
R⁴ H ist;
R⁵ unabhängig aus der Gruppe bestehend aus F, OC₁₋₅-Alkyl, optional substituiert mit einem oder mehreren, gleichen oder verschiedenen Substituenten R⁷ und OC₃₋₅-Cycloalkyl, optional substituiert mit einem oder mehreren, gleichen oder verschiedenen Substituenten R⁷ ausgewählt ist;
R⁶ aus der Gruppe bestehend aus Wasserstoff und Deuterium ausgewählt ist;
R⁷ unabhängig aus der Gruppe bestehend aus Deuterium und F ausgewählt ist; und
n 0 ist;
oder ein pharmazeutisch verträgliches Salz, Hydrat, Polymorph, Tautomer oder Solvat davon.

4. Verbindung nach einem der Ansprüche 1 bis 3, wobei:
R¹ Br ist;
R² aus der Gruppe bestehend aus Wasserstoff, Deuterium, F, Cl und Br ausgewählt ist;
R³ C₃₋₅-Cycloalkyl, substituiert mit einem oder mehreren gleichen oder verschiedenen Substituenten R⁵ ist;
R⁴ H ist;
R⁵ unabhängig aus der Gruppe bestehend aus F, OC₁₋₅-Alkyl, optional substituiert mit einem oder mehreren, gleichen oder verschiedenen Substituenten R⁷ und OC₃₋₅-Cycloalkyl, optional substituiert mit einem oder mehreren, gleichen oder verschiedenen Substituenten R⁷ ausgewählt ist;
R⁶ aus der Gruppe bestehend aus Wasserstoff und Deuterium ausgewählt ist;
R⁷ unabhängig aus der Gruppe bestehend aus Deuterium und F ausgewählt ist; und
n 0 ist;
oder ein pharmazeutisch verträgliches Salz, Hydrat, Polymorph, Tautomer oder Solvat davon.

5. Verbindung nach einem der Ansprüche 1 bis 4, wobei R³ aus der Gruppe bestehend aus Fluormethyl, Difluormethyl, 2-Fluoreth-1-yl, (1*S*)-1-Fluoreth-1-yl, (1*R*)-1-Fluoreth-1-yl, (1*S*)-1,2-Difluoreth-1-yl, (1*R*)-1,2-Difluoreth-1-yl, 3-Fluorprop-1-yl, (1*S*)-1-Fluorprop-1-yl, (1*R*)-1-Fluorprop-1-yl, (2*S*)-2-Fluorprop-1-yl, (2*R*)-2-Fluorprop-1-yl, (1*S*)-2-Fluor-1-methyleth-1-yl, (1*S*)-2-Fluor-1-methyleth-1-yl and 2-Fluor-1-(fluormethyl)eth-1-yl ausgewählt ist.

6. Verbindung nach einem der Ansprüche 1 bis 5, wobei R¹ Br ist.

7. Verbindung nach einem der Ansprüche 1 bis 6, wobei R⁴ H ist.

8. Verbindung nach einem der Ansprüche 1 bis 7, wobei n 0 ist.

9. Verbindung nach einem der Ansprüche 1 bis 8, wobei die Verbindung ausgewählt ist aus der Gruppe bestehend aus:
(2*R*)-2-[4-Brom(3,5-²H₂)phenoxy]-3-fluorpropansäure;
(2*R*)-2-[4-Brom(2,6-²H₂)phenoxy]-3-fluorpropansäure;
(2*R*)-2-(4-Brom-2-fluorphenoxy)-3,3-difluorpropansäure;
(2*R*)-2-(4-Brom-2-fluorphenoxy)-3-fluorpropansäure;
(2*R*)-2-(2-Brom-4-chlorphenoxy)-3-fluorpropansäure;
(2*R*)-2-(4-Chlorphenoxy)-3-fluorpropansäure;
(2*R*)-2-(4-Chlor-2-fluorphenoxy)-3-fluorpropansäure;
(2*R*)-2-(2,4-Dibromphenoxy)-3-fluorpropansäure;
(2*R*)-2-(4-Bromphenoxy)-3-fluorpropansäure;
(2*S*,3*E*)-2-(4-Bromphenoxy)-4-fluorbut-3-ensäure;
(2*S*)-2-(2,4-Dibromphenoxy)-3-methoxypropansäure;
(2*R*)-2-(4-Brom-2-chlorphenoxy)-3-fluorpropansäure;
(2*S*)-2-(4-Brom-2-chlorphenoxy)-4-fluorbutansäure;
(2*R*)-2-(4-Bromophenoxy)-3-fluor(2-²H)propansäure;
(2*S*)-2-(4-Brom-2-fluorphenoxy)-4-fluorbutansäure;
(2*S*)-2-(4-Bromphenoxy)-4-fluorbutanäure;
(2*R*,3*R*)-2-(4-Bromphenoxy)-3-fluorbutansäure;
(2*R*,3*R*)-2-(4-Brom-2-fluorphenoxy)-3-fluorbutansäure;
(2*S*)-2-(4-Bromphenoxy)pent-4-insäure;
(2*S*)-2-(2,4-Dibromphenoxy)pent-4-insäure;
(2*S*)-2-(4-Brom-2-chlorphenoxy)pent-4-insäure; und
(2*S*)-2-(4-Brom-2-fluorphenoxy)pent-4-insäure.

10. Verbindung nach einem der vorhergehenden Ansprüche, wobei die Verbindung ein Hemmer des CIC-1-Ionenkanals ist.

11. Zusammensetzung, umfassend die Verbindung nach einem der Ansprüche 1 bis 10 und einen pharmazeutisch verträglichen Träger.

12. Verbindung nach einem der Ansprüche 1 bis 10 oder Zusammensetzung nach Anspruch 11 zur Verwendung als ein Medikament.

13. Verbindung nach einem der vorhergehenden Ansprüche zur Verwendung beim Behandeln, Lindern und/oder Vorbeugen einer neuromuskulären Erkrankung und/oder zur Verwendung beim Umkehren und/oder Lindern einer neuromuskulären Blockade.

14. Verbindung zur Verwendung nach Anspruch 13, wobei die neuromuskuläre Erkrankung aus der Gruppe bestehend aus Myasthenia gravis (wie etwa autoimmun-vermittelte und angeborene Myasthenia gravis), Lambert-Eaton-Syndrom, Critical-Illness-Myopathie, amyotropher Lateralsklerose (ALS), spinaler Muskelatrophie (SMA), Critical-Illness-Myopathie (CIM), reversibler diabetischer Polyneuropathie, Guillain-Barré-Syndrom, Poliomyelitis, Post-Polio-Syndrom, chronischem Müdigkeitssyndrom, Critical-Illness-Polyneuropathie und hyperkaliämischer periodischer Lähmung ausgewählt ist.

15. Verbindung zur Verwendung nach Anspruch 13, wobei die neuromuskuläre Erkrankung durch ein neuromuskuläres Blockierungsmittel induziert wurde.

## Revendications

1. Composé de formule (VII) : 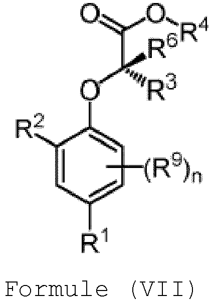 dans lequel :
R¹ est choisi dans le groupe constitué par F, Cl, Br et I ;
R² est choisi dans le groupe constitué par l'hydrogène, le deutérium, F, Cl et Br ;
R³ est choisi dans le groupe constitué par alkyle en C₁₋₅, alcényle en C₂₋₅, cycloalkyle en C₃₋₅ et cycloalcényle en C₅, chacun d'eux étant éventuellement substitué par un ou plusieurs substituants R⁵, identiques ou différents ; et
alcynyle en C₂₋₅ qui peut être éventuellement substitué par un ou plusieurs substituants R⁵, identiques ou différents ; et
- R⁴ est choisi dans le groupe constitué par H, alkyle en C₁₋₅ éventuellement substitué par un ou plusieurs substituants R⁷, identiques ou différents, cycloalkyle en C₃₋₆, éventuellement substitué par un ou plusieurs substituants, R⁷, identiques ou différents, phényle éventuellement substitué par un ou plusieurs substituants R⁸, et benzyle éventuellement substitué par un ou plusieurs substituants R⁸, identiques ou différents ;
R⁵ est indépendamment choisi dans le groupe constitué par F, Oalkyle en C₁₋₅ éventuellement substitué par un ou plusieurs substituants R⁷ identiques ou différents, et Ocycloalkyle en C₃₋₅ éventuellement substitué par un ou plusieurs substituants R⁷, identiques ou différents.
R⁶ est choisi dans le groupe constitué par l'hydrogène et le deutérium ;
R⁷ est indépendamment choisi dans le groupe constitué par le deutérium et F ; et
R⁸ est indépendamment choisi dans le groupe constitué par le deutérium, méthoxy, nitro, cyano, Cl, Br, I et F ;
R⁹ est le deutérium ; et
n est 0, 1, 2 ou 3 ;
ou un sel, hydrate, polymorphe, tautomère ou solvate pharmaceutiquement acceptable de celui-ci.

2. Composé selon la revendication 1, dans lequel :
R¹ est choisi dans le groupe constitué F, Cl, Br et I ;
R² est choisi dans le groupe constitué par l'hydrogène, le deutérium, F, Cl et Br ;
R³ est choisi dans le groupe constitué par alkyle en C₁₋₅, alcényle en C₂₋₅, alcynyle en C₂₋₅, cycloalkyle en C₃₋₅ et cycloalcényle en C₅, chacun d'eux pouvant être éventuellement substitué par un ou plusieurs substituants R⁵, identiques ou différents ;
R⁴ est choisi dans le groupe constitué par H, alkyle en C₁₋₅ éventuellement substitué par un ou plusieurs substituants, R⁷, identiques ou différents, cycloalkyle en C₃₋₆, éventuellement substitué par un ou plusieurs substituants, R⁷, identiques ou différents, phényle éventuellement substitué par un ou plusieurs substituants, R⁸, identiques ou différents, et benzyle éventuellement substitué par un ou plusieurs substituants R⁸, identiques ou différents ;
R⁵ est indépendamment choisi dans le groupe constitué par F, Oalkyle en C₁₋₅ éventuellement substitué par un ou plusieurs substituants R⁷ identiques ou différents, et Ocycloalkyle en C₃₋₅ éventuellement substitué par un ou plusieurs substituants R⁷ identiques ou différents ;
R⁶ est choisi dans le groupe constitué par l'hydrogène et le deutérium ;
R⁷ est indépendamment choisi dans le groupe constitué par le deutérium et F ; et
R⁸ est indépendamment choisi dans le groupe constitué par le deutérium, méthoxy, nitro, cyano, Cl, Br, I et F ;
R⁹ est le deutérium ; et
n est 0, 1, 2 ou 3 ;
ou un sel, hydrate, polymorphe, tautomère ou solvate pharmaceutiquement acceptable de celui-ci.

3. Composé selon l'une quelconque des revendications 1 et 2, dans lequel :
R¹ est Br ;
R² est choisi dans le groupe constitué par l'hydrogène, le deutérium, F, Cl et Br ;
R³ est alkyle en C₁₋₃ éventuellement substitué par un ou plusieurs substituants R⁵ identiques ou différents ;
R⁴ est H ;
R⁵ est indépendamment choisi dans le groupe constitué par F, Oalkyle en C₁₋₅ éventuellement substitué par un ou plusieurs substituants R⁷ identiques ou différents, et Ocycloalkyle en C₃₋₅ éventuellement substitué par un ou plusieurs substituants R⁷ identiques ou différents ;
R⁶ est choisi dans le groupe constitué par l'hydrogène et le deutérium ;
R⁷ est indépendamment choisi dans le groupe constitué par le deutérium et F ; et
n est 0 ;
ou un sel, hydrate, polymorphe, tautomère ou solvate pharmaceutiquement acceptable de celui-ci.

4. Composé selon l'une quelconque des revendications 1 à 3, dans lequel :
R¹ est Br ;
R² est choisi dans le groupe constitué par l'hydrogène, le deutérium, F, Cl et Br ;
R³ est cycloalkyle en C₃₋₅ éventuellement substitué par un ou plusieurs substituants R⁵ identiques ou différents ;
R⁴ est H ;
R⁵ est indépendamment choisi dans le groupe constitué par F, Oalkyle en C₁₋₅ éventuellement substitué par un ou plusieurs substituants R⁷ identiques ou différents, et Ocycloalkyle en C₃₋₅ éventuellement substitué par un ou plusieurs substituants R⁷ identiques ou différents ;
R⁶ est choisi dans le groupe constitué par l'hydrogène et le deutérium ;
R⁷ est indépendamment choisi dans le groupe constitué par le deutérium et F ; et
n est 0 ;
ou un sel, hydrate, polymorphe, tautomère ou solvate pharmaceutiquement acceptable de celui-ci.

5. Composé selon l'une quelconque des revendications 1 à 4, dans lequel R³ est choisi dans le groupe constitué par le fluorométhyle, le difluorométhyle, le 2-fluoroéth-1-yle, le (1*S*)-1-fluoroéth-1-yle, le (1*R*)-1-fluoroéth-1-yle, le (1*S*)-1,2-difluoroéth-1-yle, le (1*R*)-1,2-difluoroéth-1-yle, le 3-fluoroprop-1-yle, le (1*S*)-1-fluoroprop-1-yle, le (1*R*)-1-fluoroprop-1-yle, le (2*S*)-2-fluoroprop-1-yle, le (2*R*)-2-fluoroprop-1-yle, le (1*S*)-2-fluoro-1-méthyl-éth-1-yle, le (1*S*)-2-fluoro-1-méthyl-éth-1-yle et le 2-fluoro-1-(fluorométhyl)éth-1-yle.

6. Composé selon l'une quelconque des revendications 1 à 5, dans lequel R¹ est Br.

7. Composé selon l'une quelconque des revendications 1 à 6, dans lequel R⁴ est H.

8. Composé selon l'une quelconque des revendications 1 à 7, dans lequel n est 0.

9. Composé selon l'une quelconque des revendications 1 à 8, dans lequel le composé est choisi dans le groupe constitué par :
l'acide (2*R*)-2-[4-bromo(3,5-²H₂)phénoxy]-3-fluoropropanoïque ;
l'acide (2*R*)-2-[4-bromo(2,6-²H₂)phénoxy]-3-fluoropropanoïque ;
l'acide (2*R*)-2-(4-bromo-2-fluorophénoxy)-3,3-difluoropropanoïque ;
l'acide (2*R*)-2-(4-bromo-2-fluorophénoxy)-3-fluoropropanoïque ;
l'acide (2*R*)-2-(2-bromo-4-chlorophénoxy)-3-fluoropropanoïque ;
l'acide (2*R*)-2-(4-chlorophénoxy)-3-fluoropropanoïque ;
l'acide (2*R*)-2-(4-chloro-2-fluorophénoxy)-3-fluoropropanoïque ;
l'acide (2*R*)-2-(2,4-dibromophénoxy)-3-fluoropropanoïque ;
l'acide (2*R*)-2-(4-bromophénoxy)-3-fluoropropanoïque ;
l'acide (2*S*,3*E*)-2-(4-bromophénoxy)-4-fluorobut-3-énoïque ;
l'acide (2*S*)-2-(2,4-dibromophénoxy)-3-méthoxypropanoïque ;
l'acide (2*R*)-2-(4-bromo-2-chlorophénoxy)-3-fluoropropanoïque ;
l'acide (2*S*)-2-(4-bromo-2-chlorophénoxy)-4-fluorobutanoïque ;
l'acide (2*R*)-2-(4-bromophénoxy)-3-fluoro(2-²H)propanoïque ;
l'acide (2*S*)-2-(4-bromo-2-fluorophénoxy)-4-fluorobutanoïque ;
l'acide (2*S*)-2-(4-bromophénoxy)-4-fluorobutanoïque ;
l'acide (2*R*,3*R*)-2-(4-bromophénoxy)-3-fluorobutanoïque ;
l'acide (2*R*,3*R*)-2-(4-bromo-2-fluorophénoxy)-3-fluorobutanoïque ;
l'acide (2*S*)-2-(4-bromophénoxy)pent-4-ynoïque ;
l'acide (2*S*)-2-(2,4-dibromophénoxy)pent-4-ynoïque ;
l'acide (2*S*)-2-(4-bromo-2-chlorophénoxy)pent-4-ynoïque ; et
l'acide (2*S*)-2-(4-bromo-2-fluorophénoxy)pent-4-ynoïque.

10. Composé selon l'une quelconque des revendications précédentes, dans lequel le composé est un inhibiteur du canal ionique de CIC-1.

11. Composition comprenant le composé selon l'une quelconque des revendications 1 à 10 et un support pharmaceutiquement acceptable.

12. Composé selon l'une quelconque des revendications 1 à 10 ou composition selon la revendication 11 destiné à être utilisé comme médicament.

13. Composé selon l'une quelconque des revendications précédentes destiné à être utilisé dans le traitement, l'amélioration et/ou la prévention d'un trouble neuromusculaire, et/ou destiné à être utilisé dans l'inversion et/ou l'amélioration d'un blocage neuromusculaire.

14. Composé destiné à être utilisé selon la revendication 13, dans lequel le trouble neuromusculaire est choisi dans le groupe constitué par la myasthénie grave (telle que la myasténie grave autoimmune et congénitale), le syndrome de Lambert-Eaton, la myopathie critique, la sclérose latérale amyotrophique (ALS), l'amyotrophie spinale (SMA), la myopathie critique (CIM), la polyneuropathie diabétique de réversion, le syndrome de Guillain-Barré, la poliomyélite, le syndrome post-polio, le syndrome de fatigue chronique, la polyneuropathie critique et la paralysie périodique hyperkaliémie.

15. Composé destiné à être utilisé selon la revendication 13, dans lequel le trouble neuromusculaire a été induit par un agent bloquant neuromusculaire.
